# EUROPEAN PATENT SPECIFICATION

(11) **EP 4 226 163 B1**
(45) Date of publication and mention of the grant of the patent: **24.12.2025**
(21) Application number: 21816564.5
(22) Date of filing: 12.10.2021
(51) Int. Cl.: G01N 35/00, G01N 33/543, C12M 1/00

(54) **MAGNETIC PARTICLE PROCESSING SYSTEMS FOR USE WITH BIOLOGICAL CELLS AND RELATED METHODS**
MAGNETPARTIKELVERARBEITUNGSSYSTEME ZUR VERWENDUNG MIT BIOLOGISCHEN ZELLEN UND ZUGEHÖRIGE VERFAHREN
SYSTÈMES DE TRAITEMENT DE PARTICULES MAGNÉTIQUES DESTINÉS À ÊTRE UTILISÉS AVEC DES CELLULES BIOLOGIQUES ET PROCÉDÉS ASSOCIÉS

(30) Priority: 12.10.2020 US 202063090399 P; 14.01.2021 US 202163137389 P
(43) Date of publication of application: 16.08.2023
(73) Proprietor: Life Technologies AS, 0309 Oslo (NO); Life Technologies Corporation, Carlsbad, CA 92008 (US); Life Technologies Holdings Pte Limited, Singapore 739256 (SG)
(72) Inventor: LUHR, Morten, 0309 Oslo (NO); GORDON, Michael, Carlsbad, California 92008 (US); SIA, Ming Tiong, Singapore 739256 (SG); CHONG, Kok Shyong, Singapore 739256 (SG); SOH, Woon Liang Terence, Singapore 739256 (SG); LIM, Seng Leong, Singapore 739256 (SG); SCHROEDER, Ida Caroline, 0309 Oslo (NO); ALFSNES, Katrine, 0309 Oslo (NO); LIM, Terry JianHui, Singapore 739256 (SG); BOSNES, Marie, 0309 Oslo (NO); NEURAUTER, Axl Alois, 0309 Oslo (NO); PEDERSEN, Ketil Winther, 0309 Oslo (NO); WILBUR, Autumn Day, Carlsbad, California 92008 (US); ZIMMERMAN, Sean, Carlsbad, California 92008 (US)
(74) Representative: HGF
(86) International application number: PCT/US2021/054490
(87) International publication number: WO 2022/081519

(56) References cited:
- WO-A1-2016/164635
- WO-A1-2019/106207
- US-A1- 2019 010 435
- US-A1- 2019 339 261
- US-A1- 2019 345 477

## Description

### FIELD OF THE INVENTION

The present invention relates, in part, to magnetic support (e.g., bead) processing apparatus operable with select disposable consumable kits for selectively isolating and/or activating biological cells (e.g., mammalian cells, such as T cells, B cells, and NK cells), as well as purification of other biological materials. The present invention further relates to magnetic bead processing apparatus operable with select disposable consumable kits for selectively isolating and/or activating, for example, T-cells using magnetic beads or for separating the magnetic beads from the T-cell after activation of the T-cells.

### BACKGROUND OF THE INVENTION

A number of devices, methods and reagents have been developed that used magnetic attraction for the isolation and/or activation of cells. Often it is desirable to separate magnetic materials from cells at some point in workflows.

US 2019/0339261 A1 discloses methods using magnetic immunoglobulin-binding particles.

In part, provided herein are devices and reagents that allows for improvements in cell related workflows that involve the use of magnetic materials (e.g., magnetic supports, such as beads).

Some advantages of the bead processing assemblies and systems set out herein are that they are flexible in terms of steps and may be used in workflows that are scalable and automated. Further these magnetic processing systems may also be designed for closed-system and single-use workflows. One additional advantage that bead processing assemblies and systems set out herein have over many other types of magnetic processing system is possible to get the magnet closer to the magnetic particles the assemblies and systems set out herein. These advantages make these magnetic processing systems well suited for commercial manufacturing.

### SUMMARY OF THE DISCLOSURE

The invention is defined by the appended claims. In one independent aspect of the present disclosure a bead processing assembly for use in attaching magnetic beads to biological cells and/or separating magnetic beads from biological cells is provided wherein the bead processing assembly comprises:
a base assembly comprising:
   a housing assembly;
   a support panel disposed on the housing assembly and having a front face;
   a first pinch valve at least partially outwardly projecting from the front face of the support panel; and
   a first pump at least partially outwardly projecting from the front face of the support panel; and
a rocker assembly supported on the base assembly, the rocker assembly comprising:
   a mount assembly supported on the base assembly;
   a platform assembly pivotably secured to the mount assembly; and
   means for repeatedly rocking the platform assembly relative to the mount assembly.

In one alternative embodiment, the base assembly further comprises a cover panel hingedly mounted to the housing assembly, the cover panel being movable between an open position wherein the front face of the support panel is openly exposed and a closed position wherein the cover panel covers the front face of the support panel.

In another alternative embodiment, the cover panel comprises:
a perimeter frame hingedly mounted to the housing and encircling an opening; and
a transparent window disposed within the opening.

In another alternative embodiment, the support panel comprises:
a base panel having a front face, the first pinch valve and the first pump being mounted on the base panel so as to at least partially outwardly project from the front face thereof; and
an overlay panel being disposed on the front face of the base panel, the overlay panel having openings extending therethrough through which at least portions of the first pinch valve and first pump project.

In another alternative embodiment, the front face of the support panel is disposed at an angle in a range between 30° and 70° when the housing assembly is resting on a horizontal surface.

In another alternative embodiment, the base assembly further comprises a plurality of pinch valves at least partially outwardly projecting from the front face of the support panel, wherein the plurality of pinch valves comprise at least 2, 3, 4, 6, or 8 pinch valves.

In another alternative embodiment, the first pump comprises a peristaltic pump.

In another alternative embodiment, the base assembly further comprises a first bubble sensor at least partially outwardly projecting from the front face of the support panel.

In another alternative embodiment, the base assembly further comprises a plurality of bubble sensors at least partially outwardly projecting from the front face of the support panel, the plurality of bubble sensors comprising at least 2, 3, or 4 bubble sensors.

In another alternative embodiment, the base assembly further comprises a pressure sensor at least partially outwardly projecting from the front face of the support panel.

In another alternative embodiment, the base assembly further comprises:
an opening extending through the front face of the support panel; and
a first rotational assembly mounted to the support panel, the first rotational assembly comprising:
   a receiver having a keyed socket formed thereon, the keyed socket being aligned with the opening; and
   a drive motor coupled to the receiver, the drive motor being configured to selectively rotate the receiver in opposite directions.

In another alternative embodiment, the base assembly further comprises a bead vial retainer including:
a body configured to receive a vial;
an elongated arm extending from the body; and
a motor at least partially disposed within the housing assembly of the base assembly and connected to a free end of the arm, the motor being configured to vertically, reciprocally rotate the body attached thereto over an angle of at least 60°.

In another alternative embodiment, the body of the bead vial retainer comprises:
an interior surface that bounds a C-shaped channel; and
a shoulder that radially inwardly project from the interior surface.

In another alternative embodiment, the mount assembly of the rocker assembly comprises a first riser and a spaced apart second riser mounted on the base assembly, the platform assembly being pivotably coupled to the first riser and the second riser so as to be at least partially disposed between the first riser and the second riser.

In another alternative embodiment, the means for repeatedly rocking the platform assembly relative to the mount assembly comprises:
a crank;
a motor that selectively rotates the crank; and
a connecting arm that extends from the crank to the platform assembly.

In another alternative embodiment, the platform assembly comprises:
a housing assembly bounding a compartment;
a platform mounted to the housing assembly;
a magnet assembly disposed between the housing assembly and the platform, the magnet assembly having a top surface and an opposing bottom surface; and
means at least partially disposed within the compartment of the housing assembly for selectively raising and lowering the magnet assembly relative to the platform between an activation position and a deactivation position.

In another alternative embodiment, the means for selectively raising and lowering the magnet assembly comprises:
a scissor lift at least partially disposed within the compartment of the housing assembly;
   and
a motor that operates the scissor lift.

In another alternative embodiment, the scissor lift comprises:
a shelf, the magnet assembly being disposed on the shelf;
a first pair of scissor arms extending between the housing assembly and the shelf;
a second pair of scissor arms extending between the housing assembly and the shelf, the second pair of scissor arms being spaced apart from the first pair of scissor arms;
a threaded shaft coupled to the motor; and
a collar engaging the threaded shaft such that rotation of the threaded shaft by the motor facilitates linear movement of the collar along the threaded shaft, the collar also engaging with the first pair of scissor arms and the second pair of scissor arms.

An alternative embodiment further comprising:
the platform comprising a support plate having a top surface and an opposing bottom surface;
wherein when the magnet assembly is raised to the activation position, the bottom surface of the support plate is within 1 cm, 0.5 cm, or 0.2 cm of the top surface of the magnet assembly.

In another alternative embodiment, when the magnet assembly is lowered to the deactivation position, the bottom surface of the support plate is at least 4 cm, 5 cm, or 6 cm away from the top surface of the magnet assembly.

In another alternative embodiment, the magnet assembly comprises a magnet.

In another alternative embodiment, the magnet assembly comprises:
a non-magnetic casing having a top surface and an opposing bottom surface that extend to a perimeter edge, a recess being formed on the top surface and having a perimeter edge;
the magnet disposed within the recess of the casing.

In another alternative embodiment, the magnet comprises a plurality of separate and discrete magnets being disposed in a plurality of alternating orientations so as to produce a Halbach array.

In another alternative embodiment, the perimeter edge of the recess is inset from the perimeter edge of the casing at least 0.5 cm, 1 cm, 1.5 cm or 2 cm.

In another alternative embodiment, the casing and the magnet each have a rectangular configuration.

In another alternative embodiment, the platform assembly further comprises a cover assembly at least partially covering the platform and being movable relative to the platform.

In another alternative embodiment, the cover assembly comprises:
a cover housing that at least partially encircles an opening, the platform being at least partially disposed within the opening of the cover housing; and
a lid being movably mounted to the cover housing between an open and closed position, the lid at least substantially covering the opening of the cover housing when in the closed position.

In another alternative embodiment, the cover plate is hingedly mounted to the cover housing.

Another alternative embodiment further includes a first latch for securing the cover plate to the cover housing when the cover plate is in the closed position.

Another alternative embodiment further includes means for resiliently restraining movement of the cover assembly away from the platform.

In another alternative embodiment, the means for resiliently restraining movement comprises:
a rod having a first end and an opposing second end, the first end being secured to and projecting from cover assembly; and
a spring engaged with the rod such that as a force is used to move the cover assembly away from the platform the spring resiliently urges the cover assembly back toward the platform.

Another alternative embodiment further includes a stop movable between a restraining position and a non-restraining position, in the restraining position, the stop is positioned to preclude some movement of the rod relative to the platform and in the non-restraining position the stop does not interfere with movement of the rod.

Another alternative embodiment further includes a solenoid valve mounted on the housing assembly, the solenoid valve moving the stop between the restraining position and a non-restraining position

Another alternative embodiment further includes:
a hole formed on the housing assembly, the rod being slidably disposed within the hole;
a flange outwardly projecting from the second end of the rod; and
the spring extending between the flange and the housing assembly so that as the rod is concurrently lifted with the cover assembly, the springe is resiliently compressed.

Another alternative embodiment further includes a stop movable between a restraining position and a non-restraining position, in the restraining position, the stop is aligned with the flange so as to block some movement of the flange and the rod attached thereto, in the non-restraining position, the stop is not aligned with the flange and thus does not interfere with movement of the flange or rod attached thereto.

Another alternative embodiment further includes:
a recess formed on the cover housing and communicating with the opening of the cover housing; and
a clamp assembly slidably disposed within the recess, the clamp assembly comprising:
   a base clamp having a lower clamp grove formed thereon;
   a clamp closure having an upper clamp groove formed thereon; and
   a fastener selectively securing the clamp closure to the base clamp so that that upper clamp groove is aligned with the lower clamp groove.

In another alternative embodiment, the cover housing further comprises a U-shaped channel formed at each opposing end of the recess, opposing ends of the clamp assembly being slidably disposed with the U-shaped channels.

In another alternative embodiment, the platform comprises:
a support plate having a top surface and an opposing bottom surface; and
a sidewall projecting away from the support plate and being secured to the housing assembly, the support plate and sidewall at least partially bonding a cavity in which the magnet assembly is received when the magnet assembly is in the raised activated position.

A second independent aspect of the present disclosure includes a bead processing system for attaching magnetic beads to biological cells and/or separating magnetic beads from biological cells, the bead processing system comprising:
a bead processing assembly comprising:
   a base assembly comprising:
      a housing assembly;
      a support panel disposed on the housing assembly and having a front face;
      a first pinch valve at least partially outwardly projecting from the front face of the support panel; and
      a first pump at least partially outwardly projecting from the front face of the support panel; and
   a rocker assembly supported on the base assembly, the rocker assembly comprising:
      a mount assembly supported on the base assembly;
      a platform assembly pivotably secured to the mount assembly and comprising a platform; and
      means for repeatedly rocking the platform assembly relative to the mount assembly;
a consumable kit comprising:
   a tray having a front face and an opposing back face with a plurality of openings extending therebetween, the tray being removably nested on the front face of the support panel so that the first pinch valve and the first pump project through corresponding ones of the plurality of openings; and
   a line set secured to the front face of the tray, the line set comprising:
      flexible tubing secured to the front face of the tray and engaging with the first pinch valve and the first pump; and
      a plurality of flexible bags fluid coupled with the tubing, the plurality of flexible bags comprising a processing bag supported on the platform of the platform assembly.

In one alternative embodiment, the base assembly further comprises a cover panel hingedly mounted to the housing assembly, the cover panel being movable between an open position wherein the front face of the tray is openly exposed and a closed position wherein the cover panel covers the front face of the tray.

In another alternative embodiment, the base assembly further comprises a plurality of pinch valves at least partially outwardly projecting from the front face of the support panel, wherein the plurality of pinch valves comprise at least 2, 3, 4, 6, or 8 pinch valves, wherein each of the plurality of pinch valves project through corresponding ones of the plurality of openings on the tray and engage with the flexible tubing.

In another alternative embodiment, the base assembly further comprises a first bubble sensor at least partially outwardly projecting from the front face of the support panel, the first bubble sensor projecting through a corresponding one of the plurality of openings on the tray and engage with the flexible tubing.

Another alternative embodiment further includes:
an opening extending through the front face of the support panel; and
a first rotational assembly mounted to the support panel, the first rotational assembly comprising:
   a receiver having a keyed socket formed thereon, the keyed socket being aligned with the opening; and
   a drive motor coupled to the receiver, the drive motor being configured to selectively rotate the receiver in opposite directions;
   a stopcock fluid coupled with the tubing of the line set, the stopcock having a rotatable handle received within the keyed socket of the receiver; and
   an air filter fluid coupled with the stopcock.

Another alternative embodiment further includes a bead vial retainer including:
a body having a channel configured to receive a vial;
an elongated arm extending from the body;
a motor at least partially disposed within the housing assembly of the base assembly and connected to a free end of the arm, the motor being configured to vertically, reciprocally rotate the body attached thereto over an angle of at least 60°; and
a vial received within the channel of the body, the vial housing magnetic beads and a media, the tubing of the line set being fluid coupled with the vial.

In another alternative embodiment, the platform assembly comprises:
a housing assembly bounding a compartment;
a platform mounted to the housing assembly;
a magnet assembly disposed between the housing assembly and the platform, the magnet assembly having a top surface and an opposing bottom surface; and
means at least partially disposed within the compartment of the housing assembly for selectively raising and lowering the magnet assembly relative to the platform between an activation position and a deactivation position.

In another alternative embodiment, the platform assembly further comprises a cover assembly at least partially covering the platform and the processing bag thereon, the cover assembly being movable relative to the platform as the processing bag expands.

Another alternative embodiment further includes means for resiliently restraining movement of the cover assembly away from the platform.

In another alternative embodiment, the cover assembly comprises:
a cover housing that at least partially encircles an opening, the processing bag being at least partially disposed within the opening of the cover housing; and
a cover plate being movably mounted to the cover housing between an open and closed position, the cover plate at least substantially covering the opening of the cover housing when in the closed position.

Another alternative embodiment further includes:
a recess formed on the cover housing and communicating with the opening of the cover housing;
a clamp assembly slidably disposed within the recess, the clamp assembly comprising:
   a base clamp having a lower clamp groove formed thereon
   a clamp closure having an upper clamp groove formed thereon; and
   a fastener selectively securing the clamp closure to the base clamp so that that upper clamp groove is aligned with the lower clamp groove;
wherein the processing bag disposed on the platform has a port that is at least partially disposed within the aligned upper clamp groove and lower clamp groove and is clamped between the base clamp and the clamp closure.

Another alternative embodiment further includes a first media bag fluid coupled to the tubing of the line set and housing a medium.

In another alternative embodiment, the bead processing assembly further comprises a stand upstanding from the base assembly and having a catch outwardly projecting therefrom, the media bag being supported on the catch.

In another alternative embodiment, the tubing of the line set is fluid coupled with a biological cell separator.

In another alternative embodiment, the tubing of the line set is fluid coupled with a biological cell expansion system.

In another alternative embodiment, the processing bag comprise a bead separation bag that comprises:
a collapsible bag body comprised of polymeric film and bounding a compartment;
a pair of spaced apart ports coupled to the bag body and communicating with the compartment;
an elongated partition disposed within the compartment of the bag body at a location between the pair of spaced apart ports, the partition being secured to the bag body so that fluid entering one of the ports must flow around the partition before it can leave through the other port.

In a third independent aspect of the present disclosure, a consumable kit is provided for use with a magnetic bead processing assembly, the consumable kit including:
a tray having a front face and an opposing back face with a plurality of openings extending therebetween; and
a line set secured to the front face of the tray, the line set comprising:
   flexible tubing secured to the front face of the tray;
   a plurality of flexible bags fluid coupled with the tubing; and
   an air filter assembly coupled with the tubing.

In one alternative embodiment, the air filter assembly comprises:
a stop-cock fluid coupled with the tubing, the stopcock comprising:
   a sleeve outwardly projecting from the front face of the tray;
   a valve rotatably disposed within sleeve; and
   a handle secured to the valve for selectively rotating the valve, the handle outwardly projecting from the back face of the tray; and
an air filter fluid coupled with the sleeve.

Another alternative embodiment further includes a mixing bag disposed on the front face of the tray and being fluid coupled with the tubing.

Another alternative embodiment further includes:
a bead vial coupler fluid coupled to the tubing of the line set; and
a vial secured to the bead vial coupler, the vial housing a suspension comprising magnetic beads and a carrier liquid.

A third independent aspect of the present disclosure includes a method for operating a bead processing system for attaching magnetic beads to biological cells and/or separating magnetic beads from biological cells, the method comprising:
removably nesting a tray on a front face of a support panel of a bead processing assembly so that a first pinch valve and a first pump projecting from the support panel pass through corresponding openings formed on the tray;
engaging a tubing of a line set disposed on the tray to the first pinch valve and the first pump; and
placing a processing bag fluid coupled with the line set on a platform of the bead processing system.

An alternative embodiment further includes fluid coupling a vial to the tubing of the line set, the vial housing a suspension comprising magnetic beads and a carrier liquid.

Another alternative embodiment further includes supporting a media bag housing liquid media on a bag stand of the bead processing assembly.

Another alternative embodiment further includes fluid coupling the tubing of the line set to a biological cell separator.

Another alternative embodiment further includes fluid coupling the tubing of the line set to a biological cell expansion system.

Another alternative embodiment further includes activating a computer processor of the bead processing assembly so that the computer processor facilitates rocking of the platform on which the processing bag is placed.

Another alternative embodiment further includes activating a computer processor of the bead processing assembly so that the computer processor facilitates raising and lowering a magnet relative to the platform on which the processing bag is placed.

Another alternative embodiment further includes activating a computer processor of the bead processing assembly so that the computer processor facilitates controlling the first pinch valve and the first pump to deliver a fluid to the processing bag on the platform.

Another alternative embodiment further includes activating a computer processor of the bead processing assembly so that the computer processor facilitates moving a vial fluid coupled with the tubing of the line set so as to mix a suspension disposed within the vial, the suspension comprising magnetic beads and a carrier liquid.

Another alternative embodiment further includes the biological cells being T cells.

In another alternative embodiment, the biological cells are attached to the magnetic beads through an antigen-antibody interaction.

In another alternative embodiment, the biological cells are detached from the magnetic beads by disruption of the antigen-antibody interaction.

In another alternative embodiment, the disruption of the antigen-antibody interaction is mediated by cleavage of the antibody.

In another alternative embodiment, the biological cells are detached from the magnetic beads by separation of the antibody from the magnetic beads.

In another alternative embodiment, the antibody is linked to the magnetic beads by a ligand.

In another alternative embodiment, the biological cells are detached from the magnetic beads by disruption of the ligand interaction with the antibody or the magnetic bead.

In another alternative embodiment, the biological cells are T cells.

In a fifth independent aspect of the present disclosure, a method is provided for separating biological cells of a first cell type from biological cells of a second cell type using a bead processing system, the method comprising:
contacting a sample comprising the biological cells of the first cell type and the second cell type with magnetic beads so that the magnetic beads attach to the biological cells of the first cell type through an antibody linkage, the sample and magnetic beads being disposed within a processing bag resting on a platform;
raising a magnet relative to the platform so that the magnet produces a magnetic field that securely fixes in place the magnetic beads relative to the platform, the magnetic beads having the biological cells of the first cell type attached thereto;
passing a fluid through the sample and the magnetic beads while the magnetic field is applied thereto, the fluid passing with a force sufficiently strong to wash away the biological cells of the second cell type from the biological cells of the first cell type but not sufficiently strong to separate the biological cells of the first cell type from the magnetic beads; and
lowering the magnet relative to the platform so that the magnetic beads having the biological cells of the first cell type attached thereto are no longer securely fixed in place relative to the platform by magnetic field of the magnet.

In one alternative embodiment, the antibody has binding affinity for a protein selected from the group consisting of CD3, CD4, CD5, CD6, CD8, CD25, CD27, CD28, CD137, and CD278.

In another alternative embodiment, the antibody has binding affinity for a protein comprising CD3 or CD28.

In another alternative embodiment, the first cell type is T cells, B cells, or NK cells.

Another alternative embodiment includes detaching at least a majority of the biological cells of first cell type from the magnetic beads;
applying a magnetic field to the magnetic beads so as to securely fix the magnetic beads in place; and
passing a fluid through the magnetic beads and the separated biological cells of first cell type while the magnetic field is being applied, the fluid passing with a force sufficiently strong to wash away at least a majority of the biological cells of the first cell type from the magnetic beads while at least a majority of the magnetic beads remain fixed in place by the magnetic field.

In another alternative embodiment, greater than 95% (*e.g.,* from about 95% to about 99.999999%, from about 95% to about 99.9999%, from about 98% to about 99.999999%, from about 99% to about 99.999999%, from about 99.5% to about 99.999999%, from about 99.9% to about 99.999999%, etc.) of the magnetic beads are retained in place by the magnetic field while the at least a majority of the biological cells of the first cell type are washed away from the magnetic beads.

In another alternative embodiment, greater than 95% (*e.g.,* from about 95% to about 99.999999%, from about 95% to about 99.9999%, from about 98% to about 99.999999%, from about 99% to about 99.999999%, from about 99.5% to about 99.999999%, from about 99.9% to about 99.999999%, etc.) of the magnetic beads are separated from the biological cells of the first cell type.

In another alternative embodiment, greater than 95% (*e.g.,* from about 95% to about 99.999999%, from about 95% to about 99.9999%, from about 98% to about 99.999999%, from about 99% to about 99.999999%, from about 99.5% to about 99.999999%, from about 99.9% to about 99.999999%, etc.) of the magnetic beads detached from the biological cells of first cell type are separated from the biological cells of first cell type.

In another alternative embodiment, wherein the magnetic beads are from about 0.01 µm to about 3 µm, from about 0.01 µm to about 1 µm, from about 0.02 µm to about 2 µm, from about 0.04 µm to about 3 µm, from about 0.4 µm to about 1.5 µm, from about 0.5 µm to about 3 µm, from about 0.1 µm to about 3 µm, from about 0.2 µm to about 3 µm, from about 0.1 µm to about 20 µm, from about 0.1 µm to about 10 µm, from about 0.2 µm to about 5 µm, from about 0.3 µm to about 15 µm, from about 0.3 µm to about 10 µm, or from about 0.5 µm to about 3 µm in diameter.

Further provided herein, in part, are compositions and methods for separating a first biological material (*e.g.,* a nucleic acid molecule, a protein, a cell, an extracellular vesicle, a virus like particle (VLP), etc.) from a second biological material (*e.g.,* a cell lysate, a culture medium, an IVT reaction mixture, etc.) using bead processing systems. In some instances, such methods may comprise (a) contacting a sample comprising the first biological material and the second biological material with magnetic beads so that the magnetic beads attach to the first biological material through an affinity linkage, the sample and magnetic beads being disposed within a processing bag resting on a platform; (b) moving (*e.g*., raising) a magnet relative to the platform so that the magnet produces a magnetic field that securely fixes in place the magnetic beads relative to the platform, the magnetic beads having first biological material attached thereto; and (c) passing a first fluid through the sample and the magnetic beads while the magnetic field is applied thereto, thereby separating the first biological material from the second biological material. In many instances, the first fluid may be passed through with a force sufficiently strong to wash away the second biological material from the first biological material.

Methods such as those above may further comprise (d) passing a second fluid through the sample and past the magnetic beads while the magnetic field is applied thereto, wherein the second fluid induces release of the first biological material from the magnetic beads.

Methods such as those above may also further comprise (d) lowering the magnet relative to the platform so that the magnetic beads having the first biological material attached thereto are no longer securely fixed in place relative to the platform by the magnetic field of the magnet. In some instances, the magnetic beads may then be collected.

Further, when the first biological material is a nucleic acid molecule, the nucleic acid molecule may be deoxyribonucleic acid (DNA) (*e.g.,* cDNA, genomic DNA, cell-free DNA, single-stranded DNA, double-stranded DNA, plasmid DNA, viral DNA, mitochondria DNA, etc.) or ribonucleic acid (RNA) molecule (*e.g*., messenger RNA (mRNA), ribosomal RNA, transfer RNA, guide RNA, tracr RNA, crRNA, etc.). Additionally, when the first biological material is a mRNA molecule, the mRNA molecule may encode one or more protein of a pathogenic agent (*e.g.,* a virus). mRNA molecules encoding one or more protein of a pathogenic agent may be a component of a vaccine compositions. Thus, provided herein are methods for the production of mRNA molecules that may be used to produce vaccines.

When the first biological material is a cell or cells, these cells may be B cells, red blood cells, monocytes, stem cells, total T cells, helper T helper cells, regulatory T cells, cytotoxic T cells, natural killer cells, dendritic cells, thrombocytes, as well as other cell types. Further, cell may originate from a mammal (*e.g*., human, mouse, rat, pig, cow, gorilla, lama, camel, chimpanzee, etc.).

When the first biological material is a protein, the protein may be an antibody, enzyme, a receptor (*e.g.,* a cell surface receptor), etc. Further, Protein A, Protein G, Protein L, or a functional variant of one of these proteins may be used to form affinity linkages with antibodies purified using methods set out herein.

When the first biological material is an extracellular vesicle, this vesicle may be an exosome, a microvesicles, apoptotic body, etc. Further, the vesicle (*e.g*., exosome) may be generated by a T cell such as a T cell engineered to express a chimeric antigen receptor (*e.g.,* a CD19-CAR). Such vesicles (*e.g.,* exosomes) may exhibit cytotoxic properties towards particular cells *(e.g.,* tumor cells).

Vesicles (*e.g*., exosomes) generated by T cells may be separated from other biological materials using magnetic beads that form an affinity linkage with CD3, CD4 and/or CD8 receptors. In many instances, such methods may employ an anti-CD3 antibody, an anti-CD4 antibody, and/or an anti-CD8 antibody. Further, such antibodies may be, for example, monoclonal antibodies or variable heavy-heavy (VHH) antibodies. Additionally, such antibodies may be attached to a magnetic bead by a biotin or biotin derivative based linkage.

Further provided herein are compositions and methods for producing a purified ribonucleic acid (RNA) molecules. Such methods may include (a) fixing a first magnetic bead in place by a magnetic field, wherein an *in vitro* transcription (IVT) template is linked to the first magnetic bead; (b) contacting the first magnetic bead of step (a) with a reagent mixture suitable for IVT of the template under condition in which IVT occurs, thereby producing an RNA molecule, and (c) separating the RNA molecule from the first magnetic bead, thereby producing the purified RNA molecule.

In some instances, provided are methods further comprising (d) contacting the purified RNA molecule of step (c) with a second magnetic bead under conditions that allows for the purified RNA molecule to remain associated with the second magnetic bead during washing, (e) washing of the second magnetic bead while the second magnet bead is fixed in place by a magnetic field, and (f) releasing the purified RNA molecule from association with the second magnetic bead, thereby producing a highly purified RNA molecule.

In some instances, the IVT template may be produced by polymerase chain reaction (PCR). Further, one or more biotinylated primer may be used in the PCR, resulting in the formation of a biotinylated IVT template. Additionally, a biotinylated IVT template may be attached to the magnetic bead through an interaction between the biotin of the biotinylated IVT template and a group on the magnetic bead with affinity for biotin (*e.g*., avidin, streptavidin, etc.).

In some instances, the IVT template may comprise an open reading frame encoding a protein and a promoter operably connected to the open reading frame.

While the second magnetic bead may associate with RNA in a number of different ways, in some instance, free carboxylic acid groups may be present on the surface of these bead.

Further, purified RNA or highly purified RNA produced as set out herein may be messenger RNA (mRNA), such as mRNA encoding one or more protein of pathogen. Further provided herein are vaccine composition comprising such mRNA or protein encoded by such mRNA.

mRNA used in such vaccine compositions may be contained in, for example, lipid nanoparticles (*see*, *e.g.*, WO 2021/159130 A2).

### BRIEF DESCRIPTION OF THE DRAWINGS

Various embodiments of the present invention will now be discussed with reference to the appended drawings. It is appreciated that these drawings depict only typical embodiments of the invention and are therefore not to be considered limiting of its scope.
Figure 1 is a flow diagram of an instrument workflow system for use in the production of desired T-cells.
Figure 2 is a front perspective view of a magnetic bead processing apparatus used in the system of Figure 1.
Figure 3 is a rear perspective view of the apparatus shown in Figure 2.
Figure 4 is an exploded view of the apparatus shown in Figure 2.
Figure 5 is a front perspective view of the apparatus shown in Figure 2 with the front cover removed.
Figure 6 is a perspective view of a pinch valve shown on the apparatus in Figure 5.
Figure 7 is a perspective view of a rotational assembly and air filter assembly for use with the apparatus in Figure 5.
Figure 8 is an exploded view of the components shown in Figure 7.
Figure 9 is a perspective view of the apparatus shown in Figure 5 with a disposable consumable kit that operates with the apparatus.
Figure 10 is an exploded view of the consumable kit shown in Figure 9.
Figure 11 is a top plan view of the consumable kit mounted on a support panel of the apparatus shown in Figure 9.
Figure 12 is a perspective view of a bead vial retainer of the apparatus shown in Figure 2.
Figure 13 is a perspective view of the bead vial retainer shown in Figure 12 in a rotated position.
Figure 14 is a perspective view of bead vial mounted on a bead vial coupler used with the consumable kit in Figure 10.
Figure 15 is a perspective view of a rocker assembly of the apparatus shown in Figure 2.
Figure 16 is a perspective view a rocker driver of the rocker assembly shown in Figure 15.
Figure 17 is a partially exploded view of the rocker assembly shown in 15 showing a magnet assembly.
Figure 18A is a perspective view of one embodiment of a portion of a magnet of the magnet assembly shown in Figure 17.
Figure 18B is a schematic representation of a Halbach array and how magnets used to generate such an array may be positioned with respect to one another.
Figure 19 is top perspective view of a lift assembly of the rocker assembly shown in Figure 15.
Figure 20 is a cross sectional side view of the rocker assembly shown in Figure 15 showing the lift assembly in a first position.
Figure 21 is a cross sectional side view of the rocker assembly shown in Figure 20 showing the lift assembly in a second position.
Figure 22 is a partially exploded view of the rocker assembly shown in Figure 15 with the cover assembly thereof exploded.
Figure 23 is an enlarged perspective view of a spring assembly shown in Figure 22.
Figure 24 is a perspective view of the spring assembly shown in Figure 23 with a flange thereof butted against a stop.
Figure 25 is a perspective view of the spring assembly shown in Figure 24 with the stop retracted from the flange.

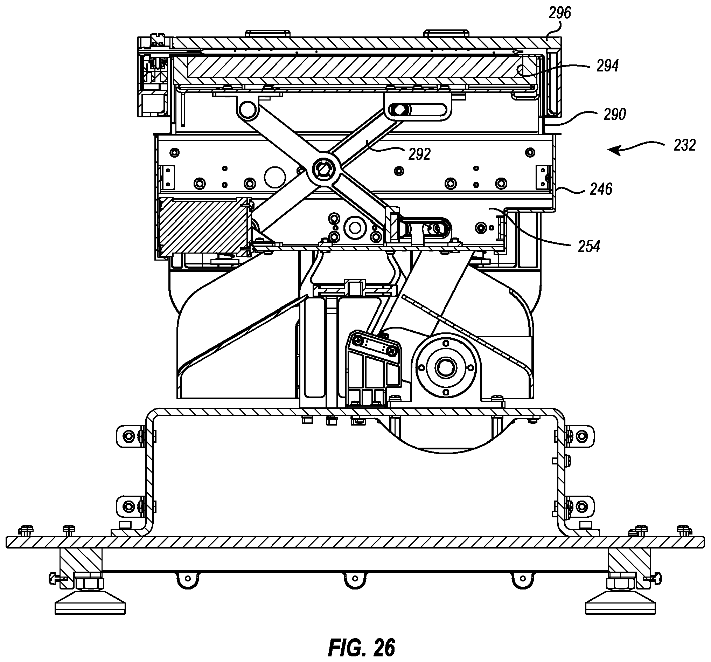

Figure 26 is a cross sectional side view the rocker assembly shown in Figure 15 with a bag therein a first expanded position.
Figure 27 is an enlarged perspective view of a clamp assembly shown in Figure 22.
Figure 28 is a perspective of the clamp assembly shown in Figure 27 coupled with an isolation bag.
Figure 29 is a top plan view of the isolation bag shown in Figure 28.
Figure 30 is a top plan view of the isolation bag shown in Figure 29 has a seal line laterally extending across.
Figure 31 is a top plan view of the isolation bag shown in Figure 29 has a pair of parallel seal line longitudinally extending across;
Figure 32 is a top plan view of the isolation bag shown in Figure 31 wherein the pair of seal lines outwardly flaring.
Figure 33 is a top plan view of an alternative consumable kit that can be used with the magnetic bead processing apparatus shown in Figure 5.
Figure 34 is a top plan view of a bead separation bag of the consumable kit shown in Figure 33.
Figure 35 is a top plan view of the consumable kit shown in Figure 33 mounted on the support panel of the apparatus shown in Figure 5.
Figure 36 is an enlarged perspective view of a clamp assembly for use with the bead separation bag shown in Figure 34.
Figure 37 is a perspective view of the clamp assembly shown in Figure 36 coupled with the bead separation bag.
Figure 38 is a schematic of an exemplary cell processing workflow that contains a series of steps, starting with blood collection, which often will not be part of an automated workflow (hence the dotted line box) and ending with formulation of cells generated during the workflow. The boxes for Step 3 and Step 4 are connected by dotted lines because they separate steps may be combined in a single step in some workflows.
Figure 39 shows a schematic representation of a cell bound to a bead. In this schematic, the antibody is bound to a surface receptor of the cell (labeled "R") by the antigen binding site of the antibody. Further, the Fc region of the antibody is linked to a linker (labeled "L"), which connects the antibody to the bead. "Cleavage Site" refers to a location in the antibody which may be used to cut the antibody, thereby separating the antibody binding sites from the region of the antibody associated with the linker.
Figure 40 is a top plan view of a bead separation bag 600 of the consumable kit shown in Figure 33.
Figure 41 is a front perspective view of an alternative embodiment of the bead processing apparatus shown in Figure 2.
Figure 42 is a rear perspective view of the bead processing apparatus shown in Figure 41.
Figure 43 is a partially exploded rear view of the rocker assembly of the bead processing apparatus shown in Figure 41 showing the mount assembly and the rocker driver thereof.
Figure 44 is partially exploded perspective view of the rocker assembly of Figure 43 showing the housing assembly, lift, shelf, and magnet assembly thereof.
Figure 45 is an exploded perspective of the platform of the rocker assembly shown in Figure 43.
Figure 46 is lateral cross-sectional view of the upper end of the rocker assembly shown in Figure 43.
Figure 47 is an exploded perspective of the cover housing of the rocker assembly shown in Figure 43.
Figure 48 is exploded perspective of the lid of the rocker assembly shown in Figure 43.
Figure 49 is front-to-back cross-sectional view of the upper end of the rocker assembly shown in Figure 43.
Figure 50 is a top perspective view of the rocker assembly shown in Figure 43 with the lid removed and a bag mounted thereon.

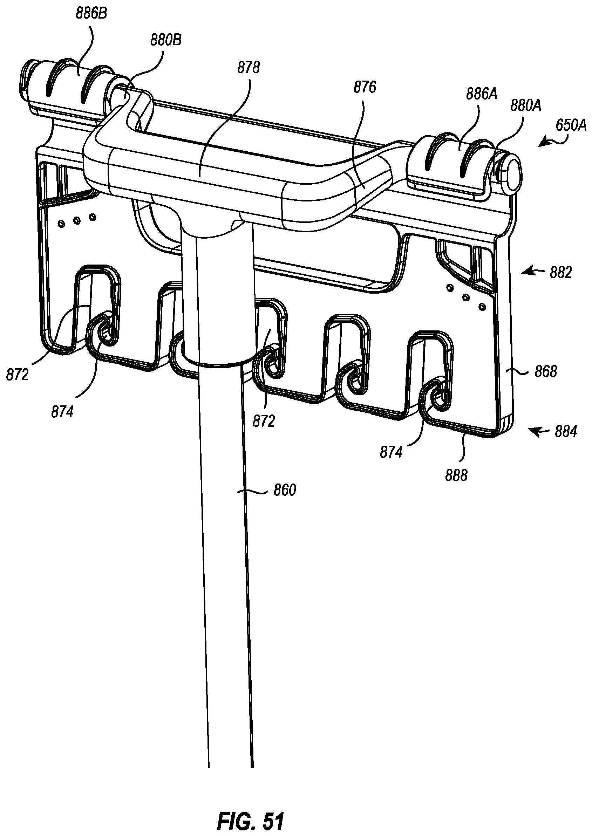

Figure 51 is a perspective view of the upper end of the bag stand of the bead processing apparatus shown in Figure 41.
Figure 52 is a top plan view of one embodiment of a consumable kit that can be used with the bead processing apparatus shown in Figure 41.
Figure 53 is a front elevational view of the consumable kit shown in Figure 52 mounted on the bead processing apparatus shown in Figure 41.
Figure 54 is a perspective view of the tubing restraint of the bead processing apparatus shown in Figure 41.
Figure 55 is a front elevational view of a bead bag with beads that can be used with the consumable kit shown in Figure 53.
Figure 56 is a top plan view of another embodiment of a consumable kit that can be used with the bead processing apparatus shown in Figure 41.
Figure 57 is a front elevational view of the consumable kit shown in Figure 56 mounted on the bead processing apparatus shown in Figure 41.
Figure 58 depicts an exemplary user interface for controlling exemplary magnetic bead processing apparatus, systems and equipment in processing workflows disclosed herein.
Figure 59 depicts an exemplary user interface for controlling exemplary magnetic bead processing apparatus, systems and equipment in processing workflows disclosed herein.
Figure 60 depicts an exemplary user interface for controlling exemplary magnetic bead processing apparatus, systems and equipment in processing workflows disclosed herein.
Figure 61 depicts an exemplary user interface for controlling exemplary magnetic bead processing apparatus, systems and equipment in processing workflows disclosed herein.
Figure 62 depicts an exemplary user interface for controlling exemplary magnetic bead processing apparatus, systems and equipment in processing workflows disclosed herein.
Figure 63 is a schematic of part of a workflow set out in Example 5 and is directed to template immobilization on Streptavidin (StA) beads.
Figure 64 is a schematic of part of a workflow set out in Example 5 is directed to solid-phase *in vitro* transcription (IVT).
Figure 65 is a schematic of a part of a workflow set out in Example 5 is directed to generic capture.

### DETAILED DESCRIPTION OF THE PREFERRED EMBODIMENTS

Before describing the present disclosure in detail, it is to be understood that this disclosure is not limited to particularly exemplified apparatus, systems, methods, or process parameters that may, of course, vary. It is also to be understood that the terminology used herein is only for the purpose of describing particular embodiments of the present disclosure and is not intended to limit the scope of the disclosure.

The following US patent documents are specifically noted for reference: US Provisional Patent Application numbers 63/090,399 (filed October 12, 2020) and 63/137,389 (filed January 14, 2021); U.S. Patent Publication No. 2017/0313772 A1; US Patent Publication No. 2019/0010435, published January 10, 2019 and U.S. Patent Nos. 9,567,346 and 10,196,631, and PCT Publication WO 2021/159130 A2.

The term "comprising" which is synonymous with "including," "containing," "having" or "characterized by," is inclusive or open-ended and does not exclude additional, unrecited elements or method steps.

It will be noted that, as used in this specification and the appended claims, the singular forms "a," "an" and "the" include plural referents unless the content clearly dictates otherwise. Thus, for example, reference to a "port" includes one, two, or more ports.

As used in the specification and appended claims, directional terms, such as "top," "bottom," "left," "right," "up," "down," "upper," "lower," "inner," "outer," "internal," "external," "interior," "exterior," "proximal," "distal" and the like are used herein solely to indicate relative directions and are not otherwise intended to limit the scope of the disclosure or claims.

A "support," as used herein, refers to any nonaqueous soluble material capable of having an antibody attached thereto and includes, without limitation, metals, glass, plastics, co-polymers, colloids, lipids, and the like. Essentially any nonaqueous soluble material capable of retaining an antibody bound or attached thereto.

As used herein, the term "magnetic support" refers to a structural material capable of being attracted to a magnetic field. In many instances, magnetic supports will comprise one or more magnetic metal (*e.g*., iron, cobalt, nickel, etc.). In many instances, a magnetic support will be a magnetic bead. Examples of commercially available magnetic beads are DYNABEADS^{™} Human T-Expander CD3/CD28 (Thermo Fisher Scientific, cat. no. 11141D), CTS^{™} DYNABEADS^{™} CD3/CD28 (Thermo Fisher Scientific, cat. no. 40203D), CTS^{™} DYNABEADS^{™} Treg Xpander (Thermo Fisher Scientific, cat. no. 46000D), and particles contained in the MACS^{™} GMP ExpAct Treg Kit (Miltenyi Biotec, cat. No. 170-076-119).

As used herein, the term "ligand" refers to a molecule that binds to one or more defined population of cells (e.g., members of T cell subpopulations). Ligand binding may be used for isolation of cells to which it binds or may induce a cellular response when bound alone or in conjunction with one or more additional ligands. For example, ligands that bind CD3 and CD28 receptors may be used to activate T cells.

Ligands may bind any cell surface moiety, such as a receptor, an antigenic determinant, or other binding site present on the target cell population. The agent may be a protein, peptide, antibody and antibody fragments thereof, fusion proteins, synthetic molecule, an organic molecule (*e.g.,* a small molecule), or the like. Within the specification and in the context of T cell stimulation, antibodies are used as a prototypical example of such an agent.

As used herein, the term "separation" refers to dividing one component of a mixture from another component of the same mixture.

As used herein, "purified" means that the amount of a material that has been increased in relationship to another materials. By way of example if Protein X is processed in a manner in which the original concentration of Protein X was 1 µg/ml and at the end of the process the final concentration of Protein X is still 1 µg/ ml but the concentration of Protein Y has gone from 2 µg/ ml to 2 µg/ml, then Protein X has been purified. Further, "purification" refers to processes by which materials (e.g., proteins, nucleic acids, etc.) are purified.

As used herein, the term "antibody" includes: (a) any of the various classes or sub-classes of immunoglobulin (*e.g.,* IgG, IgA, IgM, IgD or IgE derived from any animal *e.g.*, any of the animals conventionally used, *e.g.*, sheep, rabbits, goats, mice, camelids, or egg yolk), (b) monoclonal or polyclonal antibodies, (c) intact antibodies or fragments of antibodies, monoclonal or polyclonal, the fragments being those which contain the binding region of the antibody, *e.g*., fragments devoid of the Fc portion (*e.g.*, Fab, Fab', F(ab')2, scFv, V_{H}H antibodies, VHH antibody fragments, as well as other single domain antibodies), the so called "half molecule" fragments obtained by reductive cleavage of the disulphide bonds connecting the heavy chain components in the intact antibody (Fv may be defined as a fragment containing the variable region of the light chain and the variable region of the heavy chain expressed as two chains), and (d) antibodies produced or modified by recombinant DNA or other synthetic techniques, including monoclonal antibodies, fragments of antibodies, "humanized antibodies", chimeric antibodies, or synthetically made or altered antibody-like structures.

Anti-CD3 antibodies that may be used in compositions and methods set out here include those expressed by the following clones: OKT3 (eBioscience, cat. no. 14-0037-82), BC3 (American Type Culture Collection, Deposit No. HB-10166), HIT3a (eBioscience, cat. no. 16-0039-81), F7.2.38 (Thermo Fisher Scientific, cat. no. MA5-12577), MEM-57 (Thermo Fisher Scientific, cat. no. MA1-19454), and UCHT1 (eBioscience, cat. no. 16-0038-81). Anti-CD28 antibodies that may be used in compositions and methods set out here include those expressed by the following clones: IC6 (Graves et al., Transplantation, 91:833-840 (2011), CD28.6 (Thermo Fisher Scientific, cat. no. 16-0288-81), CD28.2 (Thermo Fisher Scientific, cat. no. MA1-10166), 10F3 (Thermo Fisher Scientific, cat. no. CD2800), and BT3 (mIgG2a, antihuCD28, Diaclone, Besanqon, France).

As used herein, the term "VHH antibody" refers to antibodies that consists only of two heavy chains and, thus, lack light chains. Antibodies of this type can be produced by cartilaginous fish and camelids (e.g., alpacas, dromedaries, camels, llamas).

VHH antibodies many be engineered to such that both heavy domains are in the same protein molecule (a single chain antibody) and contain no constant regions. Engineered VHH antibodies may be relatively small in size (*e.g.,* 12 to 15 kDa, about 120 amino acids) in comparison to monoclonal antibodies (*see*, *e.g.*, Harmsen and De Haard, "Properties, production, and applications of camelid single-domain antibody fragments, " Applied Microbiol. Biotech., 77:13-22 (2007), U.S. Patent No. 9,040,666). Such antibodies are also referred to herein as VHH antibodies. VHH antibodies may have one or two antigen binding sites and that may be monovalent or bivalent. Bivalents refer to having binding affinity to two different epitopes.

The term "Protein A," As used herein, refers to the cell surface protein of *Staphylococcus aureus* composed of five highly similar domains (Domains E, D, A, B, and C), where each domain is composed of about 58 amino acids, and functional variants and functional derivatives thereof (including Domain Z). Protein A has the functional activity of binding to antibodies (e.g., IgG molecules) and may be used for antibody purification.

In many instances, Protein A is linked to a solid support (*e.g.,* a bead) when used for antibody purification. This allows for antibodies bound to solid supports to be separated from unbound materials. Further, in many instances (*e.g*., commercial scale antibody production), it is desirable to be able to clean in place (CIP) Protein A bound to solid supports. In many instances, CIP is done using sodium hydroxide (*e.g*., 0.5M NaOH).

While full-length, wildtype Protein A exhibits some resistance to alkaline conditions, a number of modifications to various Protein A domains to enhance alkaline resistance (*see, e.g.,* Linhult et. al., "Improving the Tolerance of a Protein A Analogue to Repeated Alkaline Exposures Using a Bypass Mutagenesis Approach," Proteins, 55:407-416 (2004)). Further, alkaline sensitivity/resistance vary with the individual Protein A domains. Along these lines, Domain C has been found to be fairly alkaline resistant.

Proteins that contain repeats of one or more Protein A domains (*e.g*., five repeats of wild-type Domain B) that retain at least 50% of the binding capacity of wild-type Protein A molecules on a per domain basis are also considers to be Protein A molecules. Also considers to be Protein A molecules are proteins containing domains that share at least 90% sequenced identity to any stretch of 20 amino acids of any one of wildtype Protein A Domains E, D, A, B, and C and that retain at least 50% of the binding capacity of wild-type Protein A molecules on a per domain basis. By way of example, if wild-type Protein A molecules bind 50 units of antibody under a specified set of conditions and Protein A derivative with four repeated domains binds 20 units of antibody under the same conditions, then the Protein A derivative would be said to have 50% of the binding capacity of wild-type Protein A molecules.

As used herein, the term "virus-like particle", or VLP, refers to viral protein complexes that resemble viruses but are replication deficient. VLPs can be used to deliver nucleic acid molecules to cells and include naturally occurring replication deficient viral protein complexes such as adeno-associated virus. VLPs may be enveloped or unenveloped. One example of enveloped lentiviral particles are those produced using the BLOCK-IT^{™} Lentiviral RNAi Zeo GATEWAY^{™} Vector Kit (Thermo Fisher Scientific, cat. no. V48820).

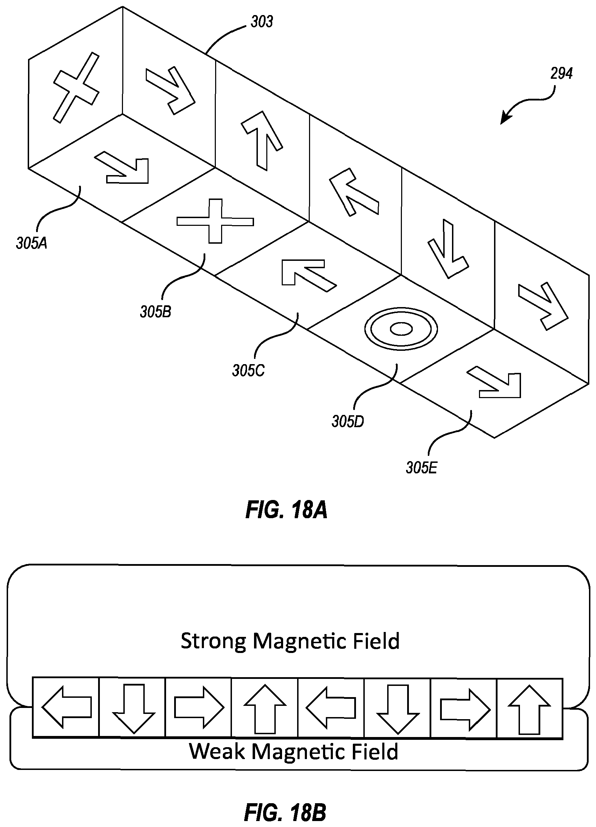

As used herein, a "Halbach array" is an arrangement of magnets (see Figures 18A and 18B) that exhibits an enhanced magnetic field on one side of the array while at least partially cancelling out the magnetic field to near zero on the other side of the array. Some advantages of Halbach arrays are that the magnetic field is stronger on one side of the array and is weak on the other side of the array. This allows for the use of a smaller and more lightweight magnet assembly to achieve desired magnetic forces. The focusing of the magnetic field on one side of a Halbach array allows for magnetic field "confinement", thereby lessening magnetic field interference and generating a magnetic field gradient conducive the magnetic particle capture.

Where possible, like numbering of elements have been used in various figures. Furthermore, alternative configurations of a particular element may each include separate letters appended to the element number. Accordingly, an appended letter can be used to designate an alternative design, structure, function, implementation, and/or embodiment of an element or feature without an appended letter. For instance, an element "80" may be embodied in an alternative configuration and designated "80a." Similarly, multiple instances of an element and or sub-elements of a parent element may each include separate letters appended to the element number. In each case, the element label may be used without an appended letter to generally refer to all instances of the element or any one of the alternative elements. Element labels including an appended letter can be used to refer to a specific instance of the element or to distinguish or draw attention to multiple uses of the element.

Various aspects of the present devices, systems, and methods may be illustrated with reference to one or more exemplary embodiments. As used herein, the term "embodiment" means "serving as an example, instance, or illustration," and should not necessarily be construed as preferred or advantageous over other embodiments disclosed herein.

Various aspects of the present devices and systems may be illustrated by describing components that are coupled, attached, and/or joined together. As used herein, the terms "coupled", "attached", "connected" and/or "joined" are used to indicate either a direct connection between two components or, where appropriate, an indirect connection to one another through intervening or intermediate components. In contrast, when a component is referred to as being "directly coupled", "directly attached", "directly connected" and/or "directly joined" to another component, there are no intervening elements present.

Unless defined otherwise, all technical and scientific terms used herein have the same meaning as commonly understood by one of ordinary skill in the art to which the present disclosure pertains. Although a number of methods and materials similar or equivalent to those described herein can be used in the practice of the present disclosure, the preferred materials and methods are described herein.

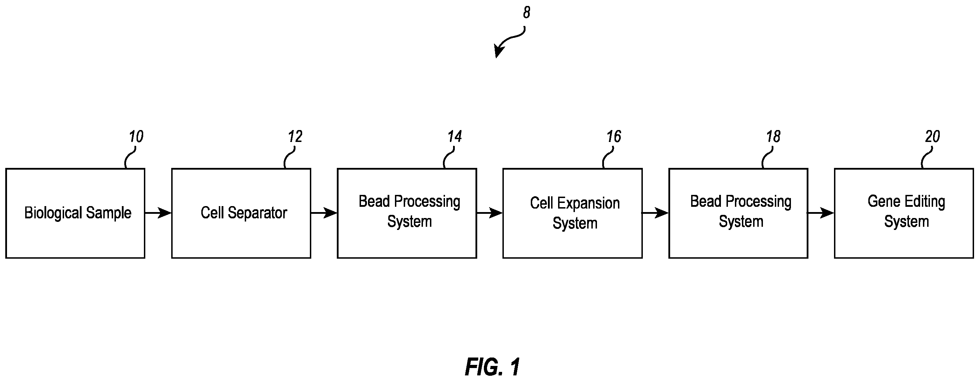

Depicted in Figure 1 is one embodiment an instrument workflow system 8 for use in the production of desired T-cells. Initially, a biological sample 10 is provided that includes the desired T-cell. Biological sample 10 typically comprises a human blood sample or a prepared biological suspension comprised of a mixture of cells and a growth medium. The cells in the biological suspension may have been grown, such as in a bioreactor, or have been otherwise processed. Initially, biological sample 10 can be processed through a cell separator 12 so as to remove at least a portion of unwanted cells. For example, if biological sample 10 is a human blood sample, cell separator 12 can separate out the white blood cells containing the desired T-cells from the red blood cells. One example of cell separator 12 is a GIBCO^{™} CTS^{™} ROTEA^{™} Counterflow Centrifugation System available from Thermo Fisher Scientific. Depending on the composition of biological sample 10, other cell separation processes and equipment can also be used. Further, cell separators may also be used in later steps, for example, to wash cells that have been released from magnetic particles and/or to wash magnetic particles with or without cells attached thereto. In other embodiments, depending on the form of the biological suspension, cell separator 12 may not be required.

The cell culture leaving cell separator 12 is then processed through a first bead processing system 14 for isolation and activation of the desired T-cells. That is, as will be discussed below in detail, first bead processing system 14 functions to mix the cell culture with magnet beads that are covalently coupled with specific antibodies (e.g., anti-CD3, anti-CD28, anti-CD137, etc.) so that the magnetic beads will automatically bind to and activate the desired T-cells. The T-cells coupled to the magnetic beads are then held in an isolation bag by a magnetic field while the remaining unwanted cells are washed away.

The isolated T-cells bound to the magnetic beads are then washed and released from the magnetic field and transferred to a cell expansion system 16. Cell expansion system 10 can comprise a conventional bioreactor system where the T-cells are grown in a growth medium under pre-establish conditions. Cell expansion system 16 can comprise a rocker or continuous stir bioreactor or any other bioreactor system, such as those using a gas permeable bag, where cells can be grown. Once the T-cells have grown to a desired density or reached another preestablished condition, the T-cells will either automatically separate from the magnetic beads or a detach reagent can be added to the suspension that causes separation of the magnetic beads from the T-cells. For example, activated T-cells will naturally downregulate CD3, and will therefore detach from the magnetic beads typically at around five days. In some embodiments, the suspension of T-cells, magnetic beads, and media can be manual or mechanical manipulated, such as within a bag, to help facilitate detachment of the T-cells from the magnetic beads. The suspension of T-cells, magnetic beads, and media is then transferred to a second bead processing system 18 where the desired T-cells are separated from the magnetic beads, as set out below in more detail. As discussed below in further detail, in one embodiment, bead processing system 18 can comprise reusing the apparatus of bead processing system 14 with a different consumable kit.

Finally, the T-cells separated from the magnetic beads can be transferred to a gene editing system 20 for genetically modifying the cells as desired. For example, DNA can be incorporated into the cells by, for examples, homologous recombination and/or non-homologous end joining. Gene editing may be mediated by site specific cleavage of intracellular nucleic acid (e.g., chromosomal nucleic acid). Further downstream processing and/or packaging of the T-cells can also be provided.

Instrument workflow system 8 can comprise a closed, sterile system where the biological sample can continuously flow between each component of the system under sterile conditions. Alternatively, the biologic sample can be separately transferred between each or select components of system 8. Each component of system 8 can be programmed to run automatically or can be operated manually. Depending on specific conditions, specific components of instrument workflow system 8 can be eliminated or additional components can be added.

A detailed description of the components and operation of bead processing systems 14 and 18 will now be described. In one embodiment, bead processing systems 14 and 18 can comprise the same reusable hardware but use different disposable, consumable components to achieve their different intended functions. This design has the advantage of reducing equipment cost and minimizing needed storage space. However, in other embodiments, separate types of bead processing systems can be used.

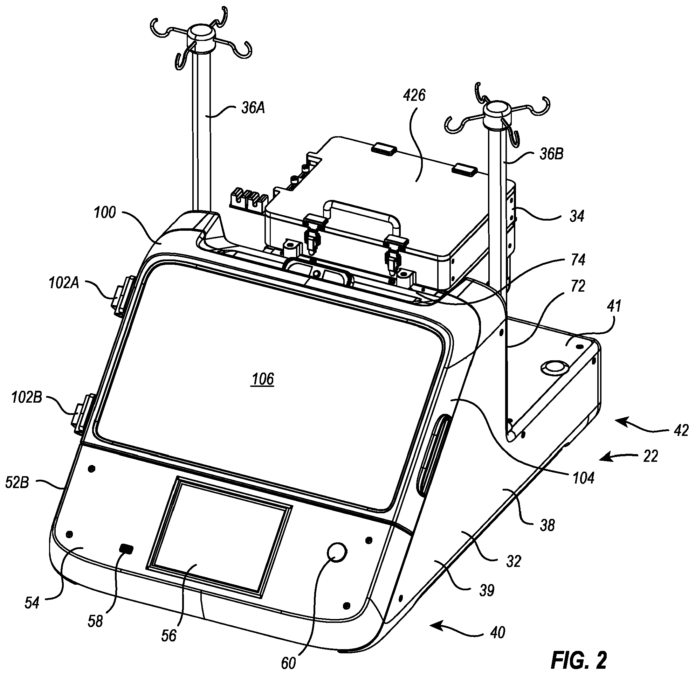

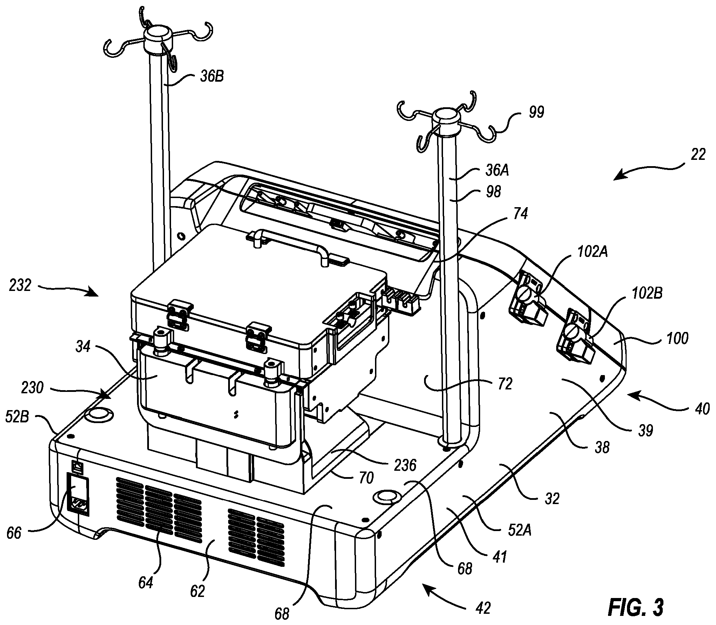

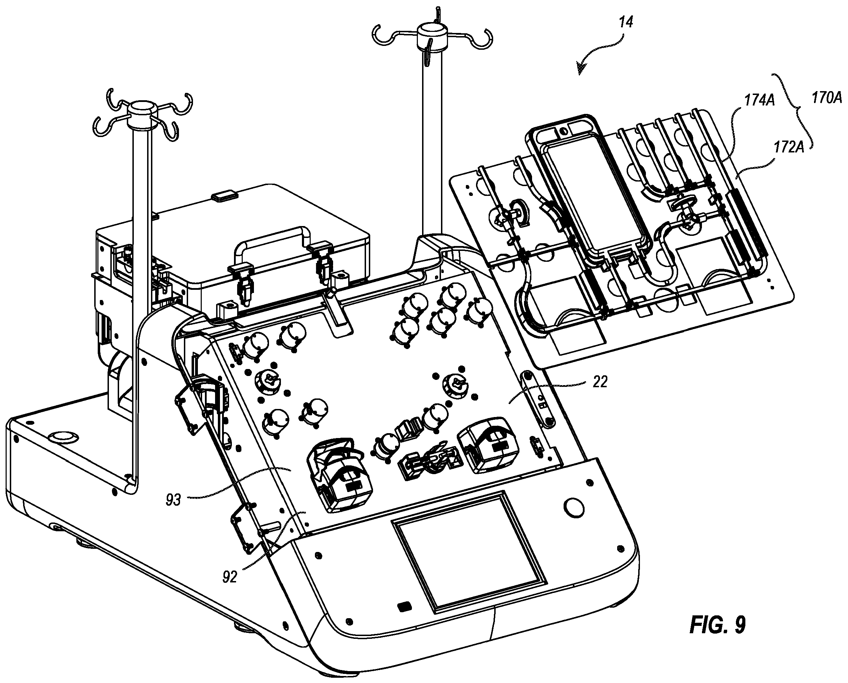

Bead processing system 14 comprises a reusable bead processing apparatus 22, as depicted in Figures 2 and 3, that may be used with a single use, disposable consumable kit 170A, as depicted in Figure 9. Returning to Figures 2 and 3, in general, bead processing apparatus 22 includes a base assembly 32, a rocker assembly 34 mounted on base assembly 32 and configured to selectively rock relative to base assembly 32, and a pair of bag stands 36A and 36B upstanding from base assembly 32 on opposite sides of rocker assembly 34.

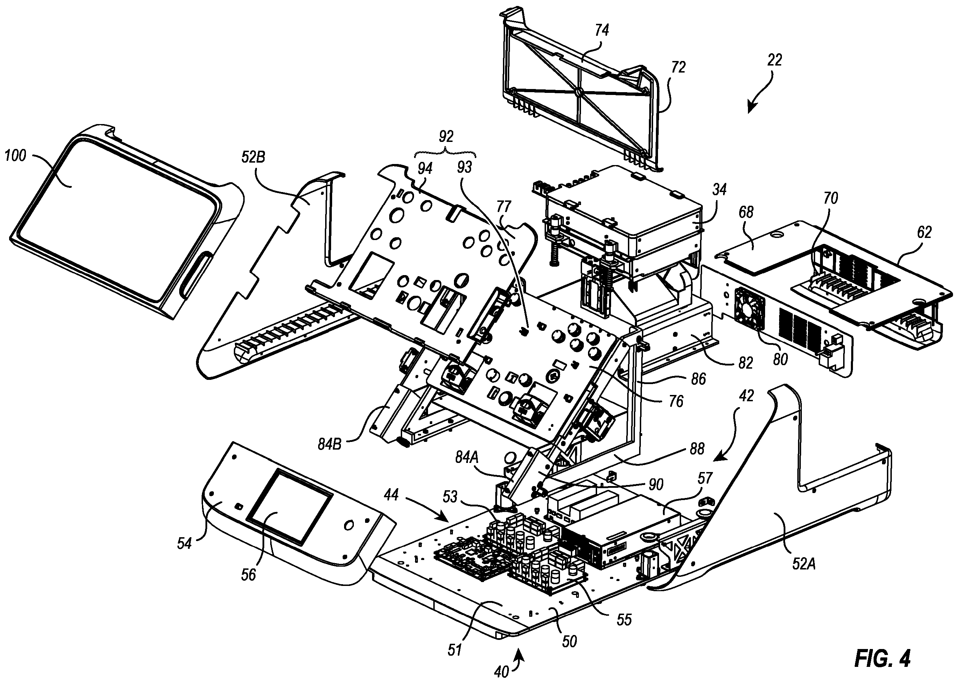

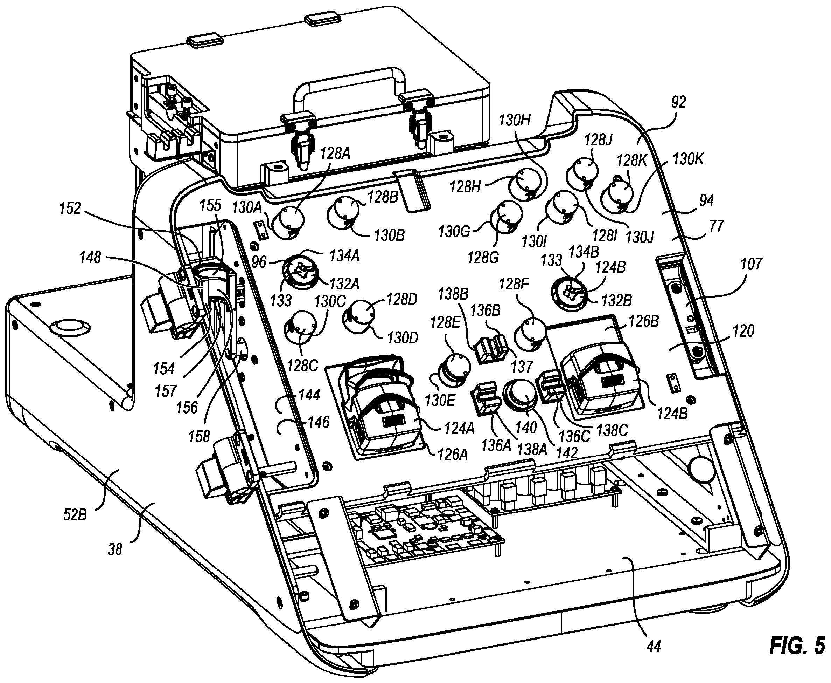

Base assembly 32 comprises a housing 38 that extends between a front end 40 and an opposing back end 42 and that bounds a compartment 44 (Figure 5). Although housing 38 can have a variety of different configurations, in one embodiment, as depicted in Figure 4, housing 38 includes a floor panel 50, that is typically horizontally disposed during use, having a top face 51 extending between opposing ends 40 and 42. Disposed on top face 51 so as to be housed within compartment 44 is electrical circuitry 53 that controls operation of bead processing apparatus 22 and that includes a programmable computer processor 55 and non-transitory memory. Other conventional electronics used in the operation of bead processing apparatus 22 can also be disposed on floor panel 50. For example, an electrical transformer 57 can be disposed on floor panel 50 for receiving and converting electricity delivered to bead processing apparatus 22.

Housing 38 further includes opposing side panels 52A and 52B that upstand from and extend along opposing sides of floor panel 50. A front panel 54 is disposed at front end 40 of floor panel 50 and extends between side panels 52A and 52B. In one embodiment, front panel 54 is sloped at an angle relative to horizontal which is typically in a range between about 25° and 65° and more commonly in a range between 35° and 55°. Other angles can also be used. A screen 56 is disposed on front panel 54 and is in electrical communication with electrical circuitry 53. In one embodiment, screen 56 can comprise a touch screen to enable user input for operating bead processing apparatus 22. In alternative embodiments, screen 56 can simply comprise a display screen for displaying operation of bead processing apparatus 22. Optionally, an electrical interface port 58 and an activation switch 60 (Figure 2) can be disposed on front panel 54 and communicate with electrical circuitry 53. Electrical interface port 58 can be used for controlling, programming, and/or gather data from electrical circuitry 53. Activation switch 60 can be used for turning bead processing apparatus 22 on and off and/or for switching bead processing apparatus 22 between different modes or stages of operation. In alternative embodiments, interface port 58 and/or activation switch 60 can be disposed at other locations on bead processing apparatus 22 or housing 38.

With continued reference to Figures 3 and 4, housing 38 further includes a back panel 62 upstanding from floor panel 50 at back end 42 and extending between side panels 52. Vent openings 64 extend through back panel 62 and are used for cooling electrical circuitry 53 and other components within compartment 44. As also shown in Figure 4, a fan 80 can be disposed within compartment 44 adjacent to vent openings 64. Fan 80 is electrically coupled to electrical circuitry 53 and is used to help cool electrical components of bead processing apparatus 22.

Also formed on back panel 62 is a power inlet 66 configured for coupling with an electrical cable for providing electrical power to bead processing apparatus 22. Projecting forward toward front end 40 at an upper end of back panel 62 is a shelf panel 68. Shelf panel 68 is typically disposed horizontally and thus is commonly disposed parallel to floor panel 50 and/or perpendicular to back panel 62. Shelf panel 68 has an opening 70 passing therethrough in which rocker assembly 34 is disposed. Upstanding from a forward end of shelf panel 68 is a riser panel 72. Riser panel 72 terminates at a top edge 74 of bead processing apparatus 22 and extends between side panels 52. In one embodiment riser panel 72 can be disposed vertical so as to be parallel to back panel 62 and/or perpendicular to shelf panel 68.

Bag stands 36A and 36B upstand from shelf panel 68 on opposite sides of rocker assembly 34. Each bag stand 36 comprises a pole 98 having an upper end with a plurality of catches 99 formed thereat. Pole 98 can have a fixed length or be configured to telescopically lengthen or shorten. Catch 99 are configured to hold and support flexible bags housing a fluid and can be in the form of a hook or other structure that can retain a bag.

A stand 82 is disposed within compartment 44 and is mounted on floor panel 50. In one embodiment, stand 82 can be U-shaped and positioned to span over transformer 57 or other components disposed on floor panel 50. Rocker assembly 34 is disposed on stand 82 so as to pass through opening 70 on shelf panel 68. Further disposed within compartment 44 and mounted on floor panel 50 are a pair of triangular brackets 84A and 84B. Brackets 84 are disposed forward of stand 82 and extend to or toward front end 40. In one embodiment, each bracket 84 includes a vertical back rail 86, a horizontal base rail 88 that is secured to floor panel 50, and a front rail 90 that extends at an angle between a forward end of base rail 88 and an upper end of back rail 86. As discussed below in more detail, spanning between brackets 84, and more specifically between front rails 90, is a support panel 92. Support panel 92 is typically disposed at an angle relative to horizontal which is typically in a range between about 25° and 65° and more commonly in a range between 35° and 55°. Other angles can also be used.

With reference to Figures 2-4, bead processing apparatus 22 also includes an optional cover panel 100 that is hingedly mounted to side panel 52B by hinges 102A and 102B and extends over support panel 92. In one embodiment, cover panel 100 spans between opposing side panels 52 and is disposed between front panel 54 and top edge 74 of riser panel 72. Cover panel 100 can be disposed within the same angle range as previously discussed with regard to front panel 54 and can be disposed within the same plane as front panel 54. In one embodiment, cover panel 100 includes a perimeter frame 104 that encircles a transparent window 106. Window 106 is typically comprised of a transparent polymeric material but could be formed from glass. Cover panel 100 can be freely rotated between a closed position, as shown in Figure 2, wherein cover panel 100 covers support panel 92, and an open position wherein support panel 92 is freely and openly exposed.

For ease of discussion, Figure 5 shows bead processing apparatus 22 with cover panel 100 and front panel 54 removed so that support panel 92 is freely exposed. As shown in this figure, a latch 107 can be used to secure cover panel 100 in the closed position. As depicted in Figures 4 and 5, in one embodiment support panel 92 comprises a base panel 93 that extends between and is secured to brackets 84 and an overlay panel 94 that sits on top of and is secured to base panel 93. Base panel 93 has a top face 76 on which overlay panel 94 is disposed. Overlay panel 94 has a top face 77 which also forms the top face of support panel 92. As depicted, a plurality of pinch valves, pumps, and other features are mounted on and upstand from support panel 92. More specifically, pinch valves, pumps, and other features are mounted directly to base panel 93 and project through openings formed on overlay panel 94. For example, as depicted in Figure 5, mounted on and projecting from base panel 93 is a first pump 124A and a second pump 124B. Pumps 124A and 124B project through openings 126A and 126B, respectively, on overlay panel 94 so as to project out beyond the top face 77 of overlay panel 94. In one embodiment, pumps 124A and 124B are peristaltic pumps or other types of pumps that can pump liquid through a tube without directly contacting the fluid. Also mounted on and projecting from base panel 93 are a plurality of spaced apart pinch valves 128A-K. Pinch valves 128A-K project through corresponding openings 130A-K, respectively, on overlay panel 94 so as to project out beyond the top face 77 of overlay panel 94.

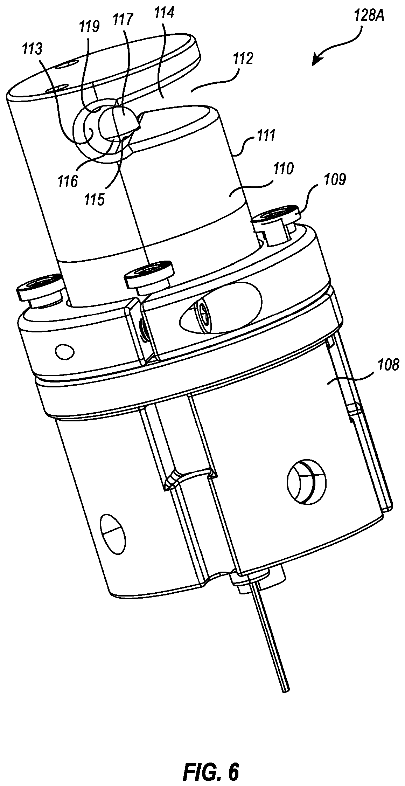

Depicted in Figure 6 is an enlarged view of pinch valve 128A. Pinch valve 128A include a solenoid valve 108 that is controlled by electrical circuitry 53 (Figure 4) and is mounted to the bottom side of base panel 93, *i.e.,* to the bottom side of support panel 92, by fasteners 109. A body 110, typically having cylindrical configuration, is coupled with solenoid valve 108 and outwardly passes through base panel 93 and overlay panel 94. Body 110 has an encircling side surface 111 with a slot 112 that is recessed into side surface 111 and passes laterally through body 110. Slot 112 includes a C-shaped channel 113 that is centrally formed on body 110 and an outwardly flaring mouth 114 that extends from C-shaped channel 113 to side surface 111. The flaring of mouth 114 helps to guide tubing into channel 113, as discussed below. A bore 115 centrally passes through body 110 from solenoid valve 108 to C-shaped channel 113. A shaft 116 is disposed within bore 115 and terminates at a rounded head 117. Solenoid valve 108 controls movement of shaft 116 within bore 115. Thus, as discussed below in further detail, when a flexible tube is disposed within and passing through C-shaped channel 113, solenoid valve 108 can selectively move shaft 116 between a lowered position and a raised position. In the lowered position, shaft 116 is at least partially removed from C-shaped channel 113 so as to permit fluid to pass through the tube. In the raised position, shaft 116 is advanced into C-shaped channel 113 so that shaft 116 pinches the tube between rounded head 117 and a top surface 119 of C-shaped channel 113, thereby closing the tube and preventing fluid from flowing therethrough. It is appreciated that other conventional types of pinch valves that can selectively open and close a flexible tube can also be used. For example, pneumatic pinch valves, such as available from Emerson Electric Co., could also be used. The remaining pinch valves 128B-K are typically identical to pinch valve 128A and thus like elements between the different pinch vales are identified by like reference characters.

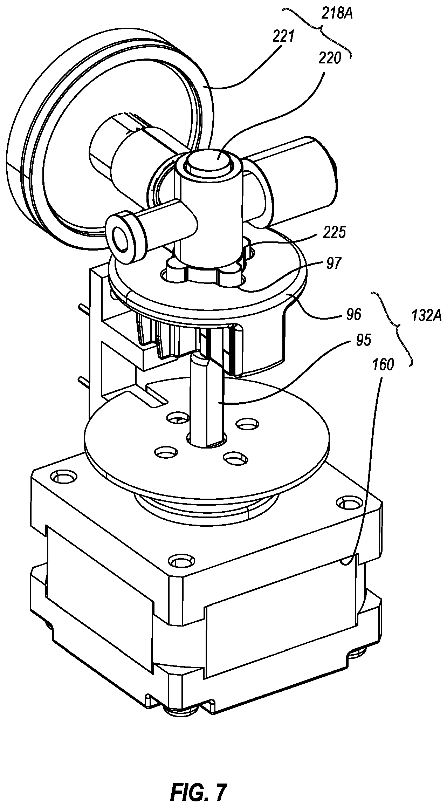

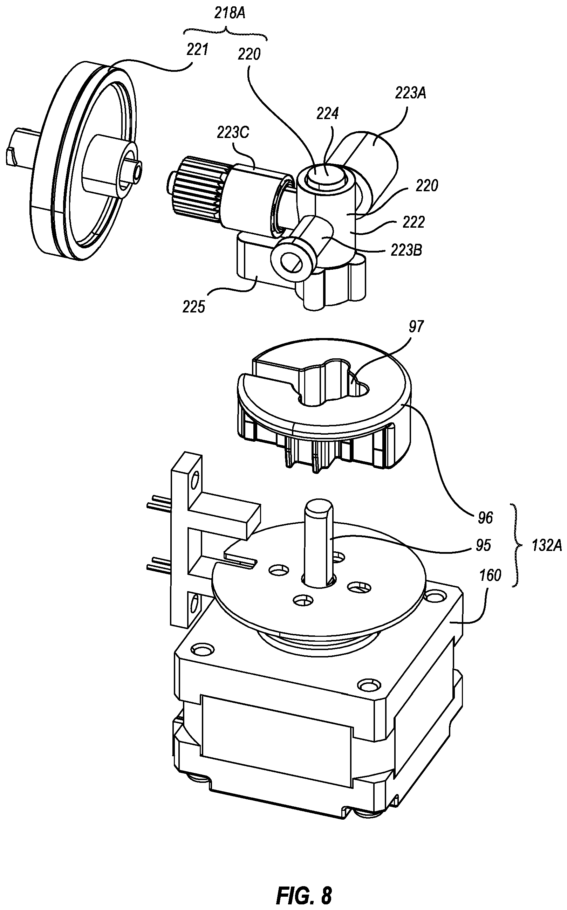

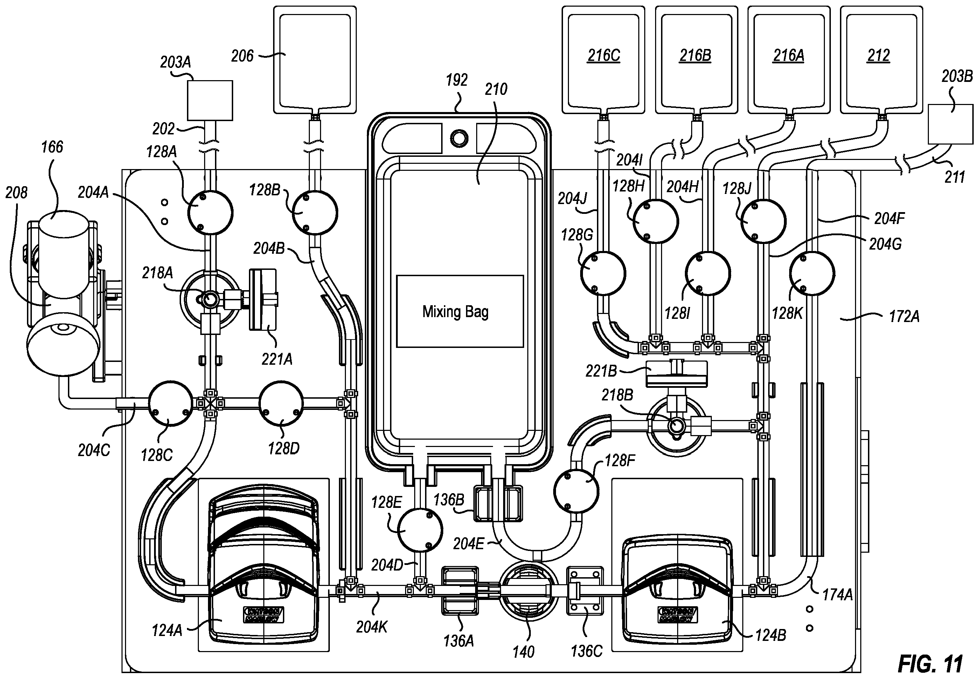

Returning to Figure 5, further mounted on support panel 92 are a pair of rotational assemblies 132A and 132B that are configured to receive and operate corresponding air filter assemblies 218A and 218B, respectively (Figure 11). Depicted in Figures 7 and 8 is rotational assembly 132A coupled with air filter assembly 218A. Rotational assembly 132A comprises a drive motor 160 that selectively rotates a drive shaft 95. In many instances, air filters used will be designed to prevent the passage of biological agents to maintain a sterile environment on one side of the filter. Drive motor 160 is controlled by electrical circuitry 53 (Figure 4). In one embodiment, drive motor 160 is a stepper motor that can selectively rotate drive shaft 95 in opposite directions. Mounted on a free end of drive shaft 95 is receiver 96 having a keyed socket 97 formed thereon.

Air filter assembly 218A comprises a stopcock 220 having an air filter 221 coupled thereto. In the depicted embodiment, stopcock 220 comprises a two-way stopcock that includes a stem 222 having ports 223A, 223B, and 223C outwardly projecting therefrom and fluid coupled therewith. A valve 224 is rotatably disposed within stem 222 and has an outwardly projecting handle 225 disposed therein. Rotation of handle 225 to a first position orientates valve 224 so that fluid can flow between ports 223A and 233B. Rotation of handle 225 to a second position orientates valve 224 so that fluid can only flow between ports 223A and 233C. Air filter 221 is coupled to port 223C and typically comprises an air sterilizing filter. In one embodiment, air filter 221 can have an average pore size of 0.2µ or smaller. As will be discussed below in more detail, handle 225 is configured to removably fit within keyed socket 97 of receiver 96 so that a secure engagement is formed therebetween. As a result, rotation of receiver 96 by drive motor 160 can facilitate rotation of handle 225 relative to stem 222 between the two operating positions. Rotational assembly 132B can have the same configuration and operation as rotational assembly 132A while air filter assembly 218B can have the same configuration and operation as air filter assembly 218A. As such, like elements between components are identified by like reference characters.

During assembly, drive motor 160 of rotational assemblies 132A and 132B can be secured to base panel 93/support panel 92 so that receivers 96 are received within corresponding openings 134A and 134B, respectively, of overlay panel 94. In this embodiment, as shown in Figure 5, receivers 96 can, but it is not necessary, project out beyond the top face 77 of overlay panel 94/support panel 92.

With continued reference to Figure 5, bubble sensors 136A, 136B, and 136C are mounted on support panel 92 between pumps 124A and 124B. Each bubble sensor 136 has a groove 137 formed thereon that is configured to securely receive a tube. In one embodiment, grooves 137 can have a C or U-shaped configuration. Bubble sensors 136 are mounted on base panel 93 and project through corresponding openings 138A, 138B, and 138C, respectively, on overlay panel 94 so as to project out beyond the top face of overlay panel 94, support panel 92. As will be discussed below in more detail, bubble sensors 136 are controlled by electrical circuitry 53 and discern between gas flow and liquid flow in the tubing used in the operation of bead processing system 14. Examples of bubbles sensors that can be used in the present disclosure include ultrasonic bubble detectors such as Introtek AD9-0075-E04 produced by INTROTEK INTERNATIONAL and those available from Sonotec GMBH. Other bubble sensors can also be used.

Finally, a pressure sensor 140 can be mounted on support panel 92/base panel 93 and project through or be aligned with a corresponding opening 142 on overlay panel 94. Pressure sensor 140 is shown disposed between bubble sensors 136A and 136C but can be disposed at other locations. As will also be discussed below in more detail, pressure sensor 140 is used to detect the pressure of the fluid within the tubing of bead processing system 14. Monitoring the pressure can help detect operational failures and prevent over pressurizing the tubing and/or bags of consumable kit 170A. An example of a pressure sensor that can be used in the present disclosure is the PRO-3000-904 produced by INTROTEK INTERNATIONAL.

It is noted that overlay panel 94 primarily functions to cover fasteners and/or other element of disposed on base panel 93 for which access is not needed during normal operation. As such, in alternative embodiments, overlay panel 94 can be eliminated, such as is shown in Figure 9. Support panel 92 can thus comprise just base panel 93 or the combination of base panel 93 and overlay panel 94.

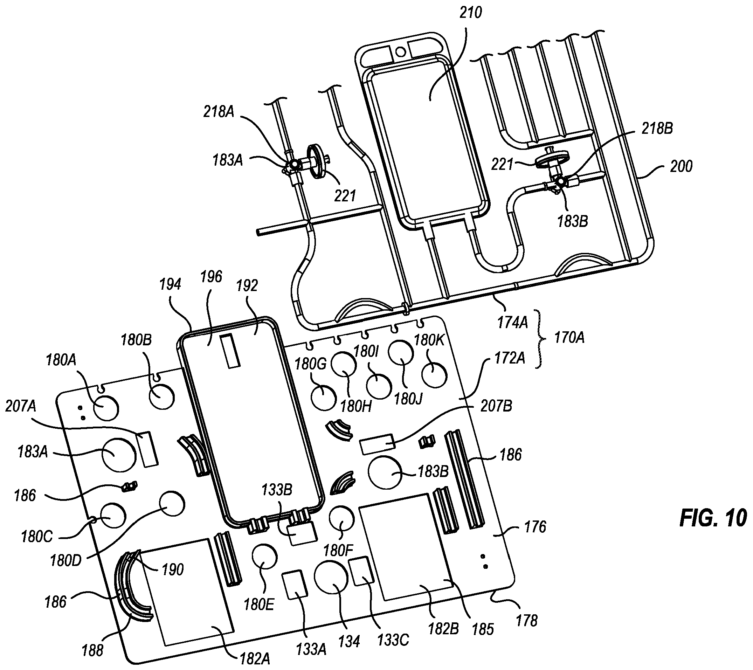

With continued reference to Figure 9, a single use, disposable consumable kit 170A can be used with bead processing apparatus 22 for isolating and activating the T-cells suppled to bead processing apparatus 22. Consumable kit 170A comprises a tray 172A and a line set 174A mounted thereon. Consumable kit 170A is configured to removably nest on top of support panel 92 so that line set 174A can engage or otherwise interact with various mechanical components disposed on support panel 92 as discussed above. To that end, as depicted in Figure 10, tray 172A has a top face 176 and an opposing bottom face 178 that are typically disposed in parallel alignment and has a thickness therebetween that is typically in a range between 0.2 cm and 1 cm with between 0.2 cm and 0.6 cm being more common. Tray 172A is typically rigid, *e.g.,* is more rigid than the tubing of line set 174A, and is commonly made of a polymer such as polycarbonate-ABS (PC/ABS), acrylonitrile butadiene styrene (ABS), or polypropylene (PP). A plurality of openings 185 extend through tray 172A between faces 176 and 178. For example, such openings 185 include openings 180A-180K that are positioned and configured on tray 172A so that pinch valves 128A-128K (Figure 11) align with and pass through openings 180A-180K when support tray 172A is disposed on support panel 92. Likewise, openings 182A and 182B extend through tray 172A and are positioned and configured so that pumps 124A and 124B (Figure 11) align with and pass therethrough, respectively, when tray 172A is disposed on support panel 92. In addition, openings 183A and 183B extend through tray 172A and are positioned and configured to align with rotational assemblies 132A and 132B while openings 133A-133C are positioned and configured align with bubble sensors 136A-136C, respectively. Finally, an opening 134 extends through tray 172A and is positioned and configured to align with pressure sensor 140.

Tray 172A also includes a plurality of spaced apart tube restraints 186 mounted on and outwardly projecting from top face 176. Although tube restraints 186 can have a variety of different configurations, in the depicted embodiment, each tube restraint 186 comprises a base 188 having a slot 190 extending therethrough. Slot 190 typically has a C-shaped or U-shaped transverse cross section that is configured to snuggly but removably received a tubing 200 of line set 174A. Tube restraints 190 function to contour and hold tubing 200 and can be formed at any desired location on tray 172A where line set 174A travels. However, tube restraints 186 are most commonly placed at or adject to where tubing 200 is being bent or manipulated. Tray 172A also includes a bag restraint 192 centrally upstanding from tray 172A. In the depicted embodiment, bag restraint 192 comprises a perimeter wall 194 upstanding from tray 172A and encircling a receiving area 196. Receiving area 196 is configured to receive a mixing bag 210 of line set 174A so that mixing bag 210 is supported and retained by perimeter wall 194.

As depicted in Figures 10 and 11, line set 174A generally comprises flexible tubing 200 fluid coupled with a plurality of bags and with air filter assemblies 218A and 218B. During production, line set 174A and tray 172A can be pre-assembled and sterilized as a unit. Alternatively, line set 174A and tray 172A can be independently sterilized and then coupled together. It is appreciated that during production, line set 174A is formed so that it is sealed closed. Line set 174A, with or without tray 172A, can be then sterilized through an irradiation process. In turn, fluid coupling with the other components of production system 8 (Figure 1) is produced through sterile fluid connections.

During use, the assembled consumable kit 170A is placed on top of support panel 92 so that the mechanical components on support panel 92 are aligned with the related openings formed on tray 172A. As discussed below in more detail, tubing 200 is then manipulated to couple with each of pinch valves 128, pumps 124, bubble sensors 136 and pressure sensor 140 while air filter assemblies 218 are mounted on rotational assemblies 132.

Tubing 200 can have a variety of different configurations. In one embodiment, tubing 200 has an inside diameter in a range between 2.0 mm and 5.0 mm or between 2.5 and 5.0 mm or more commonly in a range between 3.0 mm and 5.0 mm. Other ranges can also be used. Tubing 200 is made of material that enables tubing 200 to be easily bent without plastic deformation and enables tubing 200 to be selectively pinched closed (such as by using pinch valves 128) to prevent fluid flow therethrough but will resiliently rebound to an open position to allow fluid to flow therethrough when the pinching force is released. Commonly, tubing 200 can be bent over an angle of at least 90°, 180° or 360° without plastic deformation. Examples of material that tubing can be made of include silicone, polyvinyl chloride (PVC) and thermoplastic elastomers (TPE) such as C-FLEX formulation 374 tubing. Different sections of tubing 200 can also be made of different materials depending on their intended function. In the present embodiment, tubing 200 is sized so that it can be received within slot 112 of pinch valves 128 (Figure 6) for selective pinching closed and opening through the movement of shaft 116, as previously discussed.

Tubing 200 comprises a plurality of sections that are either integrally connected together or are connected together by fittings. For example, with reference to Figure 11, tubing 200 comprise a tubing section 204A having a first end that terminates at an inlet end 202. Inlet end 202 is intended to couple with cell separator 12 (Figure 1) or some other source for delivering a cell culture, including desired T-cells, into line set 174A. For example, inlet end 202 can have a closed connector 203 formed thereat, such as a sterile connector, or can simply be sealed closed, such as by welding, so that inlet end 202 can be cut and then sealed onto another tubing section though which the cell culture is delivered. Section 204A has air filter assembly 218A coupled thereto. During assembly for use, section 204A is coupled with pinch valve 128A while air filter assembly 218A is coupled with rotation assembly 132A (Figure 5), as previously discussed. With reference to Figure 10, it is noted that narrow slots 207A and 207B are formed on tray 172A adjacent to opening 183A and 183B, respectively. Slots 207 are formed to receive air filters 221 so as to help prevent air filters 221 and stems 220 (Figure 8) from turning as handle 225 is rotated by rotational assembly 132.

Returning to Figure 11, line set 174A also includes a tubing section 204B having first end that is connected to and communicates with an isolation bag 206. Isolation bag 206 is typically permanently secured to tubing section 204B but can be coupled through a connector. In either event, isolation bag 206 and tubing section 204B are typically coupled together prior to irradiation. The configuration of isolation bag 206 is discussed below in more detail but is configured to be secured on rocker assembly 34 (Figure 2). Isolation bag is one example of a processing bag. During assembly for use, tubing section 204B is coupled with pinch valve 128B. A tubing section 204C of tubing 200 has a first end that is connected with a bead vial coupler 208 and an opposing second end that connects with both tubing sections 204A and 204B through the use of fittings. Bead vial coupler 208 will be discussed below in more detail. Tubing section 204C couples with pinch valves 128C and 128D.

Tubing 200 also includes tubing sections 204D and 204E each having a first end that is fluid coupled with a mixing bag 210 positioned within bag restrainer 192. Mixing bag 210 can be permanently or removably fluid coupled to tubing sections 204D and 204E but they are all typically sterilized together in a closed state. Tubing section 204D couples with pinch valve 128E. Tubing section 204E couples with bubble sensor 136B, pinch valve 128F and air filter assembly 218B.

A tubing section 204F of tubing 200 terminates at an outlet end 211. Outlet end 211 is typically intended to couple with cell expansion system 16 (Figure 1) for growth of the isolated T-cells. However, in other embodiments, outlet end 211 can be coupled with a container or some other downstream processing system. To facilitate the coupling, outlet end 211 can have a closed connector 205 formed thereat, such as a sterile connector, or can simply be sealed closed, such as by welding, so that outlet end 211 can be cut and then sealed onto another line to deliver the isolated cells. Tubing section 204F is coupled with pinch valve 128K.

A tubing section 204G of tubing 200 has a first end coupled with a collection bag 212. Collection bag 212 is used to collect the unwanted cells that are washed from the desired T-cells within isolation bag 206 and other negative fractions. Tubing section 204G is coupled with pinch valve 128J and connects with a second end of tubing section 204E. Tubing sections 204H, 204I, and 204J of tubing 200 each have a first end that is permanently or removably coupled to a separate media bag 216A, 216B, and 216C, respectively and an opposing send end that is fluid coupled, either directly or indirectly to tubing section 204G. Media bags 216 house a medium that is used for washing and other processing of the cells, as discussed later below. Tubing sections 205H, 205I, and 205J are coupled to pinch valves 128G, 128H and 128I, respectively. It is appreciated that the number of media bag 216 used can depend on the application and the size of bags 216. Thus, in other embodiments, one, two or four or more media bags 216 can be used with corresponding sections of tubing 200, rather than having three media bag 216.

A tubing section 204K of tubing 200 couples with a second end of tubing sections 204A, 204B, 204D, 204F and 204G. During assembly for use, tubing section 204K is coupled with pumps 124A and 124B and also with bubble sensors 136A and 136C and pressure sensor 140. Pumps 124A and 124B are used to facilitate controlled flow of fluid through line set 174A.

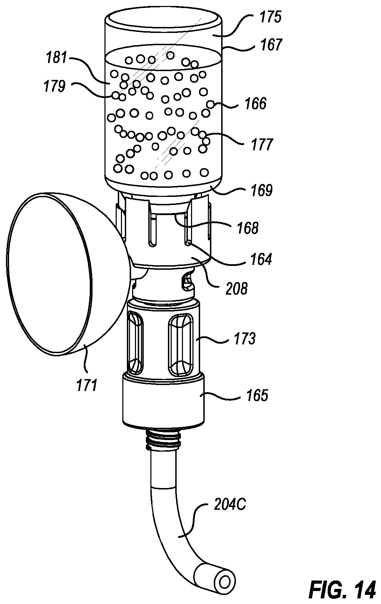

As previously discussed, the first end of tubing section 204C is connected to bead vial coupler 208. Turning to Figure 14, one embodiment of bead vial coupler 208 is shown having a first end 164 and an opposing second end 165. Second end 165 is fluid coupled to the first end of tubing section 204C. A vial 166 is shown mounted to first end 164 of bead vial coupler 208. Vial 166 includes a cylindrical body 167, a constricted neck 168 that encircles an opening, and an annular shoulder 169 that radially outwardly flares at is extends from neck 168 to body 167. Vial 166 bounds a compartment 175 in which a bead mixture 177 is disposed. Bead mixture 177 includes a plurality of beads 179 and a carrier liquid 181. Carrier liquid 181 functions to enable beads 179 to flow out of vial 166 and through line set 174A. Carrier liquid 181 can comprise a cell culture medium or other free flowing liquids that will not negatively influence the cells. In alternative embodiments, vial 166 can be replaced with a flexible bag or other type of container that houses bead mixture 177. Vials and flexible bags have alternative advantages for housing bead mixture 177. For example, a rigid vial is easily rocked for resuspension of the beads and there is low risk of puncture. However, the use of flexible bags eliminates the formation of a negative pressure within the container as the bead mixture 177 is withdrawn, as discussed further below.

As will be discussed below in more detail, beads 179 are used for the isolation and activation of desired T-cells. Beads 179 will typically be composed of a magnetic material and a polymer. While the actual structure of the bead can vary greatly, the beads may, as examples, have a magnetic core and a polymeric exterior or can be composed of a polymeric material with embedded magnetic particles. Exemplary magnetic beads are CTS^{™} DYNABEADS^{™} CD3/CD28 (Thermo Fisher Scientific, cat. no. 40203D). For example, in one embodiment beads 179 can comprise a polymer matrix in which magnetic particles, such as nano- or microparticles, are embedded. In other embodiments beads 179 can comprise a polymer core that is covered by a magnetic shell. The magnetic material can comprise a magnetic metal (*e.g*., iron, cobalt, nickel, etc.). Other configurations can also be used. In one embodiment, beads 179 can comprise paramagnetic beads or superparamagnetic beads. Beads 179 also include one or more antibodies (e.g., anti-CD3, anti-CD4, anti-CD8, anti-CD28, and/or anti-CD137) coupled to the core. The antibodies bind with and activate the desired T-cells for therapeutic functionality or other purposes. Beads 179 typically have a substantially spherical configuration with an average diameter in a range between 50 nm and 1 mm, between 100 nm and 1 mm, between 500 nm and 100 µm, between 1 µm and 10 µm, between 1 µm and 5 µm, or between 4 µm and 5 µm. Example of beads 179 is DYNABEADS produced by Thermo Fisher Scientific. Specific examples of commercially available magnetic beads are DYNABEADS^{™} Human T-Expander CD3/CD28 (Thermo Fisher Scientific, cat. no. 11141D), CTS^{™} DYNABEADS^{™} CD3/CD28 (Thermo Fisher Scientific, cat. no. 40203D), CTS^{™} DYNABEADS^{™} Treg Xpander (Thermo Fisher Scientific, cat. no. 46000D), and particles contained in the MACS^{™} GMP ExpAct Treg Kit (Miltenyi Biotec, cat. No. 170-076-119).

Bead vial coupler 208 comprises a tubular stem 173 extending between first end 164 and opposing second end 165. First end 164 is configured to couple with neck 168 of vial 166 so that a secure fluid coupling is formed between compartment 175 of vial 166 and first end 164. Second end 165 is fluid coupled with tubing section 204C of tubing 200. In the depicted embodiment, bead vial coupler 208 further includes an expansion chamber 171 that outwardly projects from stem 173. Expansion chamber 171 is configured to automatically equalize the pressure within compartment 175 of vial 166 as bead mixture 177 is dispensed into line set 174A, thereby preventing the formation of a vacuum within chamber 171 that can interfere with fluid flow. Two examples of bead vial coupler 208 incorporating expansion chamber 171 are the PHASEAL^{™} drug vial access device produced by Becton Dickinson and a needle free vial spike valve available from OriGen Biomedical (cat. no. VSV). In alternative embodiments, bead vial coupler 208 need not include expansion chamber 171 and other configurations of bead vial couples can be used.

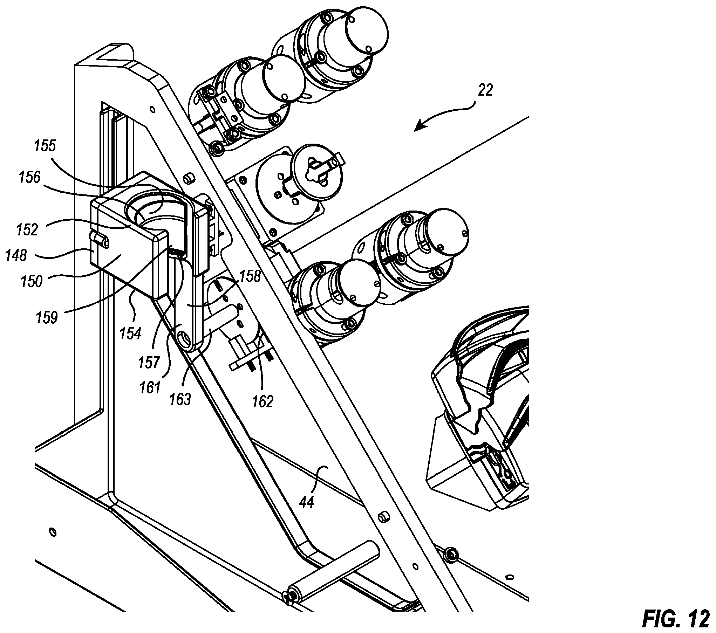

Turning to Figure 5, a slot 144 passes through support panel 92 alongside panel 52B so as to communicate with a compartment 146. Compartment 146 is configured to receive bead vial coupler 208. More specifically, disposed within compartment 146 is a bead vial retainer 148. As depicted in Figures 5 and 12, bead vial retainer 148 has a body 150 having a top face 152, an opposing bottom face 154 and an interior face 155 that bounds a C-shaped channel 156 extending therebetween. A shoulder 157 inwardly projects from interior face 155 at toward bottom face 154 so as to constrict the diameter of channel 156. A slot 159 passes through a front face of body 150 between top face 152 and bottom face 154 so as to communicate with channel 156. Bead vial retainer 148 further includes an elongated arm 158 having a first end connected to body 150 and an opposing second end 161 that projects down below bottom face 154.

Supported within compartment 44 of bead processing apparatus 22 is a drive motor 162 that is controlled by electrical circuitry 53 (Figure 4) and that rotates a drive shaft 163. In one embodiment, drive motor 162 comprises a stepper motor that can selectively rotate drive shaft 163 in opposite directions. The free end of drive shaft 163 is coupled with and supports second end 161 of arm 158. Drive shaft 163 and arm 158 typically extend orthogonally. As such, selective rotation of drive shaft 163 by drive motor 162 selectively rotates bead vial retainer 148 about a rotational axis of drive shaft 163.

During assembly and use, bead vial coupler 208 having vial 166 attached thereto is advanced down through channel 156 of bead vial retainer 148 until shoulder 169 of vial 166 (Figure 14) comes to rest on shoulder 157 of bead vial retainer 148. Body 150 of bead vial retainer 148 is configured to snugly and securely receive vial 166 within channel 156 under frictional engagement. During this assembly, expansion chamber 171 can project out through slot 159 of bead vial retainer 148.

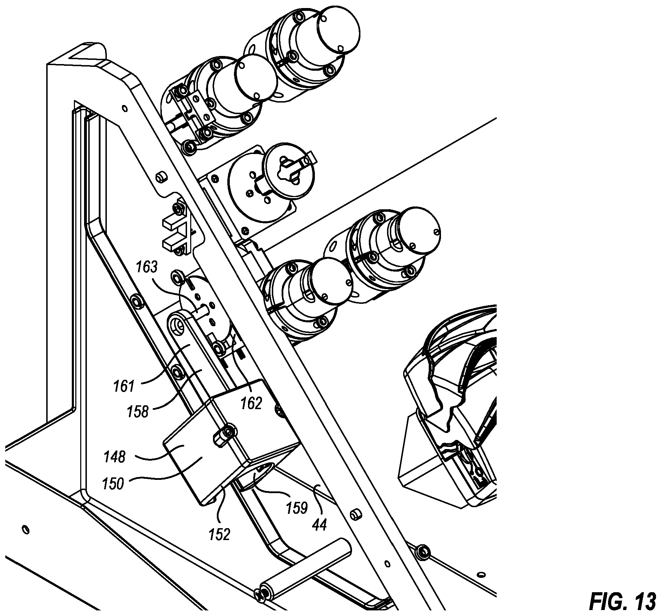

Bead mixture 177 is more efficiently dispensed from vial 166 into line set 174A if beads 179 are suspended in carrier liquid 181. However, if inverted vial 166 mounted on bead vial retainer 148 remains stationary in a vertical orientation for a period, beads 179 can settle at neck 168 of vial 166 and thereby restrict or block flow out of vial 166. Accordingly, once bead vial coupler 208 with vial 166 are mounted on bead vial retainer 148 and it is desired to dispense bead mixture 177 into line set 174A, drive motor 162 can be activated to rotate drive shaft 163 so that bead vial retainer 148 is rotated to a forward position, as shown in Figure 13 and then rotated back to the upstanding position as shown in Figure 12. This rotation of vial 166 causes beads 179 to mix and resuspend within carrier liquid 181 for efficient dispensing. In one embodiment, vial 166 can be rotated to the forward position and left for a sufficient period for beads 179 to settle toward the bottom end of vial 166, *i.e.,* the end opposite of neck 168, before vial is rotated back to the upstanding position. In other embodiments, vial 166 could be quickly and/or repeatedly moved back and forth between the forward and upstanding position to facilitate the desired resuspension. When in the upstanding position, vial 166 is typically inverted but in a vertical orientation. As vial 166 is moved to the forward position, vial 166 is typically rotated over an angle relative to vertical of at 90°, 120°, 160° or 180° or in a range between any two of the foregoing. Other angles can also be used.

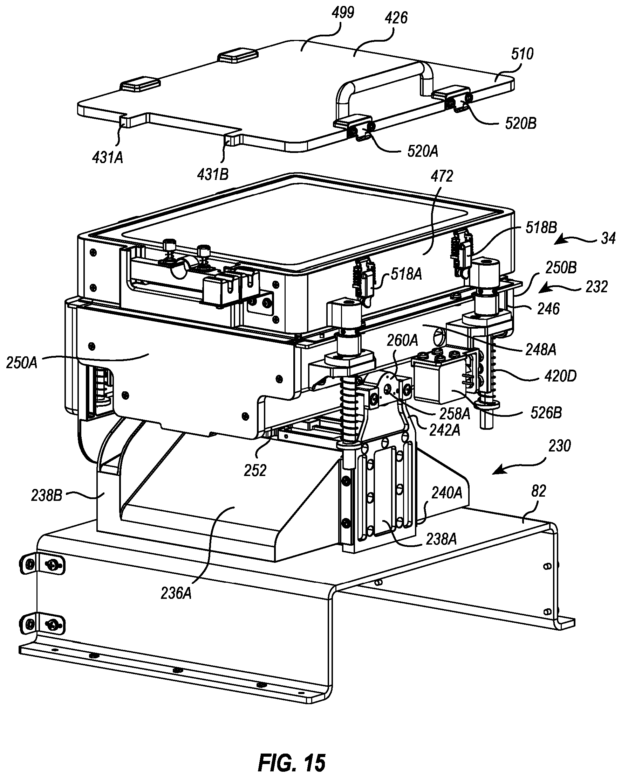

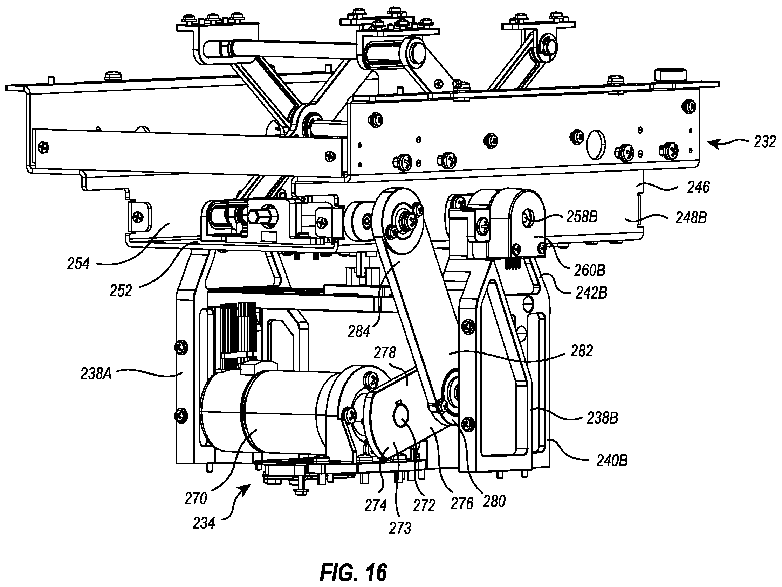

Further use and operation of consumable kit 170A will be discussed later after describing rocker assembly 34. Turning to Figure 15, rocker assembly 34 is separated from the remainder of bead processing apparatus 22 and is resting on stand 82. In general, rocker assembly 34 comprises a mount assembly 230 that is disposed on stand 82, a platform assembly 232 that is movably coupled to mount assembly 230 and a rocker drive 234 (Figure 16) that is used to selectively rock platform assembly 232 relative to mount assembly 230. Mount assembly 230 includes an outer housing 236 that is disposed on stand 82 and partially cover rocker drive 234. Turning to Figure 16, outer housing 236 and other components have been removed to better reveal that mount assembly 230 further includes a first riser 238A and a spaced apart second riser 238B that upwardly extend from stand 82. With reference to Figure 15, riser 238A has a lower end 240A secured to stand 82 and an opposing upper end 242A.

Platform assembly 232 is pivotally connected to upper end 242A. More specifically, platform assembly 232 includes, in part, a housing assembly 246 having a front wall 250A and an opposing back wall 250B with opposing lateral walls 248A and B that extend therebetween. Walls 240 and 250 upstand from a floor 252 (Figure 16) and bound a compartment 254. In the depicted embodiment, upper end 242A of riser 238A is centrally rotatably coupled to housing assembly 246. More specifically, upper end 242A centrally rotatably coupled to lateral wall 248A. In one embodiment, an axle 258A outwardly projects from lateral wall 248A and is rotatably coupled to upper end 242 of riser 238A. A bearing 260A can be disposed at upper end 242A of riser 238A for receiving and facilitating easy rotation of axle 258A. It is of course appreciated that there are a variety of alternative ways in which housing assembly 246/platform assembly 232 can be rotatably mounted to riser 238A.

Returning to Figure 16, riser 238B also has a lower end 240B that is secured to stand 82 and opposing upper end 242B. Again, platform assembly 232 is pivotally connected to upper end 242B and, more specifically, upper end 242B is centrally and pivotably connected to housing assembly 246/lateral wall 248B. An axle 258B can project from lateral wall 248B and be rotatably mounted to upper end 242B such and through a use of a bearing 260B. Axles 258A and 258B are disposed on a common rotational axis about which platform assembly 232 rotates relative to risers 238.

With continued reference to Figure 16, rocker drive 234 includes a motor 270 mounted to stand 82 (Figure 13). Motor 270 is controlled by electrical circuitry 53 (Figure 4) and rotates a drive shaft 272. A link 276 extends orthogonal to drive shaft 272 and is rotated thereby. In the depicted embodiment, link 276 is elongated having a first end 274 connected to drive shaft 272 and an opposing second end 278 that is connected to a first end 280 on an elongated connecting arm 282. In alternative embodiments link 276 could be circular or have other shapes. Drive shaft 272 and link 276 from a crank 273 to which connecting arm 282 is attached. Connecting arm 282 has an opposing second end 284 that is rotatably connected to platform assembly 232. More specifically, second end 284 is rotatably coupled to housing assembly 246 at or toward one end of lateral wall 248B. Accordingly, as motor 270 is activated, drive shaft 272 and link 276, *i.e.,* crank 273, are rotated. In turn, the rotation of link 276 causes connecting arm 282 to selectively raise and lower which in turn causes platform assembly 232/housing assembly 246 to rock back and forth about the common axis passing through axles 258A and 258B. The rocking motion can be facilitated by drive shaft 272 being rotated continually or in a reciprocating motion.

In one embodiment of the present invention, means are provided for selectively rocking platform assembly 232/housing assembly 246. One example of the means for rocking is rocker drive 234. In alternative embodiments, rocker drive 234 could be replaced with a variety of different mechanical mechanisms that achieve the same function. For example, rocker drive 234 could be replaced with a cam follower system where motor 270 rotates a cam while a follower connected to the cam raises and lowers and end of platform assembly 232/housing assembly 246 as the cam rotates. In other embodiments, motor 270 could reciprocally dive a worm gear that selectively raises and lowers the end of platform assembly 232/housing assembly 246. In other embodiments, hydraulic or pneumatic systems could be used to raise or lower a piston that in turn selectively raises and lowers and end of platform assembly 232/housing assembly 246. Other conventional system can also be used.

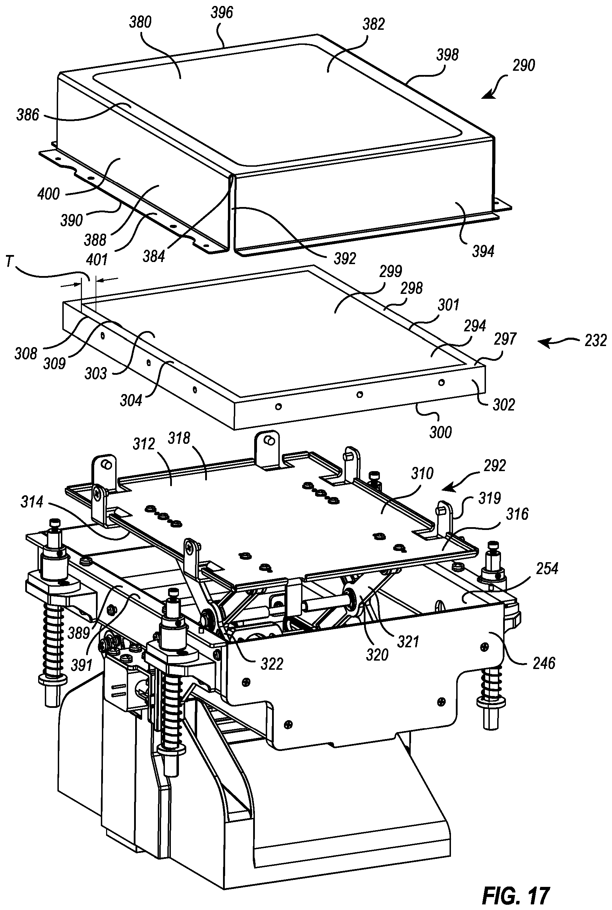

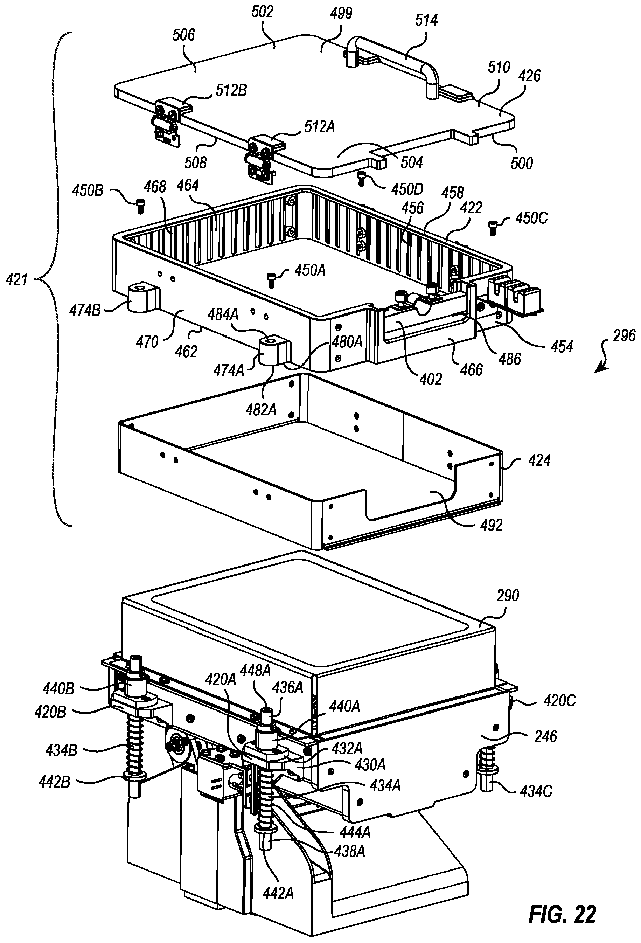

With reference to Figure 17, platform assembly 232 generally comprises a platform 290 on which isolation bag 206 (Figure 10) is removably supported, housing assembly 246, as previously discussed, on which platform 290 is secured and supported, a lift assembly 292 that is at least partially disposed within compartment 254 of housing assembly 246, a magnet assembly 294 that is supported on and raised and lowered by lift assembly 292, and a restraining assembly 296 (shown in Figure 22) that at least partially covers isolation bag 206. These various components will not be discussed in further detail.

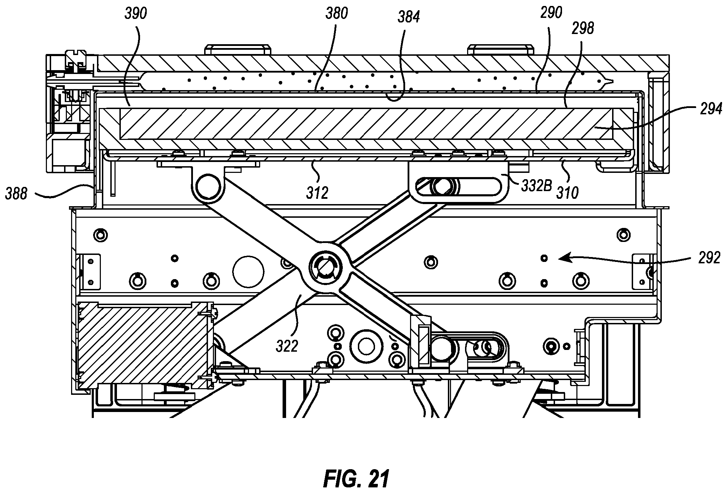

With continued reference to Figure 17, platform 290 comprises a support plate 380 having a top surface 382 and an opposing bottom surface 384 that each extend to a perimeter edge 386. Support plate 380 typically has a rectangular configuration with surfaces 382 and 384 being disposed in parallel alignment. As magnet assembly 294 will need to produce a magnetic force through support plate 380, support plate 380 typically has a thickness between surfaces 382 and 384 that is less than 5 mm, 3 mm, 2 mm or 1 mm or can be in a range between any two of the foregoing values. Downwardly projecting from perimeter edge 386 is a sidewall 388. Sidewall 388 and bottom surface 384 of support plate 380 at least partially bound a cavity 390 (Figure 21). Sidewall 388 can completely encircle cavity 390 or, as depicted, can have slots 392 extending therethrough, such as at the corners, so as only partially encircle cavity 390. Sidewall 388 includes a front wall 394 and an opposing back wall 396 with opposing lateral walls 398 and 400 extending therebetween. Disposed at a lower terminal end of sidewall 388 is a flange 401. Sidewall 388 sits on a top perimeter edge 389 of housing assembly 246 so as to span across and at least partially cover compartment 254 thereof. A flange 391 can also be formed on perimeter edge 389 of housing assembly 246 to facilitate attachment between housing assembly 246 and platform 290 by fasteners, such as screws, bolts, clips, or the like, passing through or connecting together flanges 391 and 401. Platform 290 is typically made of a material that will not be attached to magnet assembly 294. For example, platform 290 is commonly made of aluminum or a polymer.

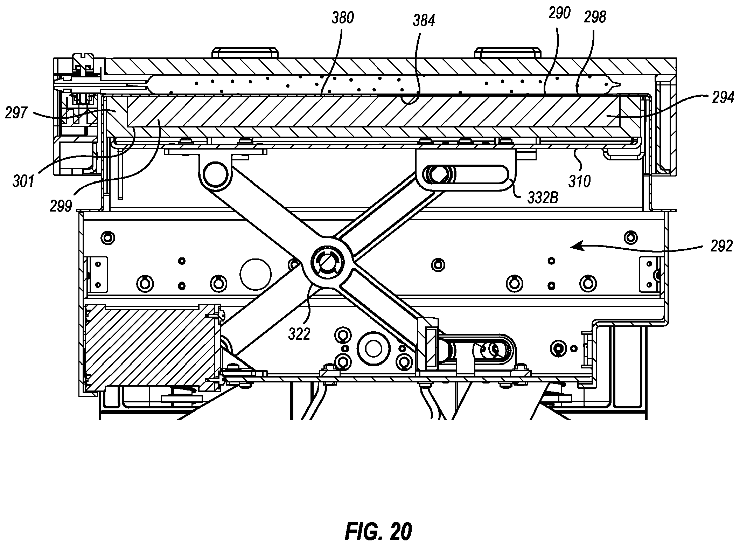

Continuing with Figure 17, magnet assembly 294 is shown in the form of a plate typically having a square or rectangular configuration. In one embodiment, magnet assembly 294 includes a non-magnetic casing 297 on which a magnet 299 is disposed. Casing 297 has a square or rectangular configuration having a top surface 298 and an opposing bottom surface 300 which extend to an encircling sidewall 302. Top surface 298 and bottom surface 300 are typically planer and disposed in parallel alignment. A recess 301 (Figure 20) is formed on top surface 298 in which magnet 299 is positioned and secured. Recess 301 is centered on top surface 298 and has a square or rectangular configuration complementary to but smaller than casing 297. Magnet 299 has a square or rectangular configuration that is complementary to recess 301 so as to substantially occupy all of recess 301. Magnet 299 has a planar top surface 303 that is typically disposed in the same plane as top surface 298 of casing 297. In this assembled configuration, a non-magnetic edge 304 is formed by casing 297 that encircles magnet 299. That is casing 297/ top surface 298 has an outer perimeter edge 308 and recess 301/magnet 299 has an outer perimeter edge 309. Non-magnetic edge 304 has a thickness T extending between perimeter edges 308 and 309 that is typically in a range between 0.2 cm and 2 cm and more commonly in a range between 0.3 cm and 1.5 cm or 0.4 cm and 1 cm. Magnet 299 is disposed so that the positive or attractive magnetic force thereof extends upward from top surface 303 toward platform 290. As discussed later, this enables magnet to attract beads 179 (Figure 14) when disposed within isolation bag 206 positioned on platform 209.

In one embodiment, magnet 299 can comprise a single continuous magnet. In an alternative embodiment, magnet 299 can comprise a plurality of separate magnets that are adjacently disposed with recess 301. For example, in one embodiment, magnet 299 can comprise a plurality of magnets that are adjacently disposed so as to form a Halbach array. A Halbach array is a special arrangement of permanent magnets that augments the magnetic field on one side of the array while cancelling the field to near zero on the other side. This is achieved by having a spatially rotating pattern of magnetization. The rotating pattern of permanent magnets (on the front face; on the left, up, right, down) can be continued indefinitely and have the same effect. Depicted in Figure 18A is an example of a portion of magnet 299 viewed from the bottom and comprised of a plurality of adjacently disposed separate magnets 305A-305E. Magnets 305 are disposed in the form of a Halbach array. The arrows shown on each magnet 305 identify the orientation of the magnetic field of each magnet 305. The orientation shown produces a strong magnetic field extending upward from top surface 303 toward platform 290 while the magnetic field below magnet 305 would be cancelled. Additional magnets 305 can be added in the same repeating pattern to occupy recess 301 and complete the formation of magnet 299. Forming magnet 299 as a Halbach array increases the magnetic force for better attracting beads 179 while eliminating the magnetic field below magnet 299 that could potentially interfere with the components or operation of bead processing apparatus 22. However, placing magnets 305 in a rotating pattern to achieve the Halbach array results in magnets 305 wanting to push away from each other. Casing 297 can be used to help mechanically hold magnets 305 together. Alternatively, or in addition, magnets 305 can be secured together by an adhesive or by using other coupling techniques. Figure 18B is a schematic representation of a Halbach array that can be used with the present disclosure and that both identifies the orientation of the magnets and the relative locations of the strong and weak magnetic fields.

Returning to Figure 17, lift assembly 292 is at least partially disposed within compartment 254 of housing assembly 246 and is used to support and also raise and lower magnet assembly 294. Lift assembly 292 includes a shelf 310 having a top surface 312 and an opposing bottom surface 314 that extend between a front end 316 and an opposing back end 318. A plurality of spaced apart fasteners 319 are used to secure magnet assembly 294 to top surface 312 of shelf 310. Secured to and projecting below bottom surface 314 of shelf 310 is scissor lift 321.

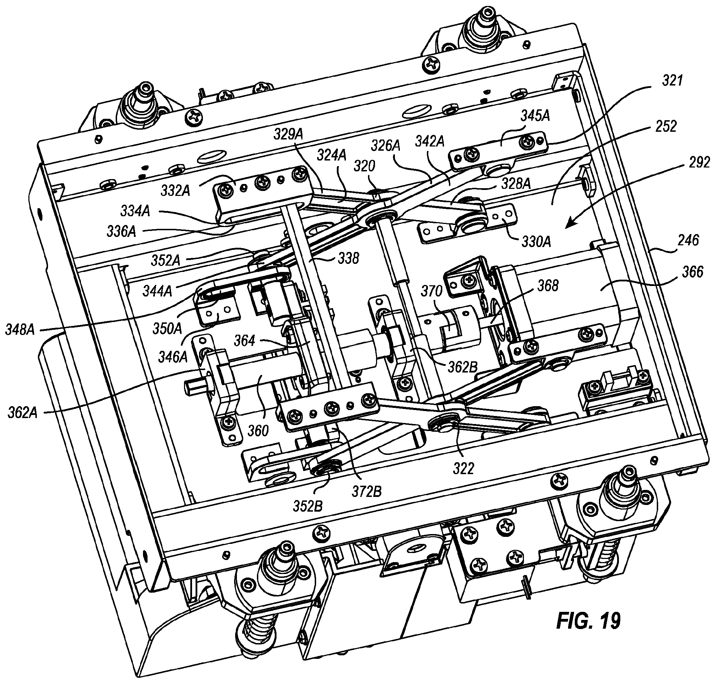

Turning to Figure 19, scissor lift 321 comprises a first pair of scissor arms 320 and a spaced apart second pair of scissor arms 322. First pair of scissor arms 320 include a first arm 324A which centrally crosses and is pivotally coupled to a second arm 326A. First arm 324A and second arm 326A are pivotably coupled together by an axle 377 that orthogonally extends to second pair of scissor arms 322. First arm 324A has a first end 328A and an opposing second end 329A. First end 328A is hingedly coupled to floor 252 of housing assembly 246 at front end thereof by a hinge joint 330A. Second end 329A is both slidably and hingedly coupled to bottom surface 314 of shelf 310 at back end 318 by a guide 332A. (See corresponding guide 332B in Figure 20 use with second scissor arms 322.) That is, guide 332A is securely fixed to bottom surface 314 of shelf 310 at back end 318 and includes a downwardly projecting flange 334A having an elongated, horizontally extending slot 336A. A guide bar 338 orthogonally projects from second end 329A of first arm 324A, through slot 336A and toward second pair of scissor arms 322. In this configuration, second end 329A of first arm 324A can slide horizontally relative to shelf 310 by guide bar 338 sliding within slot 336 and can freely rotate by either guide bar 338 rotating within slot 336 and/or second end 329A rotating relative to guide bar 338, depending on the desired configuration.

Second arm 326A also has a first end 342A and an opposing second end 344A. First end 342A is hingedly coupled to bottom surface 314 of shelf 310 at front end 316 by a hinge joint 345A. Second end 344A of second arm 326A is both slidably and hingedly coupled to floor 252 of housing assembly 246 at a back end by a guide 346A. That is, guide 332A is securely fixed to interior surface of 252 of floor 252 at the back end and includes an upwardly projecting flange 348A having an elongated, horizontally extending slot 350A formed thereon. A guide pin 352A orthogonally projects from second end 344A of second arm 326A, through slot 350A and toward second pair of scissor arms 322. In this configuration, second end 344A of second arm 326A can slide horizontally relative to floor 252 by guide pin 352A sliding within slot 350A and can freely rotate by either guide pin 352A rotating within slot 350A and/or second end 344A rotating relative to guide pin 352A.

Second pair of scissor arms 322 has the same configuration, structural elements and attachment to floor 252 and shelf 310 as first pair of scissor arms 320. As such, the above discussion is also applicable to second pair of scissor arms 322 and like elements between first pair of scissor arms 320 and second pair of scissor arms 322 are identified by like reference characters except that the reference characters for second pair of scissor arms 322 include the suffix "B".

With continued reference to Figure 19, lift assembly 292 further includes a threaded shaft 360 that is rotatably mounted at opposing ends to floor 252 by bearings 362A and 362B. Threadedly mounted on shaft 360 is a collar 364. Collar 364 is configured and mounted such that rotation of shaft 360 in opposite directions causes collar 364 to travel along the length of shaft 360 in opposite directions. Shaft 360 is selectively rotated by a motor 366 that is mounted on floor 252 and is electrically coupled to and controlled by electrical circuitry 53 (Figure 4). In the depicted embodiment, motor 366 rotates a drive shaft 368 that is coupled with shaft 360 through a union 370. Union 370 provides play to facilitate proper alignment between drive shaft 368 and shaft 360. In other embodiments, however, union 370 can be eliminated and drive shaft 368 can connect directly to shaft 360. Collar 364 is connected to guide pin 352A of first scissor arms 320 and guide pin 353B of second scissor arms 322. More specifically, in one embodiment, a first extension 372A extends between collar 364 and guide pin 352A while a second extension 372B extends between collar 364 and guide pin 352B. As needed, extensions 372 can be adjusted for facilitating proper alignment between collar 364 and guide pins 352. In alternative embodiments, collar 364 can be directly coupled to guide pins 352.

By the controlled operation of motor 366, lift assembly 292 can be used to selectively control moving shelf 310/magnet assembly 294 between a raised position and a lowered position. For example, depicted in Figure 20, lift assembly 292 has raised magnet assembly 294 relative to platform 290 in an activation position. That is, first pair of scissor arms 320 and second pair of scissor arms 322 are positioned so that top surface 298 of magnet assembly 294 is disposed against or directly adjacent to bottom surface 384 of support plate 380/platform 290. In one embodiment, the distance between top surface 298 of magnet assembly 294 and bottom surface 384 of support plate 380/platform 290 when magnet assembly 294 is raised to the activation position is less than 10 mm, 7 mm, 4 mm, or 2 mm or in a range between any two of the foregoing values. That is, as discussed further below, magnet assembly 294 has to be sufficiently close to platform 290 to attract and hold beads 179 disposed within isolation bag 206 when isolation bag 206 is supported by platform 290.

For moving magnet assembly 294 lower relative to platform 290 to a deactivation position, motor 366 is activated so as to rotate threaded shaft 360 which advances collar 364 horizontally away from motor 366. In turn, collar 364 concurrently moves extensions 372 and guide pins 352 horizontally rearward along guides 346 which in turn moves second end 344A of first arm 324 horizontally away from first end 328A of second arm 326A. As ends 344A and 328A horizontally separate, arms 324 and 326 pivot relative to each other at axle 377 which causes the upper ends of arms 324 and 326 to both horizontally separate and lower, thereby lowering shelf 310 and magnet assembly 294 relative to support plate 380 as shown in Figure 21. The distance between top surface 298 of magnet assembly 294 and bottom surface 384 of support plate 380/platform 290 when lift assembly has moved magnet assembly 294 to the lowered deactivation position is typically greater than 3 cm, 3.5 cm, 4 cm, 4.5 cm, 5 cm or 6 cm or in a range between any two of the foregoing values. By reversing the direction that motor 366 rotates drive shaft 368, the above process is reversed and lift assembly 292 can be moved back to raised position.

One embodiment of the present invention includes means at least partially disposed within the compartment of housing assembly 246 for selectively raising and lowering magnet assembly 294 relative to platform 290 between a raised activation position and a lowered deactivation position. One example of such means includes scissor lift 321 as described above. In alternative embodiments, scissor lift 21 can be replaced with a variety of alternative mechanism that can achieve the same function. By way of example and not by limitation, shelf 310 could be mounted on tracks that enable shelf 310 to vertically slide up and down relative to platform 290 while remaining horizontally disposed. Various mechanical system can then be used to move shelf 310 along the tracks. By way of example, motor 366 could be used to rotate a crank that raises and lowers a connecting arm connected to shelf 310. Alternatively, a cam follower system can be used where motor 366 rotate a cam while a follower connected to the cam and extending to shelf 310 raises and lowers shelf 310 as the cam rotates. In other embodiments, motor 366 could reciprocally dive a worm gear that selectively raises and lowers shelf 310. In other embodiments, hydraulic, pneumatic, cable or chain systems could be used to raise and lower shelf 310 along the tracks. Other conventional systems can also be used.

Turning to Figure 22, restraining assembly 296 generally comprises four spaced apart spring assemblies 420A - 420D disposed on housing assembly 246 and a cover assembly 421 coupled thereto. Cover assembly 421 comprises a cover housing 422 that is coupled to each of spring assemblies 420 and encircles platform 290, an optional sleeve 424 that is positioned between platform 290 and cover housing 422, and a lid 426 that is hingedly mounted on cover housing 422. The various components of restraining assembly 296 will now be discussed.

Spring assembly 420A comprises a bracket 430A mounted to and outwardly projecting from housing assembly 246. Bracket 430A can also form a portion of housing assembly 246. Bracket 430A has a hole 432A that vertically extends therethrough. A rod 434A is slidably received within hole 432A and extends between a first end 436A and opposing second end 438A. First end 426A projects above bracket 430A and has a collar 440A securely coupled thereto. Collar 440 has an outer diameter larger than an outer diameter of hole 432A so that collar 440 prevents rod 434A from passing down through hole 432A. A flange 442A outwardly projects from second end 438A of rod 434A. A spring 444A extends between flange 442A and the bottom surface of bracket 430A such that as rod 434A is pushed upward through hole 432A, spring 444A is resiliently compressed so as to urge rod 434A downward. First end 436A of rod 434A terminates at an end face 446A having a threaded socket 448A formed thereon. As will be discussed below in greater detail, socket 448A is configured to receive a threaded fastener 450A. Spring assemblies 420B, 420C, and 420D are identical to spring assembly 420A and are each located adjacent to a corresponding corner of cover housing 422. As such, the above discussion of spring assembly 420A is likewise applicable to spring assemblies 420B-D. Furthermore, like elements of spring assembly 420A and spring assemblies 420B-D are identified by like reference characters except that each of the reference characters for spring assemblies 420B-D include the suffix B, C, and D, respectively.

Cover housing 422 includes an annular perimeter wall 454 having an interior surface 456 and an opposing exterior surface 458 that extend between an upper edge 460 and opposing lower edge 462. Interior surface 456 encircles an opening 464 that passes through perimeter wall 454 between upper edge 460 and lower edge 462. Opening 464 is configured to receive platform 290. Perimeter wall 454 comprises a front wall 466 and an opposing back wall 468 with lateral walls 470 and 472 extending therebetween. Outwardly projecting from exterior surface 458 of cover housing 422 are four mounts 474A-474D. Mounts 474A and 474B outwardly project from lateral wall 470 at a forward end and rearward end thereof, respectively. Likewise, mounts 474C and 474D outwardly project from exterior surface 458 of lateral wall 472 at a forward and rearward end thereof, respectively. Mounts 474 are positioned to align with spring assemblies 420 when cover housing 422 is received over platform 290. Mount 474A is configured to couple with first end 436A of rod 434A. For example, in one embodiment, mount 474A has a top surface 478A and an opposing bottom surface 480A. A recess 482A is formed on bottom surface 480A and is configured to receive first end 436A of rod 434A. An opening 484A is formed on top surface 478A that communicates with recess 482A. Opening 484A has a diameter smaller than the outer diameter of rod 434A as first end 436A. Fastener 450A is configured to pass down through opening 484A and thread into socket 448A so as to secure first end 436A of rod 434A to mount 474A. It is appreciated that a variety of alternative techniques can be used for securing together rod 434A and mount 474A. For example, the structures can be secured together by press-fit connection, adhesive, welding, or a variety of other types of mechanical fasteners. Mounts 474B - 474D all have the same structural elements and configuration as mount 474A and are similarly configured to couple with rods 434B-D of spring assemblies 420B-D, respectively. Like elements between mount 474A and mounts 474B-D are identified by like reference characters except that reference characters for mounts 474B-D includes the suffixes B, C, and D, respectively.

Front wall 466 of cover housing 422 has a recess 486 formed thereon that extends down from upper edge 460. As discussed below, recess 486 is configured to receive a clamp assembly 402.

Sleeve 424 also includes an encircling perimeter wall 490 that is configured to encircle platform 290 and be received within opening 464 of cover housing 422. Sleeve 424 is coupled to cover housing 422 such as by fasteners and other conventional techniques so that sleeve 424 moves concurrently with cover housing 422. Sleeve 424 also has a recess 492 formed therein that aligns with recess 486 and is configured to received clamp assembly 402. Sleeve 424 can function, in part, to increase the structural integrity cover housing 422. For example, in one embodiment cover housing 422 can be made of a plastic while sleeve 424 is formed of a metal such as aluminum. This design reduces cost but achieves desired strength. In alternative embodiments, the structural integrity of cover housing 422 can be increased and sleeve 424 can be eliminated.

Lid 426 comprises a lid plate 499 having an inside face 500 and an opposing outside face 502 that extend between a front edge 504, a back edge 506, and opposing lateral edges 508 and 510. A pair of spaced apart hinges 512A and 512B extend between lateral edge 508 of lid plate 499 and lateral wall 470 of cover housing 422. As a result, lid 426/lid plate 499 is hingedly mounted to covering housing 422. Lid 426 further includes a handle 514 upwardly extends from lid plate 499 at or towards lateral edge 510. As better depicted in Figure 15, a pair of latches 518A and 518B are mounted on lateral wall 472 of cover housing 422 while a pair of corresponding catches 520A and B are mounted on lateral edge 510 of lid plate 499. Latches 518A and B are configured to releasably engage catches 520 to selectively hold lid 426/lid plate 499 in a closed position. That is, with cover housing 422 secured to spring assemblies 420, lid 426 can be selectively moved between an open position wherein lid plate 499 is rotated about hinges 512 so that platform 290 is openly exposed and freely accessible and a closed position wherein inside face 500 of lid plate 499 is disposed against or adjacent to top surface 382 of support plate 380 of platform 290. In one embodiment, inside face 500 of lid plate 499 can be disposed in parallel alignment with top surface 382 of support plate 380. When in the closed position, latches 518 can be secured to catches 520 for securing lid 426 in the closed position.

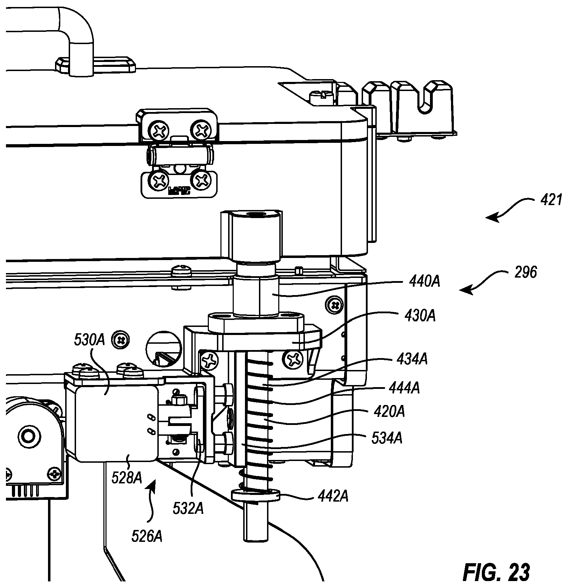

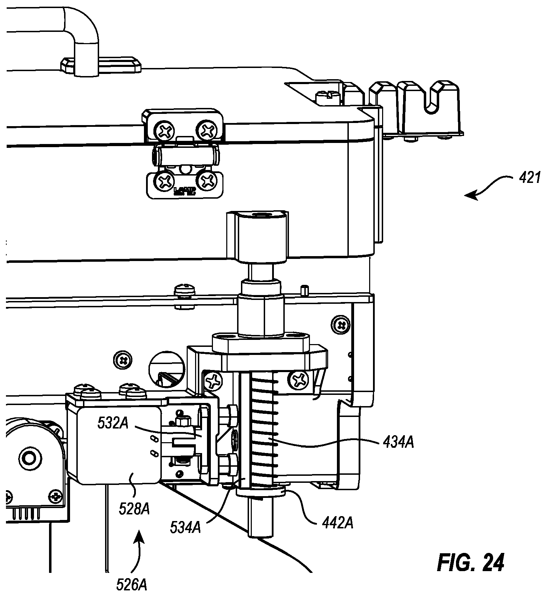

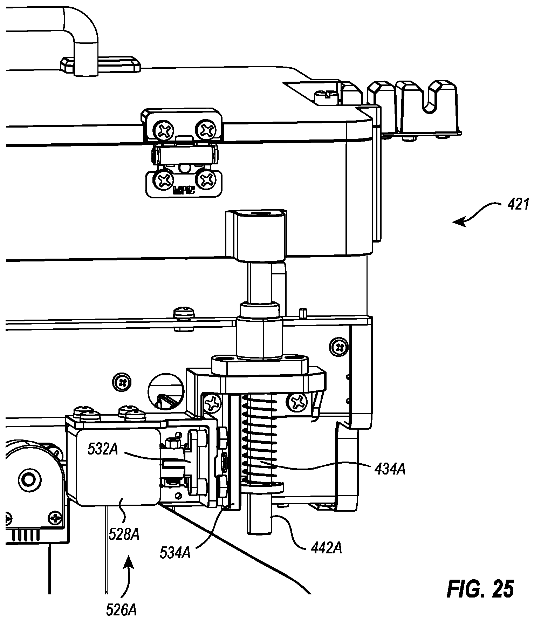

Turning to Figure 23, restraining assembly 296 can further includes a stop assembly 526A that operates with spring assembly 420A. Stop assembly 526A comprises a solenoid valve 528A that is in electrical communication with and is operated by electrical circuitry 53 (Figure 4). Solenoid valve 528A includes a housing 530A and a piston 532A that is selectively moved relative to housing 530A. Mounted on piston 532A is a stop 534A. In the depicted embodiment, stop 534A is in the form of an elongated plate. In other embodiments, stop 534 can have different configurations. In still other embodiments, stop 534 can be eliminated and piston 532A can function as a stop. Stop 534A is disposed in parallel alignment with rod 434 and is located between bracket 430A and flange 442A. By solenoid valve 528A moving piston 532A in and out, stop 534A can be moved between an advanced restraining position and a retracted non-restraining position. In the advanced restraining position, as shown in Figure 23, stop 534A is vertically aligned with flange 442A. As a result, rod 434A can only be moved vertically upward until flange 442A comes into contact with stop 534A as depicted in Figure 24. When stop 534A is moved to the retracted non-restraining position, as shown in Figure 25, stop 534A is horizontally offset from flange 442A. As such, rod 434A and flange 442A can freely advance upward without restriction by stop 534A.

A stop assembly 526 can be associated with each spring assembly 420 or with only select spring assemblies. In the present depicted embodiment, a stop assembly 526B is associated with spring assembly 420D as depicted in Figure 15. Stop assembly 526B includes all the elements of and operates in the same way with spring assembly 420D as stop assembly 526A operates with spring assembly 420A. As such, like elements between stop assembly 526A and 526B are identified by like reference characters except that the elements of stop assembly 526B includes the suffix B.

As will be discussed below in further detail, during operation, isolation bag 206 is positioned on top of platform 290. Lid 426 is then moved to the closed position and latches 528 are used to lock lid 426 in the closed position. In this position, fluid is delivered into and removed out of isolation bag 206. As isolation bag 206 expands and contracts by fluid flowing in and out of isolation bag 206, cover assembly 421 is moved between different positions relative to platform 290. For example, when isolation bag 206 is empty, springs 444 resiliently urge rods 434 downward away from brackets 430 so that collars 440 of each spring assembly 420 rests on top of brackets 430. In this configuration, a minimum gap is formed between lid plate 499 and platform 290. As fluid enters isolation bag 206, isolation bag 206 begins to expand which produces a force that pushes cover assembly 421 and rods 434 upward and away from platform 290. As rods 434 are raised, springs 444 are compressed so as to increasingly resist expansion of isolation bag 206 and upward movement of cover assembly 421. Using springs 444 to resiliently compress isolation bag 206 between cover assembly 421 and platform 290 as isolation bag 206 expands and contracts, helps to maintain isolation bag 206 with a more uniform thickness, as opposed to bulging in the center. As a result, beads 179 disposed within isolation bag 206 are maintained closer to platform 290 and thus closer to the magnetic field produced by magnet assembly 294, thereby improving retention of beads 179 by magnet assembly 294.

With stop 534A in the advanced restraining position, cover assembly 421 can only raise to a first elevated position relative to platform 290, as previously discussed with regard to Figure 24, wherein flange 444 strikes against stop 534. As a result, the expansion of isolation bag 206 between lid 426/lid plate 499 and platform 290 is limited. This helps to further minimize thickness that isolation bag 206, or other bags that are disposed therein, can expand so as to further increase the magnetic field that is applied to beads 179 therein by magnet assembly 294. With stop 534 moved to the retracted non-restraining position, as shown in Figure 25, upward movement of cover assembly 421are not restrained by stop 534. As such, isolation bag 206 can continue to expand between lid plate 499 and platform 290 until isolation bag 206 reaches a filled capacity or the compression of springs 444 is greater than the outward expansion force produced by the fluid entering isolation bag 206.

One embodiment of the present disclosure includes means for resiliently restraining movement of cover assembly 421 away from platform 290. One example of such means is spring assemblies 420 as discussed above and the alternatives thereof. However, it is appreciated that a variety of other structures can accomplish the same function. For example, in one alternative, springs 444 could be replaced with elastomeric sleeves that encircle rods 434 or other forms of elastomeric material. In another alternative, in contrast to compressing each spring 444 between flange 442 and bottom of bracket 430, springs 444 could extend between and connect to collar 440 and the top of bracket 430 so that springs 444 are resiliently stretched as rods 434 move vertically upward. In other embodiments, resilient members can extend between and connect to cover assembly 421 and housing assembly 246 either directly or indirectly at spaced apart locations. As such, the resilient members again resiliently stretch as cover assembly is raised relative to platform 290. The resilient members can comprise springs, elastic bands, or other forms of elastomeric material. Other configurations can also be used.

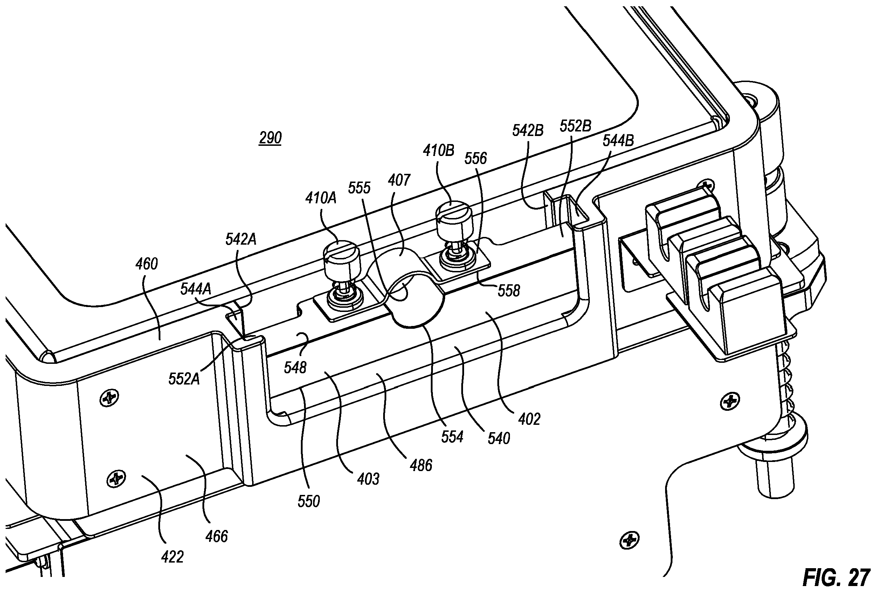

Returning to Figure 22, clamp assembly 402 is removably disposed on front wall 466 of cover housing 422. Clamp assembly 402 is used in part for securing and centering isolation bag 206 on top of platform 290. More specifically, turning to Figure 27, recess 486 is centrally formed on front wall 466 of cover housing 422 that extends down from upper edge 460. Recess 486 has an elongated U-shaped configuration that is bounded by a floor 540 and opposing sides 542A and 542B that upstand from floor 540. A U-shaped channel 544A and 544B is vertically recessed into each side 542A and 542B, respectively.

Clamp assembly 402 comprises an elongated clamp base 403 having a top surface 548 and an opposing bottom surface 550 that extend between opposing ends 552A and 552B. Ends 552A and 552B are slidably received within channels 544A and 544B of cover housing 422, respectively, so that clamp base 403 can slide vertically within channels 544 but is retrained from lateral movement. Centrally recessed on top surface 548 of clamp base 403 is a lower capture groove 554. Clamp assembly 402 also includes a clamp closure 407 that can be removably coupled to clamp base 403. Clamp closure 407 has a top surface 556 and an opposing bottom surface 558. An upper capture groove 555 is centrally recess in bottom surface 558. Fasteners 410A and 410B, such as screws or bolts, pass through clamp closure 407 on opposing sides of upper capture groove 555 and can selectively secure to clamp base 403 by threaded or other engagement. Upper capture groove 555 aligns with lower capture groove 554 when clamp closure 407 secured to clamp base 403. Lower capture groove 554 and upper capture groove 555 each typically have a semi-cylindrical configuration that can be the same size and shape. As such, aligned capture grooves 554, 555 can form a cylindrical opening. In other embodiments, capture grooves 554, 555 can other shapes such as semi-polygonal configurations.

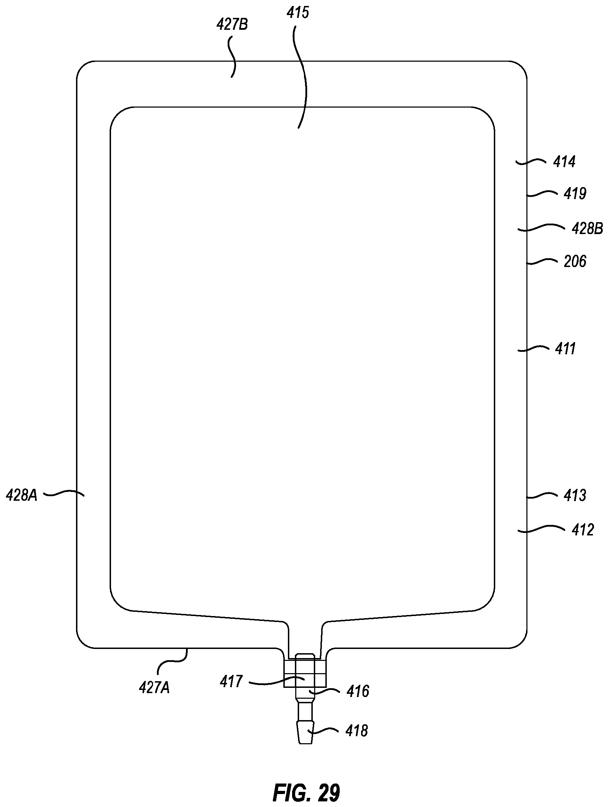

As previously mentioned, clamp assembly 402 is used in part for centering and securing isolation bag 206 on top of platform 290. Figure 29 shows one embodiment of isolation bag 206. Isolation bag 206 comprises a bag body 411 having a port 416 coupled thereto. Bag body 411 is typically a collapsible pillow type bag comprised of a top sheet 412 that overlies a bottom sheet 413. Sheets 412 and 413 are bonded together around their perimeter edge to form a perimeter seal 414. The bonding can comprise using methods known in the art such as welding through heat energies, radiofrequency (RF) energies, sonic energies or sonics, or other sealing energies or by use of an adhesive. The perimeter edge can comprise a front edge 427A, a back edge 427B, and opposing side edges 428A and 428B extending therebetween. Bounded between sheets 412 and 413 and substantially encircled by perimeter seal 414 is a compartment 415. Also partially sealed between sheets 412 and 413 on front edge 427A is port 416 that is tubular and communicates with compartment 415. In the depicted embodiment, port 416 includes a collar 417 and a stem 418 outwardly projecting therefrom. Stem 418 is typically barbed and during use is coupled with the end to tubing section 204B of line set 174A (Figure 11) so as to provide fluid communication between line set 174A and compartment 415 of isolation bag 206. The size of compartment 415 depends on the intended use and typically has a volume that is at least or less than 10 mL, 40 mL, 100 mL, 200 mL, 400 mL, 600 mL, 800 mL, 1,200 mL, 1,400 mL or is in a range between any two of the foregoing volumes. Other volumes can also be used depending on the intended use.

Sheets 412 and 413 are typically comprised of a flexible polymeric film. The film can be single ply but more commonly comprises multiple layers that are laminated or co-extruded. For example, each sheet can comprise between 3-9 layers. The film for sheets 412 and 413 commonly have a thickness in a range between 4 mil- 15 mil with between 4 mil - 10 mil being more common. Furthermore, the film is typically sufficiently flexible that it can be rolled into a tube without plastic deformation and/or can be folded over an angle of at least 90°, 180°, 270°, or 360° without plastic deformation. The film is typically approved for contact with living cells and can be sterilized by irradiation. One example of an extruded material that can be used in the present invention is the Thermo Scientific CX3-9 film available from Thermo Fisher Scientific. The Thermo Scientific CX3-9 film is a three-layer, 9 mil cast film produced in a cGMP facility. The outer layer is a polyester elastomer coextruded with an ultra-low-density polyethylene product contact layer. Another example is the Thermo Scientific CX5-14 cast film also available from Thermo Fisher Scientific. The Thermo Scientific CX5-14 cast film comprises a polyester elastomer outer layer, an ultra-low-density polyethylene contact layer, and an ethylene vinyl alcohol (EVOH) barrier layer disposed therebetween.

The above discussion with regard to the formation, materials and properties of isolation bag 206 is also applicable to the other bags disclosed herein including mixing bag 210, collection bag 212 and media bags 216. However, depending on the bag and the intended use, the bags can also have multiple ports and different port configurations. Furthermore, in some applications, the bags can be three dimensional bags as opposed to pillow type bags and can have different volumes.

Isolation bag 206 has an outer perimeter edge 419 that typically has a size and shape comparable to a perimeter edge 386 of support plate 380 of platform 290. Having this similar configuration makes is easy to properly position and align isolation bag 206 on support plate 380. For example, in one embodiment, when isolation bag 206 is centered on support plate 380, any gap between perimeter edges 386 and 419 is typically less than 10 mm, 8 mm, 6 mm 4 mm or 2 mm. However, other gaps can also be used.

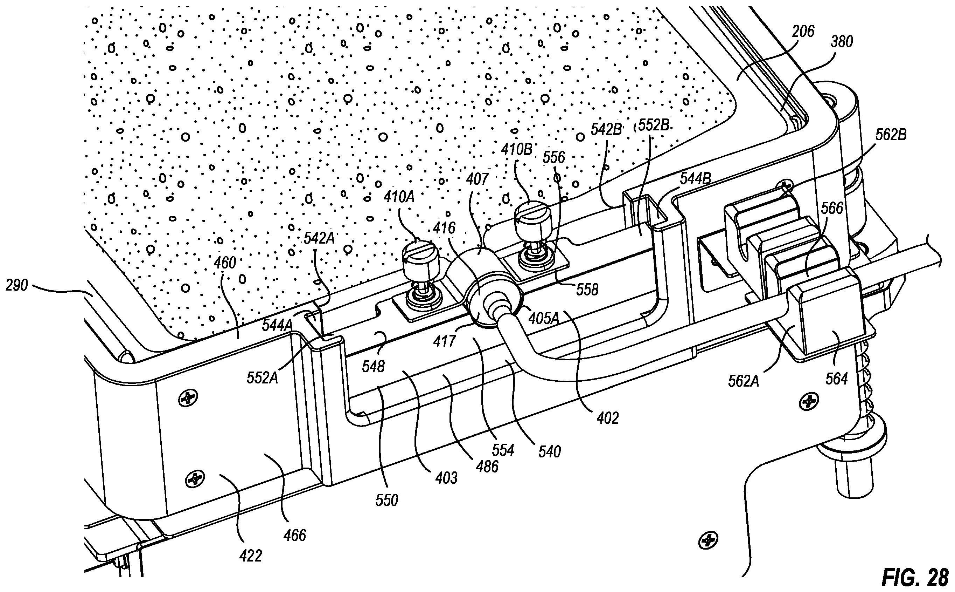

During assembly, as depicted in Figure 28, isolation bag 206 is placed on platform 290/support plate 380 while collar 417/port 416 is received within lower capture groove 405A of clamp base 403. The back of collar 417/port 416 can be butted against the face of front wall 394 of platform 290 to help ensure proper centering and alignment of isolation bag 206 on platform 290. Clamp closure 407 is then secured to clamp base 403, as discussed above, so that collar 417/port 416 is securely clamped between clamp closure 407 and clamp base 403, thereby both centering isolation bag 206 on platform 290 and helping to limit any unwanted movement during use. Proper centering of isolation bag 206 on platform 290 helps to improve the efficiency at which the magnetic field produced by magnet assembly 294 is applied to beads 179 within isolation bag 206.

Once isolation bag 206 is properly positioned on platform 290 and secured to clamp assembly 402, lid 426 is moved to the closed positioned, as shown in Figure 2, and secured in place by latches 518. Projections 431A and 431B, as shown in Figure 15, project from lid plate 499 and extend over the top of channels 544A and 544B (Figure 28) so as to lock clamp assembly 402 within recess 486. As isolation bag 206 is filled with fluid, isolation bag 206 expands causing collar 417/port 416 to raise relative to platform 290. The ability of clamp assembly 402 to freely slide vertically up and down within channels 544A and 544B of cover housing 422 as isolation bag 206 is filled and emptied enables port 416 to remain properly orientated relative to the remainder of isolation bag 206, *e.g.,* helps prevent kinking or folding of isolation bag. This helps to ensure that fluid can properly flow into and out of isolation bag 206. Although cover housing 422/cover assembly 421 also adjusts up and down as isolation bag 206 is filled and emptied, as discussed above, because port 416 is centrally maintained between overlapping sheets 412 and 413 of isolation bag 206, port 416 moves vertically at a different rate than cover housing 422/cover assembly 421.

One or more bubble sensors, such as bubble sensors 562A and 562B, can also be secured to front wall 466 of cover housing 422. Bubble sensors 562 communicate with electrical circuitry 53 (Figure 4) and have a body 564 with a U-shaped channel 566 extending therethrough. Channel 566 is configured to snuggly and removably receive tubing 200, such as tubing section 204B extending from isolation bag 206, and functions to detect when air or liquid is flowing through tubing section 204B for use in calculating the quantity of fluid flowing into and/or out of isolation bag 206.

In some applications it can be desirable to use an isolation bag having a smaller volume. For example, where a small volume of cell culture is being processed, it can be desirable in some situations to concentrate the cells in an isolation bag having a volume smaller than isolation bag 206. By using smaller bags for smaller volumes of cell culture being processed, it is easier to control flow of the cell culture within the bag, thereby optimizing mixing of the cells and beads, as discussed below. The use of smaller bags can also assist in controlling flow of the cell culture out of the bag. Furthermore, there is a lower risk of dead space within the bag that can potentially be damaging to the cells.

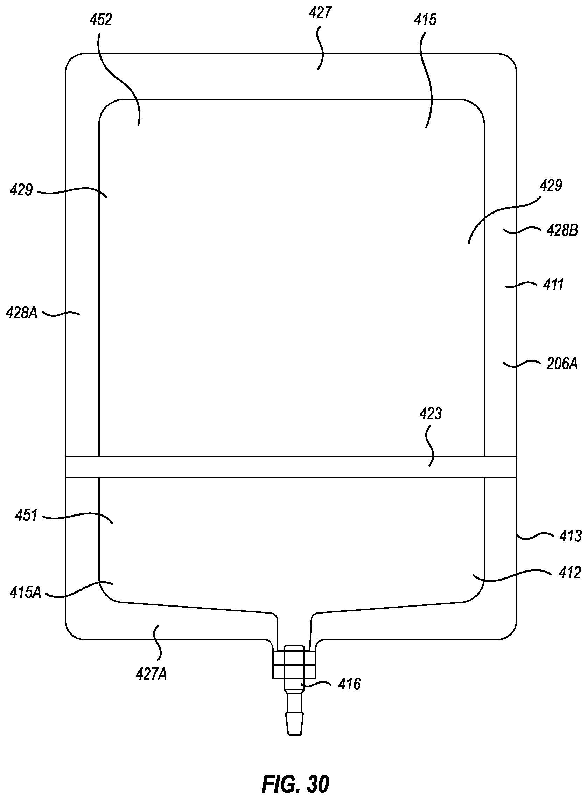

Although an isolation bag having a smaller volume and corresponding smaller overall shape could be used, such a bag can be problematic in that it can be difficult to properly center and retain on platform 290. Furthermore, it can be expensive to design, produce and stock bags of different sizes. Accordingly, one of the unique features of one embodiment of the present disclosure is the design of isolation bags where all of the isolation bags have the same outer size and geometer but where some of the bag have been further processed to reduce the internal volume of compartment 415. For example, depicted in Figure 30 is an alternative isolation bag 206A wherein like elements between isolations bags 206 and 206A are identified by like reference characters. Isolation bag 206A is identical in size, shape, material, and properties to isolation bag 206 except that an additional seal line 423 has been formed that seals sheets 412 and 413 together and that extends laterally across compartment 415. Specifically, seal line 423 extends laterally between sealed side edges 428A and 428B. Seal line 423 divides compartment 415 into an active compartment 451 and an inactive compartment 452. That is, as a result of seal line 423, fluid entering isolation bag 206A through port 416 can only occupy active compartment 451 that is bounded between seal line 423 and front edge 427A. The fluid is prevented by seal line 423 from passing into inactive compartment 452. Active compartment 451 comprises only a portion of and thus is small than compartment 415.

Seal line 423 can be formed using a conventional film bonding technique, such as those discussed above with regard to sealing the perimeter edge of isolation bag 206. Furthermore, seal line 423 can be formed as part of the initial production process of forming isolation bag 206A or can be added later after initial isolation bag 206 has been formed. Isolation bag 206A also includes one or more vent holes 429 that pass through sheet 412 and/or 413 and into inactive compartment 452. Vent holes 429 permit air that may have been captured within inactive compartment 452 during production to escape so that isolation bag 206A will lay flat. Depending on the intended use, the volume of active compartment 451 can be selectively adjusted during production by moving seal line 423 toward front edge 427A or toward back edge 427B.

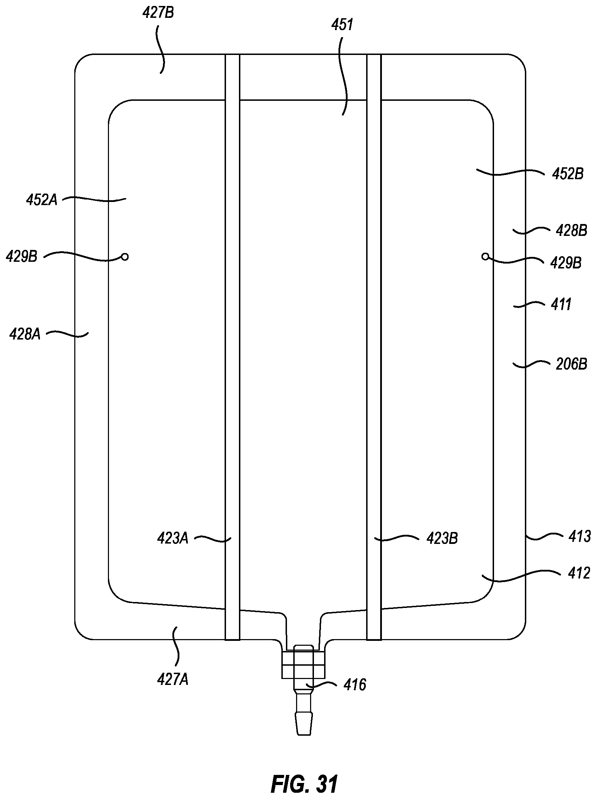

Other alternative isolation bags can be also be formed by placing one or more seal lines as different locations. For example, depicted in Figure 31 is an isolation bag 206B where like elements between isolation bag 206 and 206B are identified by like reference characters. Again, isolation bag 206B can be identical in size, shape, material, and properties to isolation bag 206 except that additional spaced apart seal lines 423A and 423B have been formed that seal sheets 412 and 413 together and that extend between sealed front edge 427A and sealed back edge 427B on opposite sides of port 416. Seal line 423A and 423B divide compartment 415 into an active compartment 451 and two inactive compartment 452A and 452B. Active compartment is bounded between seal line 423A and 423B. Inactive compartment 452A is bounded between seal line 423A and side edge 428A while inactive compartment 452B is bounded between seal line 423B and side edge 428B. As a result of seal lines 423A and 423B, fluid entering isolation bag 206B through port 416 can only occupy active compartment 451 and cannot access inactive compartment 452A and 452B.

Isolation bag 206B also includes at least one vent hole 429A that passes through sheet 412 and/or 413 and into inactive compartment 452A and at least one vent hole 429B that passes through sheet 412 and/or 413 and into inactive compartment 452B. Vent holes 429 permit air that may have been captured within inactive compartment 452A and 452B during production to escape so that isolation bag 206B will lay flat. Depending on the intended use, the volume of active compartment 451 can be selectively adjusted during production by moving seal line 423A and 423B toward each other or laterally outward toward side edges 428A and 428B.

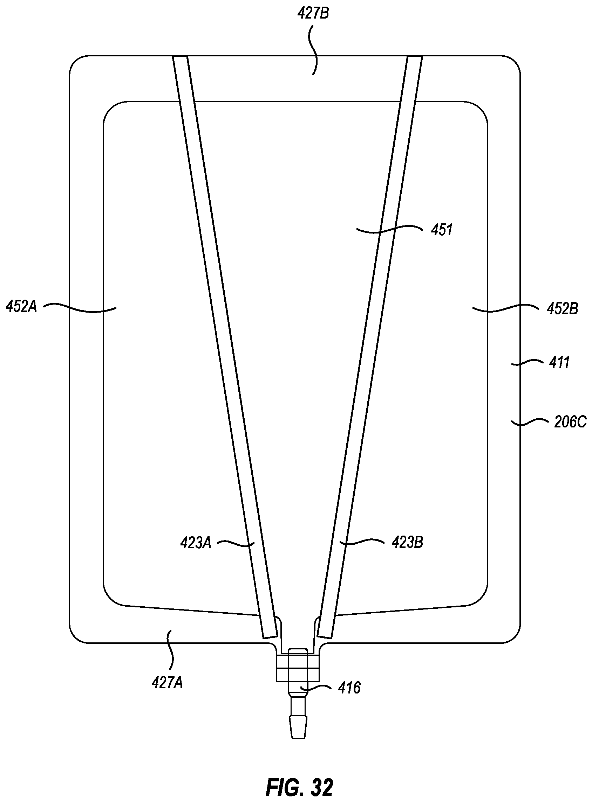

In the depicted embodiment of isolation bag 206B, seal lines 423A and 423B are disposed in parallel alignment. Depicted in Figure 32 is another alternative embodiment of an isolation bag 206C wherein like elements between isolation bag 206B and 206C are identified by like reference characters. Isolation bag 206C is identical to isolation bag 206B except that in contrast to being parallel, seal lines 423A and 423B now slope outward away from each other as they extend from front edge 427A to back edge 427B. The remainder of the above discussion with regard to isolation bag 206B is thus also applicable to isolation bag 206C. In other embodiments, combination of the foregoing seal lines can be used and or seal lines can be formed in other orientations or configurations so as to decrease the volume of compartment 415. In each embodiment, however, it is typically preferred to keep the active compartment centered on the bag to help maximize the effect of the magnetic field produced by magnet assembly 294 that can be applied thereto.

In addition to the above, the various disclosed isolation bags also have a number of other unique features. For example, in some embodiments, each isolation bag is only formed with a single port 416 as opposed to having two or more ports. Having a single port through which all fluid flows in and out, helps to ensure that liquid, beads, and/or cells do not collect in an unused port where they can stagnate and/or potentially release when undesired. Furthermore, the seal lines, particularly those having the V-shaped configuration shown in Figure 32, help to guide liquid, beads, and cells to port 416 to help ensure that all of the material is easily and efficiently removed from the bag. Commercially available products that may be used as isolation bags, as well as other bags, is available from OriGen Biomedical (cat. nos. CSD400Y9 and CSD2500Y14). One advantage of such bags in that they have little to no corner space near port 416 where beads and fluid can collect, potentially resulting in retention within the bag.

In light of the above discussion of the components of bead processing system 14, one example of the method of using bead processing system 14 will now be described. Initially, cover panel 100 of bead processing apparatus 22 is moved to the open position to expose support panel 92. A sterilized consumable kit 170A is then nested onto support panel 92. As part of this nesting process, tubing 200 of line set 174A is engaged with pinch valves 128, pumps 124 and bubble sensors 136. Likewise, air filter assemblies 218 are coupled with rotational assemblies 132. Vial 166 containing bead mixture 177 is fluid coupled with bead vial coupler 208 and is mounted to bead vial retainer 148. Lid 426 of rocker drive 234 is also moved to the open position and isolation bag 206 placed on platform 290/support plate 380 and secured to clamp assembly 402 for proper centering thereon. Lid 426 is then moved to the closed position and secured closed by latches 518. Cover panel 100 can also be moved to the closed position so as to cover consumable kit 170A. If not previously done, the desired number of media bags 216 containing media can be fluid coupled to tubing sections 204H-204J and suspended from catches 99 of bag stands 36 or otherwise suspended. Finally, inlet end 202 of tubing section 204A can be fluid coupled to a source for delivering a cell culture to line set 174A. The source can be cell separator 12 or to some other container or source. Outlet end 211 of tubing section 204F if fluid coupled with cell expansion system 16, a container, or some other downstream processing system for either colleting or processing the isolated and active T-cells that are produced by bead processing system 14. It is appreciated that each of the foregoing assembly steps can be performed in a variety of different orders.

Next, bead pressing apparatus 22 is activated such as through display screen 56 or some other user interface. Each of the following process steps can be performed either automatically through the control of pre-programmed electrical circuitry 53 or can be controlled manually through manual inputs to a user interface. Upon activation of bead processing apparatus 22, all of pinch valves 128 are typically moved to a closed position so as to preclude fluid flow through the tubing section coupled with the pinch valves. In the following method steps where it is discussed that select pinch valves are opened, it is understood that the remaining pinch valves remain closed to control fluid flow through line set 174A. The following step are primarily made with reference to Figure 11.

Step 1: Inject air into isolation bag 206 for partial inflating. This can be accomplished by opening pinch valve 128B and air filter assembly 218A and activating pump 124A. Pump 124A draws air into tubing section 204A through air filter 221A and then pumps the air up through tubing section 204B to isolation bag 206. Injecting air into isolation bag 206 can substantially improve liquid flow and movement within isolation bag 206 by decreasing contact between the liquid and the surface of isolation bag 206. As such, the injected air, as discussed below, can assist with mixing of the cells and magnetic beads.

Based on the operation of different pinch valves 128, it is appreciated that fluids can travel through a variety of different paths to achieve an intended function. As such, the described process steps set forth herein are only examples and other process steps can be used to achieve the same function.

Step 2: Prime tubing and isolation bag 206 with media. Pinch valves 128B and 128G can be opened while pump 124B is used to pass media from media bag 216C, through pump 124B, bubble sensors 136A and 136C, up through tubing section 204B and into isolation bag 206.

Step 3: Inject air into mixing bag 210. Air filter assembly 218B and pinch valve 128E can be opened and pump 124B used to pump air from air filter 221B, through pump 124B and pinch valve 128E to mixing bag 120. Again, injecting air within mixing bag 120 can help facilitate mixing of fluid therein.

Step 4: Suspend beads 179 within vial 166. This can be accomplished by activating motor 162 to facilitate rotation of vial retainer 148 and vial 166 as previously discussed. In other embodiments, such as where beads 179 are retained within a suspended bag or other container, it may not be necessary to manually or mechanically suspend beads 179 prior to injection.

Step 4: Inject suspended bead 179 from vial 166 into mixing bag 210. Pinch valves 128C, 128D, and 128F can be opened and pump 124B used to pump bead mixture 177 from vial 166, through pinch valves 128C, 128D, and 128F and into mixing bag 210.

Step 5: Inject media into mixing bag 210. Pinch valve 128G and 128E can be opened and pump 124B used to pump media from media bag 216C through pinch valves 128G and 128E and into mixing bag 210. It is appreciated that the media could have been drawn from any of media bags 216A-C or any combination thereof. For simplicity, media in the present steps will be drawn from media bag 216C with the understanding that it could likewise be drawn from any of media bags 216A-C.

In each of the steps disclosed herein wherein a fluid or suspension is passed through tubing and into a bag or container, quantities of fluid/suspension being delivered can be measured based on known factors such as lengths of tubing, size of tubing, duration of pump operation and detections made by the bubble sensors as to the transition between the flow of gas and liquid through the tubing. Based on these factors, it can be determined as to when to open one of air filter assemblies to dispense filtered air into the tubing. One or more of the pumps can then be used pump the air so as to push the measured quantity of fluid/suspension in the container while also assisting in removing liquid from the tubing. Using air to remove liquid from the tubing helps to facilitate the precise measurement of future quantities of liquid/suspension to be passed through the tubing and into a bag or container.

Step 6: Mix beads 179 within mixing bag 120. Pinches valves 128E and 128F can be opened and pump 124B used to continuously flow the mixture within mixing bag 210 out through tubing section 204E, through pump 124B and back into mixing bag 120 through tubing section 204D in a continuous loop. This flow homogeneous suspends beads 179 with the media.

Step 7: Transfer beads 179 from mixing loop into isolation bag 206. Pinch valves 128F and 128B can be opened and pump 124B used transfer the suspension containing beads 179 from mixing bag 210, through pinch valve 128F and pinch valve 128B into isolation bag 206. It is noted that the number of beads 179 dispensed into the isolation bag 206 can depend on the number of cells that are to be delivered from cell separator 12 or other container into isolation bag 206. Thus, not all of beads 179 within mixing bag 210 and/or vial 166 may be disposed into isolation bag 206. Rather, in one embodiment, the number of beads 179 delivered into isolation bag 206 may be substantially equal to or only slightly larger than the number of cells that are to be delivered into isolation bag 206. Furthermore, in one alternative embodiment, mixing bag 210 can be eliminated and beads 179 can be directly dispended into isolation bag 206 from vial 166 or from some other bag or container housing beads 179 and fluid coupled with the tubing. As isolation bag 206 begins to fill with fluid, isolation bag 206 expands between cover assembly 421 and platform 290/support plate 380 so as to raise cover assembly 421.

Step 8: Flush tubing containing beads 179 into isolation bag. Pinch valves 128B and 128G can be opened and pump 124B used to pass media from media bag 216C through tubing into isolation bag 206.

Step 9: Move magnet assembly 294 to raised activation position and activate rocketing. Lift assembly 292 is activated to raise magnet assembly 294 to the raised activation position relative to platform 290/support plate 380. Concurrently or consecutively with the movement of magnet assembly 294, rocker drive 234 is activated to facilitate repeated rocking of platform assembly 232 having isolation bag 206 mounted thereon. As beads 179 mix within isolation bag 206 by rocking, they are attracted to and held against platform 290/isolation bag 206 by the magnetic force produced to magnet assembly 294. If desired, stop 534A could be moved to the advanced restraining position prior to filling of isolation bag 206. Although this would increase the rate at which beads 179 are attracted to platform 290/support plate 380, it also would limit that amount of fluid that could be processed within isolation bag 206.

In one method of operation, the magnetic field can be applied after completion of the rocking. For example, prior to, concurrently with, or after pumping media into isolation bag 206, rocker drive 234 can be activated to facilitate repeated rocking of platform assembly 232 having isolation bag 206 mounted thereon. This rocking can help unbind any agglomeration of beads 179 and suspend any unwanted matter. Rocker drive 234 can then be deactivated and lift assembly 292 activated to raise magnet assembly 294 to the raised activation position relative to platform 290/support plate 380. Beads 179 settle under gravity and are attracted to and held against isolation bag 206//support plate 380 by the magnetic force produced to magnet assembly 294. In one embodiment, rocker drive 234 can rearwardly or negatively tilt platform assembly 232/isolation bag 206 so that port 416 of isolation bag 206 is elevated. A small amount of air or media can then be passed through port 416 and into isolation bag 206 to help ensure that no beads 179 are retained within port 416. The magnetic field can then be applied to isolation bag 206 while in this rearward tilt position.

Step 10: Transfer liquid from isolation bag 206 into collection bag 212. Rocker drive 234 can be controlled to forward or positive tilt platform 290 so that port 416 is disposed lower than the remainder of isolation bag 206. This orientation helps to ensure that fluid freely flows out of isolation bag 206 through port 416. Pinch valves 128B and 128J can be opened and pump 124 used to transfer fluid from within isolation bag 206, through pinch valves 128B and 128J and into collection bag 212. Beads 179 are retained within isolation bag 206 under the magnetic force of magnet assembly 294. The above steps are used as a pre-wash beads 179 to remove any free antibodies in the mix not covalently bound to beads 179. The above washing of beads 179 can be repeated as needed.

Step 11: Transfer media into isolation bag 206. Pinch valves 128B and 128G can be opened and pump 124B used to transfer a defined quantity of media from media bag 216C into isolation bag 206 so that beads 179 are diluted to a desired concentration.

Step 12: Mix beads 179 within isolation bag 206. Lift assembly 292 is activated to lower magnet assembly 294 to the deactivation position and rocker drive 234 is activated to repeatedly rock platform 290/support plate 380 and isolation bag 206 disposed thereon, thereby homogenously mixing beads 179 within the media.

Step 13: Transfer cell culture to isolation bag 206. Pinch valves 128A and 128B can be opened and pump 124A used to transfer the cell culture from cell separator 12 or some other container or source connected to inlet end 202 into isolation bag 206.

Step 14: Facilitate isolation and activation of desired T-cells. Rocker drive 234 can be activated or remains activated from Step 12 to facilitate rocking of platform 290/support plate 380 and isolation bag 206 thereon which mixes beads 179 with the cell culture containing the desired T-cells. Beads 179 having a desired antibody thereon will bind to and activate desired T-cells as beads 179 come in contact with the desired T-cells during the mixing process. Such mixing can occur for an extended period of time. By way of example, and not by limitation, the mixing can be between 15 minutes to 60 minutes and more commonly between 20 minutes to 40 minutes. Other time durations can also be used.

Step 15: Flush port 416. With rocker drive 234 deactivated, pinch valves 128B and 128G can be opened and pump 124B used to pump a small quantity of media from media bag 216 into isolation bag 206 so as to remove any cells and/or beads 179 that may have been caught within port 416. This flushing of port 416 can occur while rocker drive 234 rearwardly tilts platform 290/support plate 380 and isolation bag 206 so that port 416 is elevated.

Step 16: Capture T-cells bound with beads 179. Lift assembly 292 can then be activated to raise magnet assembly 294 relative to platform 290/support plate 380 to the activation position. The magnetic force produced by magnet assembly 294 causes beads 179 and T-cells bound to beads 179 to be held against platform 290/support plate 380 while retained within isolation bag 206.

Step 17: Transfer liquid from isolation bag 206 into collection bag 212. Rocker drive 234 can positively tilt platform 290 so that port 416 is downwardly positioned. Pinch valves 128B and 128J can be opened and pump 124B used to pump fluid from isolation bag 206 into collection bag 212 while beads 179 and the cells attached thereto remain securely retained within isolation bag 206 under the magnetic force produced by magnet assembly 294. This step is to remove the negative cell fraction, *i.e.,* the cells that did not bind to a bead. from isolation bag 206.

Step 18: Deliver media to isolation bag 206. Pinch valves 128B and 128G can be opened and pump 124B used to pump media from media bag 216 into isolation bag 206.

Step 19: Wash cells bound with beads 179. Lift assembly 292 is activated to move magnet assembly 294 down to the deactivation position. Consecutively or concurrently, rocker drive 234 is activated to facilitate mixing of the beads 179 with bound T-cells in the freshly delivered media. This mixing can again occur for an extended period of time. However, the primary objective of this rocking/mixing is to free any cells or other biological material that may have been unintentionally captured within isolation bag 206 so that it can be removed.

Step 20: Remove liquid from isolation bag 206. Lift assembly 292 is activated to move magnet assembly 294 up to the activation position so as to again capture beads 179 and the T-cells bound thereto. Rocker drive 234 positively tilts platform 290 so that port 416 is downwardly positioned. Pinch valves 128B and 128G can be opened and pump 124B used to pump liquid from isolation bag 206 to collection bag 212. The washing set forth in Steps 18-20 can be repeated as many times as desired.

Step 21: Deliver media into isolation bag 206. Rocker drive 234 is controlled to disengage tilt and lift assembly 292 is activated to lower magnet assembly 294 to the disengaged position. Pinch valves 128B and 128G can be opened and pump 124B used to pump media from media bag 216 into isolation bag 206. The quantity of media delivered is dependent upon the desired concentration for the T-cells as they are dispensed out of the system.

Step 22: Mix cells within isolation bag 206. Rocker drive 234 is activated to mix beads 179 with T-cells attached thereto within the freshly delivered media so as to produce homogenous mixture.

Step 23: Transfer suspension within isolation bag 206 to cell expansion system 16 or other downstream processing equipment or collection container. Pinch valves 128A and 128K can be opened and pump 124B used to transfer suspension within isolation bag 206 to cell expansion system 16 or to other downstream processing equipment or collection container through tubing section 204F. In yet another alternative, the suspension could be returned to the container that originally held the cell culture prior to mixing with the beads and then subsequently moved to cell expansion system 16. The above steps 21-23 can be repeated until the cell concentration within the downstream equipment has reached a desired level.

Returning to Figure 1, after the cells have grown to a desired density or have reached some other predefined condition within cell expansion system 16, one or more detaching reagents may be added to the cell culture to cause the cells to detach from beads 179. Alternatively, as previously discussed, the cells can be allowed to expand until the cells automatically detach from beads 179. In this case, no detaching reagent is needed. In other embodiments, the cells with beads 179 can be manually or mechanically agitated, such as within a bag, to help facilitate detachment of the cells from beads 179. The resulting suspension of beads, cells and media is then transferred to bead processing system 18 to separate beads 179 from the cells. In one embodiment, bead processing system 18 is the same as bead processing system 14 except that bead processing system 18 is used with a consumable kit 170B that is modified relative to consumable kit 170A. However, the same bead processing apparatus 22 can still be used. For example, the suspension could be looped back to the same bead processing system 14 that has been fitted with a different consumable kit 170B or could be transferred to a different bead processing system 18 that includes an identical bead processing apparatus 22 but is fitted with consumable kit 170B. In other embodiments, bead processing system 18 could be otherwise modified relative to bead processing system 14.

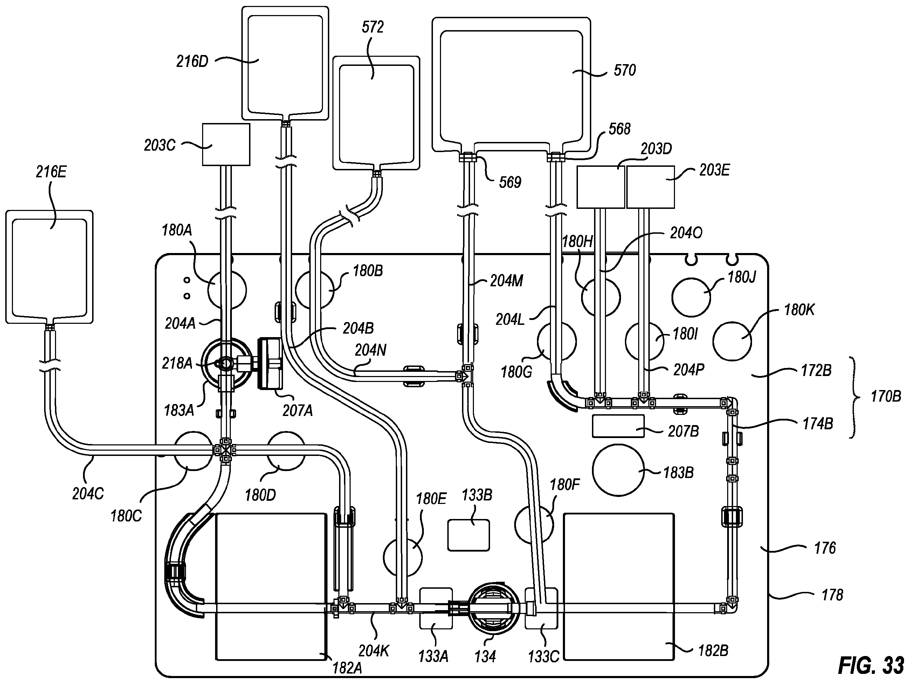

As depicted in Figure 33, consumable kit 170B includes a tray 172B and a line set 174B. Tray 172B is substantially similar to tray 172A and like elements between trays 172A and 172B are identified by like reference characters. Try 172B is again in the form of a plate that is configured to nest on top of support panel 92 (Figure 4). As such, tray 172B has top face 176 and opposing bottom face 178 with openings 180 thereon for receiving pinch valves 128, opening 182 to receive pumps 124, openings 183 to receive rotational assemblies 132, openings 133 to receive bubble sensors 136, and opening 134 to receive pressure sensor 140. Again, as support panel 92 and the components mounted thereon are modified, trays 172 can likewise be modified. Tray 172B differs from tray 172A in that it does not include bag restrainer 192 upstanding from top face 176 and, because of changes in the line set, the position of tube restraints 186 have been modified.

Line set 174B is also substantially similar to line set 174A and like elements between line sets 174A and 174B are identified by like reference characters. Line set 174B includes tubing section 204A that extends between inlet end 202 and tubing section 204K. Disposed at inlet end 202 is a connector 203C which, in this case, can be coupled with cell expansion system 16, *e.g.,* a bioreactor or container thereof, or can be coupled with a separate container or separate source for receiving a culture comprised of cells, beads 179 and a liquid medium. Air filter assembly 218A is disposed on tubing section 204A. Tubing section 204B now has the first end coupled to a media bag 216D housing a liquid medium and an opposing second end coupled with tubing section 204K. Similarly, tubing section 204C has a first end coupled with a media bag 216E housing a liquid medium and an opposing second end that is now coupled directly with tubing section 204K rather than tubing section 204B.

Line set 174B also other new tubing sections. Specifically, line set 174B includes a tubing section 204L having a first end connect to a first port 568 of a bead separation bag 570 and an opposing second end connected to the end of tubing section 204K. A tubing section 204M has a first end connected to a second port 569 of bead separation bag 570 and an opposing second end connected to tubing section 204K upstream of the connection with tubing section 204L. Bead separation bag 570 will be discussed below in greater detail and is another example of a processing bag. A tubing section 204N has a first end connected to a bead waste bag 572 and an opposing second connected to tubing section 204M. Finally, tubing sections 204O and 204P are provided. Tubing section 204P has a first end coupled to a connector 203D and an opposing second end coupled with tubing section 204L while tubing section 204P has a first end coupled to a connector 203E and an opposing second end coupled with tubing section 204L. Connectors 203C-D can comprise any of the connectors previously discussed with regard to connector 203A. Connectors 203D and E can be coupled with gene editing system 20, a container, or other downstream processing equipment in which it is desired to receive the cells separated from beads 179. In alternative embodiments, only one of tubing sections 204O and 204P may be required. As with line set 174A, line set 174B is typically preassembled with tubing 200 and bags 216, 570, and 572, being sterilized, such as by irradiation, as a closed system. Tray 172B can be attached to line set 174B prior to or after sterilization. During use, sterile connection processes can be used to connect connectors 203C-203E to their corresponding containers or equipment. In alternative embodiments, one or more of bags 216, 570, 572 can be attached to tubing 200 using a sterile connection process after tubing 200 is sterilized. Bag 216, 570, and 572 can have the same properties and be produced using the same materials and processes previously described with regard to isolation bag 206.

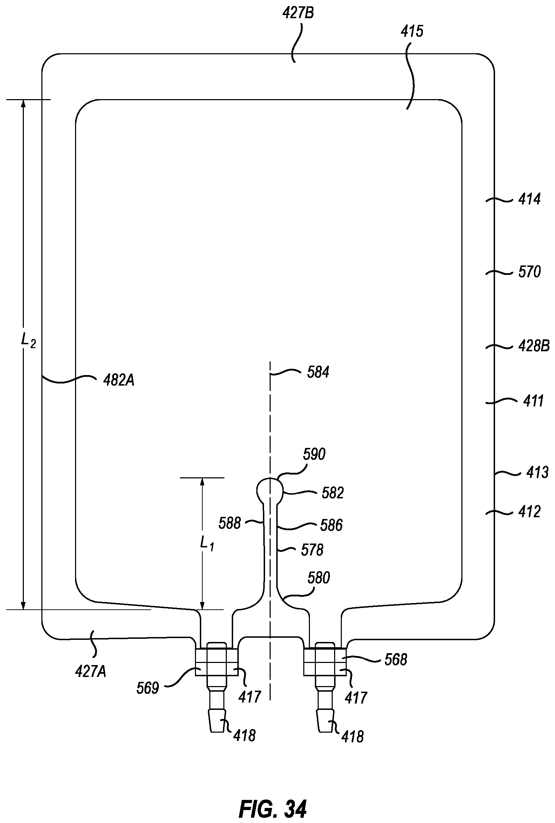

Turning to Figure 34, bead separation bag 570 comprises a bag body 576, ports 568 and 569 and a partition 578. Bag body 576 of bead separation bag 570 can be the same or substantially the same sizes, configuration, materials, properties and alternatives as bag body 411 of isolation bag 206. As such, like elements between bag bodies 411 and 576 are identified by like reference characters. For example, bag body 576 is typically a collapsible, pillow type bag comprised of top sheet 412 that overlies bottom sheet 413. Sheets 412 and 413 are bonded together around their perimeter edge to form perimeter seal 414. The perimeter edge can comprise front edge 427A, back edge 427B, and opposing side edges 428A and 428B extending therebetween. The bonding can comprise using methods known in the art such as welding with heat energies, radiofrequency (RF) energies, sonic energies or sonics, or other sealing energies or by use of an adhesive. Bounded between sheets 412 and 413 and substantially encircled by perimeter seal 414 is a compartment 415.

Partially sealed between sheets 412 and 413 on front edge 427A are ports 568 and port 569. Ports 568 and 569 are spaced apart and disposed on opposite sides of partition 578. Furthermore, ports 568 and 569 are tubular and communicate with compartment 415. In the depicted embodiment, each port 568 and 569 includes collar 417 and stem 418 outwardly projecting therefrom. Each stem 418 is typically barbed, although not required. During use, stem 418 of port 568 is coupled with tubing section 204L and stem 418 of port 569 is coupled with tubing section 204M of line set 174B (Figure 33) so as to provide fluid communication between line set 174B and compartment 415 of bead separation bag 570. Although only ports 568 and 569 are shown on bag body 576, in other embodiments, 3, 4, or more ports can be disposed on bag body 576 so as to communicate with compartment 415.

Partition 578 has a first end 580 that is connected to perimeter seal 414 of front edge 427A and extends toward back edge 427B to a terminal second end 582. In one embodiment, partition 578 is linear and extends along a linear axis 584 that is centrally disposed between side edges 428A and 428B and bisects compartment 415. Partition 578 has opposing sides 586 and 588 that extend along a length L₁ of partition between opposing ends 580 and 582. In one embodiment, at least a portion of opposing sides 586 and 588 are disposed in parallel alignment. In another embodiment, for at least a majority of the length L₁ of partition, opposing sides 586 and 588 are disposed in parallel alignment. Second end 582 can comprise an enlarged area 590 that protrudes perpendicular to partition 578 that is at least 75%, 100%, 150%, 200% or 250% wider than a width between opposing sides 586 and 588 spaced from enlarged area 590. Enlarged area 590 is designed to deflect fluid traveling along the length of partition 578 away from the center of compartment 415. This deflection increases the distance that fluid needs to travel between ports 568 and 569. Using the schematic of Figure 34 for purposes of illustration, assume the distance between the center of ports 568 and 569 is 25 mm and the length of partition 578 is 30 mm, then the shortest distances that fluid could travel in compartment 415 of bead separation bag 570 between ports 568 and 569 is 25 mm when partition 578 is not present but about 66 mm when partition 578 is present. Partition 578 thus results in an increase in the shortest distance fluid could travel in compartment 415 between ports 568 and 569 by 2.64 fold. The use of enlarged area 590 further increases the minimum distance that fluid must travel to pass between ports 568 and 569. Partition 578 can be of any number of shapes that result in increases in the shortest distances that fluid could travel in compartment 415between ports 568 and 569. Such increases in the shortest distances that fluid could travel in compartment 415 between ports 568 and 569 may be from about 1 to about 50 *(e.g.,* from about 1 to about 50, from about 2 to about 50, from about 3 to about 50, from about 4 to about 50, from about 8 to about 50, from about 2 to about 20, from about 2 to about 30, from about 2.5 to about 10, from about 8 to about 30, from about 10 to about 50, etc.) fold.

The length L₁ of partition 578 can be dependent on a number of factors such as fluid flow rate, the size of ports 568 and 569 and the size of compartment 415. L₁ is at least 5 mm, 10 mm, 20 mm, 40 mm, 50 mm, 60 mm, 65 mm, 70 mm, 75 mm, 80 mm, 100 mm, 150 mm, 175 mm, or 200 mm or is in a range between any two of the foregoing values. In another embodiment, compartment 415 has a length L₂ extending between perimeter seal 414 at front edge 427A and perimeter seal 414 at back edge 427B. Length L₁ of partition 578 can be at least 5%, 10%, 15%, 20%, 25%, 30%, 35%, 40%, 50%, or 60% of L₂ or in a range between any two of the foregoing. In other embodiments, length L₁ of partition 578 may extend from about 10% to about 90% (*e.g.,* from about 25% to about 85%, from about 25% to about 80%, from about 25% to about 75%, from about 25% to about 70%, from about 25% to about 65%, from about 25% to about 60%, from about 25% to about 55%, from about 25% to about 50%, from about 25% to about 45%, from about 25% to about 40%, from about 25% to about 35%, from about 40% to about 90%, from about 40% to about 80%, from about 40% to about 70%, from about 50% to about 90%, from about 55% to about 85%, etc.) of the length L₂ of compartment 415.

In one embodiment, partition 578 is formed by welding together overlapping sheets 412 and 413 using one or more of the above described processes for forming perimeter seal 414. In an alternative embodiment, an insert, such as a polymeric insert, can be positioned between overlapping sheets 412 and 413. Sheets 412 and 413 can then be secured to the opposing sides of the insert, such as by welding or adhesive, so as to form partition 578. Partition 578 can also be formed by releasably pressing sheets 412 and 413 together at the location of partition 578 so as to form a sealed engagement between sheets 412 and 413. For example, this could be accomplished through the use of a clamp. Regardless of the specific configuration, partition 578 is configured to restrict fluid communication between port 568 and port 569. Specifically, partition 578 is configured so that beads 179 cannot pass therethrough and, in more common embodiments, partition 578 is liquid tight so that beads 179, cells, liquid media and other biological components disposed within compartment 415 cannot pass through partition 578. Instead, a fluid or other component flowing into compartment 415 through inlet port 568 must pass (or flow) around partition 578, *(e.g.,* second end 582) in order to exit compartment 415 through port 569. Thus, a direct, linear access between ports 568 and 569 is prevented by partition 578.

As a result of partition 578, compartment 415 has or forms a fluid pathway that extends from port 568, around second end 582 of partition 578, to port 569. As previously discussed, the extended length of the fluid pathway, caused by the addition of partition 578, increases the retention or residence time of fluid within compartment 415 as compared to a bag that does not include partition 578. As discussed below in more detail, this increased residence time induces and/or increases exposure of beads 179 disposed in the fluid to the magnetic field produced by magnet assembly 294 within compartment 415 and thus increases the capture of beads 179 within bead separation bag 570. It is appreciated that bead separation bag 570 and partition 578 thereof can have a variety of different configurations and be used in a variety of different ways. Examples of alternative configurations, materials, properties, designs, and uses of bead separation bag 570 are disclosed in US Patent Publication No. 2019/0010435, published January 10, 2019.

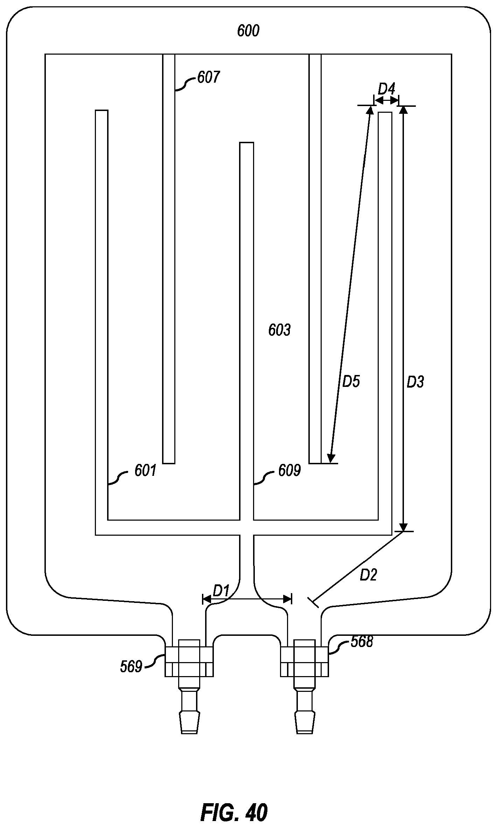

Figure 40 shows bead separation bag 600 that is similar to Figure 34 but has a serpentine flow path 603 through the bag. The serpentine flow path 603 is formed by a combination of two lower flow path partitions 601, two upper flow path partitions 607, and a central flow path partition 609. Bead separation bag 600 is a design that requires material to travel an extended distance through the bag to go from first port 568 to second port 569.

The minimum distance of serpentine flow path 603 for a bead separation bag of the type set out in FIG. 40 can be calculated by twice the distance of length D2, twice the distance of length D3, five times the distance of length D4, and four times the distance of length D2. This is so because the serpentine flow path 603 bead separation bag 600 is generated by five flow path partitions.

Embodiments of the type shown in Figure 40 can be used to increase the residence time induces and/or increases exposure of beads disposed in the fluid to the magnetic field produced by magnet assembly within compartment and thus increases the capture of beads within bead separation bag. The embodiment shown in Figure 40 allow for material passing through bead separation bag 600 to travel a minimum distance from first port 568 to second port 569 that is greater than D1. The distance that materials need to travel is determined by the number of partitions in the bag and the lengths of those partitions. The number of partitions may vary from about one to about fifty (*e.g.,* from about two to about fifty, from about three to about fifty, from about four to about fifty, from about five to about fifty, from about six to about fifty, from about seven to about fifty, from about eight to about fifty, from about three to about thirty, from about four to about twenty, from about five to about ten, from about five to about twelve, from about five to about twenty-five, from about ten to about twenty, from about ten to about thirty, from about fifteen to about forty, etc.). The lengths of the partitions may independently vary from about 60% to about 98% *(e.g.,* from about 60% to about 98%, from about 70% to about 98%, from about 75% to about 98%, from about 80% to about 98%, from about 85% to about 98%, from about 90% to about 98%, from about 60% to about 90%, from about 70% to about 90%, from about 80% to about 90%, from about 60% to about 80%, from about 70% to about 80%, etc.) of the interior length of the bead separation bag.

Further, the bead separation bag may be designed such that the minimum distance of serpentine flow path 603 between first port 568 to second port 569 by way of serpentine flow path 603 is at least is at least twenty (*e.g*., from about twenty to about one thousand, from about thirty to about one thousand, from about forty to about one thousand, from about fifty to about one thousand, from about sixty to about one thousand, from about seventy to about one thousand, from about eighty to about one thousand, from about eighty to about five hundred, from about sixty to about three hundred, from about sixty to about two hundred, etc.) times the distance of D1.

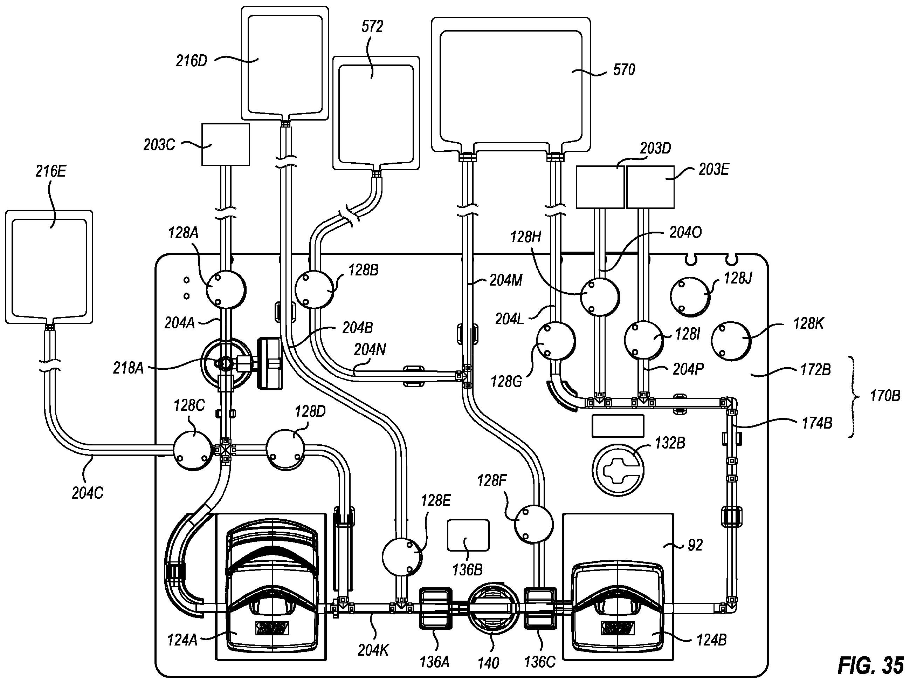

Turning to Figure 35, during use, consumable kit 170B is nested on top of support panel 92 in the same manner, as previously discussed, that consumable kit 170A was mounted on support panel 92. Line set 174B is the coupled with the mechanical components of support panel 92. For example, tubing section 204A is coupled with pinch valve 128A while air filter assembly 218A is coupled with rotational assembly 132A (Figure 5). Connector 203C is connected to cell expansion system 16 or, as discussed above, another source for receiving a culture comprising cells and separated beads 179. Tubing section 204B is coupled within pinch valve 128F while media bag 216D is support on one of bag stands 36 or is otherwise supported. Tubing section 204C is coupled with pinch valves 128C and 128D while media bag 216E is likewise supported on one of stands 36 or is otherwise supported. Tubing section 204K is coupled to pumps 124A and 124B, bubble sensors 136A and 136C, and pressure sensor 140. Tubing section 204L is coupled to pinch valve 128G while tubing section 204M is coupled with pinch valve 128F. As discussed further below, bead separation bag 570 fluid coupled with tubing sections 204L and 204M and is mounted on platform 290. Finally, tubing sections 204O and 204P are coupled with pinch valves 128H and 128I, respectively, while connectors 203D and 203E thereof are coupled with gene editing system 20 or some other downstream container or processing system.

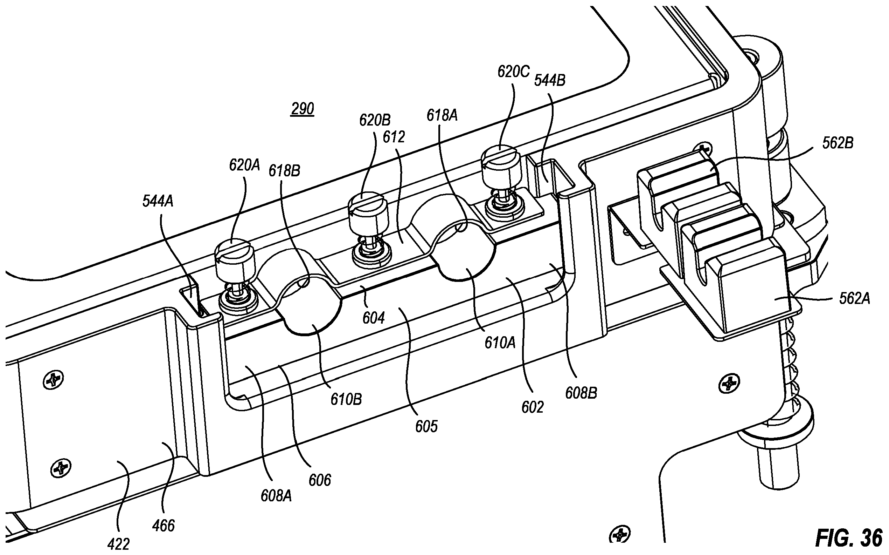

Turning to Figure 36, a clamp assembly 605 is removably disposed on front wall 466 of cover housing 422. Clamp assembly 605 is similar in design to clamp assembly 402 previously discussed with regard to Figure 27 but is specifically designed for engaging with bead separation bag 570. Clamp assembly 605 is used in part for securing and centering bead separation bag 570 on top of platform 290. More specifically, clamp assembly 605 comprises an elongated clamp base 602 having a top surface 604 and an opposing bottom surface 606 that extend between opposing ends 608A and 608B. Ends 608A and 608B are slidably received within channels 544A and 544B of cover housing 422, respectively, so that clamp base 602 can slide vertically within channels 544 but is retrained from lateral movement. Recessed on top surface 604 of clamp base 602 is a first lower capture groove 610A and a spaced apart second lower capture groove 610B.

Clamp assembly 605 also includes a clamp closure 612 that can be removably coupled to clamp base 602. Clamp closure 612 has a top surface 614 and an opposing bottom surface 616. A first upper capture groove 618A and a spaced apart second upper capture groove 618B are recessed into bottom surface 558. Fasteners 620A and 620C pass through clamp closure 612 on opposing sides of upper capture groove 618A and 618B and can selectively secure to clamp base 602 by threaded or other engagement. Likewise, a fastener 620B pass through clamp closure 612 between upper capture grooves 618A and 618B and can selectively secure to clamp base 602 by threaded or other engagement. Fasteners 620 can comprise bolts, screws, clamps, pins or other types of removably fasteners. During assembly, upper capture grooves 618A and 618B align with lower capture grooves 610A and 610B, respectively, as clamp closure 612 is secured to clamp base 602. Lower capture grooves 610 and upper capture grooves 618 each typically have a semi-cylindrical configuration that can be the same size and shape. As such, aligned capture grooves 610 and 618 can form a cylindrical opening. In other embodiments, capture grooves 610 and 618 can other shapes such as semi-polygonal configurations.

It is appreciated that the entirety of clamp assembly 605 is freely slidable within channels 544A and 544B. As such, clamp assembly 605 can be easily removed from cover housing 422 and replaced with different clamp assembly, such as clamp assembly 402 depending on the configuration of bag that is being positioned on platform 290.

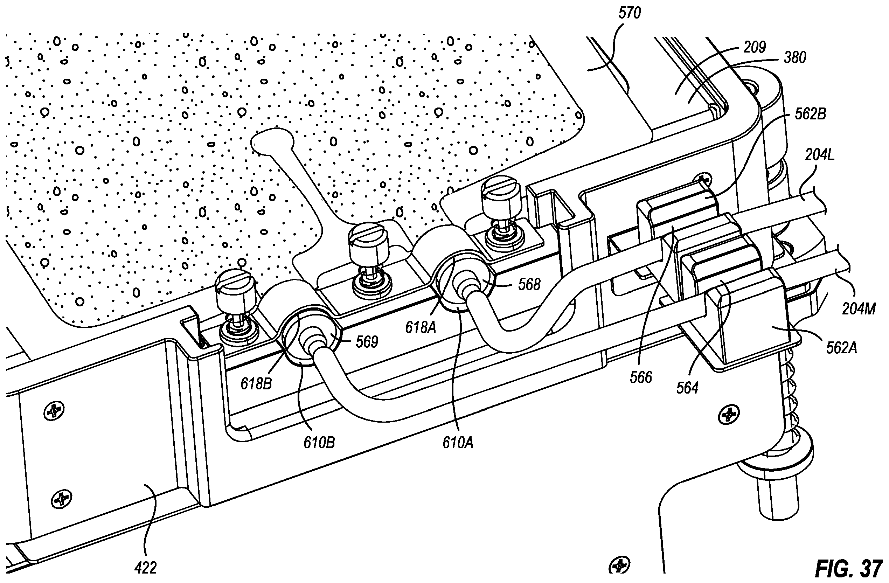

During assembly, as depicted in Figure 37, bead separation bag 570 is placed on platform 290/support plate 380 while collar 417 of port 568 and port 569 are received within lower capture grooves 610A and 610B of clamp base 602, respectively. Although bead separation bag 570 is used in this exemplary embodiment, it is appreciated that bead separation bag 600 or any other bead separation bag discussed herein could be used in the same manner. The back of collars 417 can be butted against the face of front wall 394 to help ensure proper centering and alignment of bead separation bag 570 on platform 290/support plate 380. Clamp closure 612 is then secured to clamp base 602, as discussed above, so that collars 417 are securely clamped between clamp closure 612 and clamp base 602, thereby both centering bead separation bag 570 on platform 290/support plate 380 and helping to limit any unwanted movement during use. As bead separation bag 570 is filled with fluid, bead separation bag 570 expands causing collars 417/ports 568, 569 to raise relative to platform 290/support plate 380. The ability of clamp assembly 605 to freely slide vertically up and down within channels 544A and 544B of cover housing 422 as bead separation bag 570 is filled and emptied enables ports 568 and 569 to remain properly orientated relative to the remainder of bead separation bag 570, *e.g.,* helps prevent kinking or folding of bead separation bag 570. This helps to ensure that fluid can properly flow into and out of bead separation bag 570. Although cover housing 422 also adjusts up and down as bead separation bag 570 is filled and emptied, as discussed above, because ports 568 and 569 are centrally maintained between overlapping sheets 412 and 413 of bead separation bag 570, port 568, 569 move vertically at a different rate than cover housing 422. In the assembled position, tube sections 204L and 204M coupled with ports 568 and 569, respectively, can be removably secured to bubble sensors 562A and 562B. Once separation bag 570 is properly positioned on platform 290/support plate 380, lid 426 is closed and secured in position by latches 518 (Figure 15).

With consumable kit 170B coupled with bead processing apparatus 22, as discussed above, bead processing system 18 is activated, such as through display screen 56 or some other user interface. Each of the following process steps can be performed either automatically through the control of pre-programmed electrical circuitry 53 or can be controlled manually through manual inputs to a user interface. Upon activation of bead processing system 18, all of pinch valves 128 are typically moved to a closed position so as to preclude fluid flow through the tubing section coupled with the pinch valves. In the following method steps where it is discussed that select pinch valves are opened, it is understood that the remaining pinch valves remain closed to control fluid flow through line set 174B.

Step 1: Lift magnet assembly 241. Lift assembly 292 is used to elevate magnet assembly 294 relative to platform 290/support plate 380 to the raised activation position. Stop 534A can be moved to the advanced retraining position so as to limit the ability of cover assembly 421 to rise relative to platform 290/support plate 380.

Step 2: Transfer suspension comprised of cells, beads 179 and media through bead separation bag 570. With reference to Figure 35, pinch valves 128A, 128F, 128G and 128H can be opened. Pump 124A can then be used to transfer the culture comprised of cells, beads 179 and media that is housed within cell expansion system 16 or other container coupled to connector 203C through tubing sections 204A, 204K and 204M and into bead separation bag 570 through port 569. With reference to Figure 34, the suspension then flows within compartment 415 of bead separation bag 570 around second end 582 of partition 578 and then out of compartment 415 through port 568. As the suspension is passing through compartment 415, beads 179 are attracted to and secured against platform 290/support plate 380 by the magnetic field produced by magnet assembly 294. In one embodiment, mixing of the suspension can be produced by the activation of rocker drive 234 which can help move beads 179 closer platform 290/support plate 380 where they are better captured by the magnetic field. In other embodiment, however, the debeading can be performed without activation of rocker drive 234. The positioning of stop 534A in the advanced restraining position, limits the expansion of bead separation bag 570 so as to further optimize the force of the magnetic field on beads 179. The forgoing enables the efficient collection of beads 179 against platform 290/support plate 380 as the suspension continuously flows through bead separation bag 570. The media and cells leaving separation bag 570 through port 568 travels along tube section 204L and then to gene editing system 20 or some other container or system through tubing section 204O.

In one modified version of Step 2, pinch valves 128G and 128H can remain closed until bead separation bag 570 is at least 30%, 40%, or 50% filled with the mixture of cells, beads 179 and media. Rocker drive 234 then tilts platform 290/support plate 380 so that ports 568 and 569 are upwardly tilted. Pinch valves 128G and 128H are then opened. As additional suspension is pumped into bead separation bag 570 through port 569. any air within bead separation bag 570 flows out through port 568. Once all of the air is removed, rocker drive 234 then tilts platform 290/support plate 380 horizontally. The remainder of the suspension is then be pumped through bead separation bag 570 with the debeaded fluid flowing into gene editing system 20 or some other container.

Step 3: Flow media to output. Pinch valves 128C, 128F, 128G and 128H can be opened. Pump 124A can be used to pump media from media bag 216E, through bead separation bag 570 and to gene editing system 20 or some other container or system where the cells have been collected. This process helps to flush out any remaining cells within bead separation bag 570 and/or the tubing.

Step 4: Transfer beads 179 to bead waste bag 572. Lift assembly 292 lowers magnet assembly 294 to the lowered deactivation position. Pinch valves 128E, 128G and 128B are opened and pump 124B is used to transfer media from media bag 216D, though pump 124B, along tubing section 204L and into bead separation bag 570 through port 568. The media then flows within compartment 415 of bead separation bag 570 around second end 582 of partition 578 and then out of compartment 415 through port 569. As the media is passing through compartment 415, beads 179 are no longer secured to platform 290 under the magnetic force. As such, beads 179 are carried away with the flowing media out through port 569. The media and beads 179 leaving bead separation bag 570 continue to flow through tubing section 204N and into bead waste bag 572. In one method of operation, rocker drive 234 can be activated so that platform 290/platform assembly 232 and bead separation bag 570 are continuously rocking as the media is pump through bead separation bag 570, thereby helping to detach and remove beads 179 from bead separation bag 570. Furthermore, once the media is finished pumping through bead separation bag 570, filtered air from air filter assembly 218A can be pumped through bead separation bag 570 to further help remove beads 179.

As an additional optional step, in one embodiment a collection container is coupled to connectors 203D and 203E where the debeaded cells are collected. Once above Step 4 is completed, pinch valves 128I, 128D and 128A can be opened and pump 124B can be used to pump the cells from the collection container back to the original container coupled to connector 203C. The above process Steps 1-4 can then be repeated to remove any residual beads 179 mixed with the cells.

Embodiments of the present disclosure have a number of unique advantages over conventional systems. By way of example, in contrast to conventional systems where magnetic beads and/or cells must be processed or transferred through multiple different apparatus to achieve cell activation and isolation, one embodiment of the present disclosure achieves isolation and activation of T-cells within a single apparatus. Further, some conventional systems require repeated manual manipulation of the beads and/or cells to achieve cell isolation and activation. In contrast, one embodiment of the present disclosure achieves all necessary steps for activation and isolation without any manual manipulation or input other than loading and starting the system.

One embodiment of the present disclosure also uses a single bead processing apparatus with multiple different disposable consumable kits to achieve different functions, *e.g.*, activation and isolation of T-cells versus separation of T-cells from magnetic beads. Such a system minimizes cost and storage requirements by having multiple different uses for the same apparatus. Furthermore, the single use consumable kits which can be discarded eliminates the need for cleaning or sterilizing and avoids risk of cross contamination.

Furthermore, one embodiment of the disclosure provides a simple and elegant solution of combining the disposable consumable kit with the processing apparatus by designing the consumable kit to simply and easily nest on a front panel of the apparatus while needed components of the apparatus pass through the consumable kit. The assembly is simple and intuitive, thereby limiting errors in assembly and improving efficiency in use. Furthermore, the components passing through the consumable kit, *e.g.,* pinch valves, pumps, and sensors, are easy accessed and inspected and can be easily coupled with the line set of the consumable kit.

The present disclosure provides a unique solution to a common problem of magnetic beads clogging when attempting to dispense from a vial, especially when the vial has set for an extended period. That is, automatically or selectively rotating the vial using the disclosed vial retaining system just prior to dispensing, resuspends the magnetic beads and helps to eliminate clogging.

The automated lift assembly of the present disclosure is also unique and advantages in that it enables easy activation and deactivation of the magnet for optimal processing of the magnet beads without the need for manual manipulation or transferring the beads to different apparatus.

The disclosed restraining apparatus used with the cover assembly is also a significant improvement over known art in that it passively restrains bulging of the processing bags which can hamper or produce irregular bead separation. That is, the springs help to keep the bag in a certain maximum height so as to ensure in some applications that the beads passing through the bag are subjected to a sufficient magnetic field produced by the magnet. Furthermore, use of springs in the system is beneficial in some embodiments in that the restraining force increases as the expansion of the bag increases. In addition, the resilient nature of the springs assists in applying a force to the bag that helps with dispensing fluid from the bag. Further, the springs may be used to keep the bag at a specified maximum height to ensure that all of the fluid in the bag is exposed to sufficient magnetic force to attract the magnetic beads therein.

The ability to selectively activate the stop is also particularly useful in some embodiments. Depending on the situation and the processing conditions, use of the stop can provide significant versatility to the system by enabling an operator to quickly and easily select between limiting bag expansion to a predefined thickness so as to optimize the application of the magnetic force to the beads or permitting free expansion of the bag so as to optimize the amount of fluid that can processed.

The clamp assembly of the present disclosure is also unique in that it is free floating to account for bag and port movement through expansion and contraction of the bag, provides a simply mechanism for centering the bag on the platform for optimal application of the magnetic force, and can easily be switched out for use with other clamp assemblies designed for use with different bags.

Numerous other advantages are also found within the disclosed systems and parts and uses thereof.

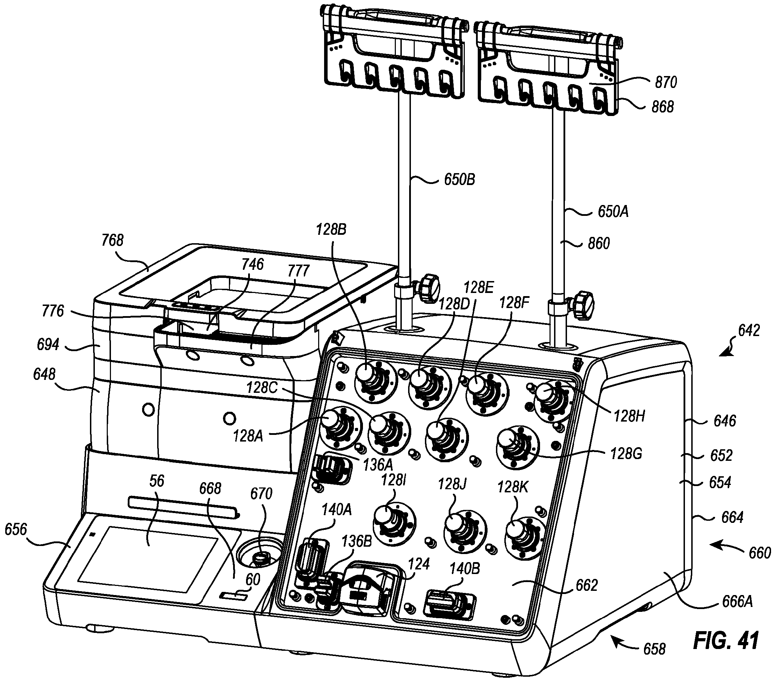

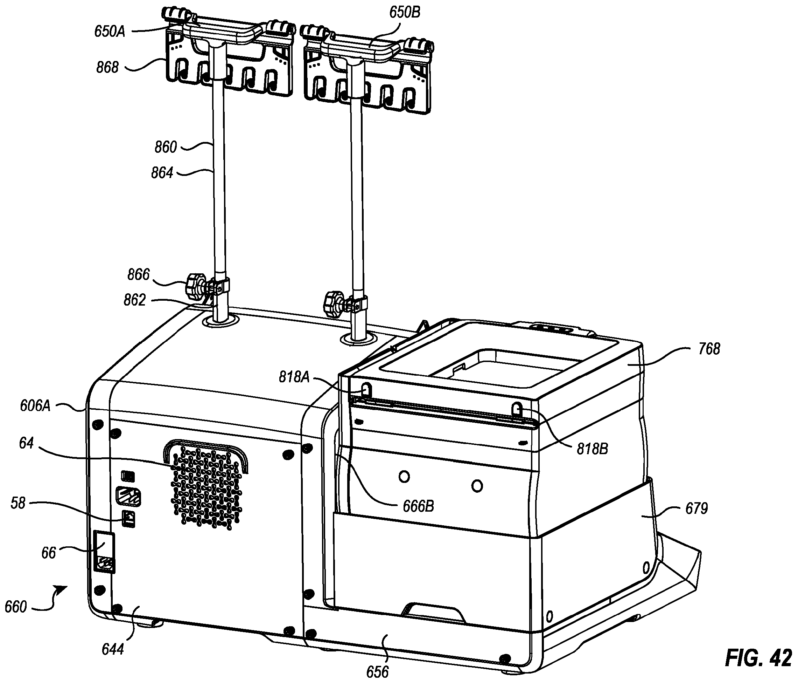

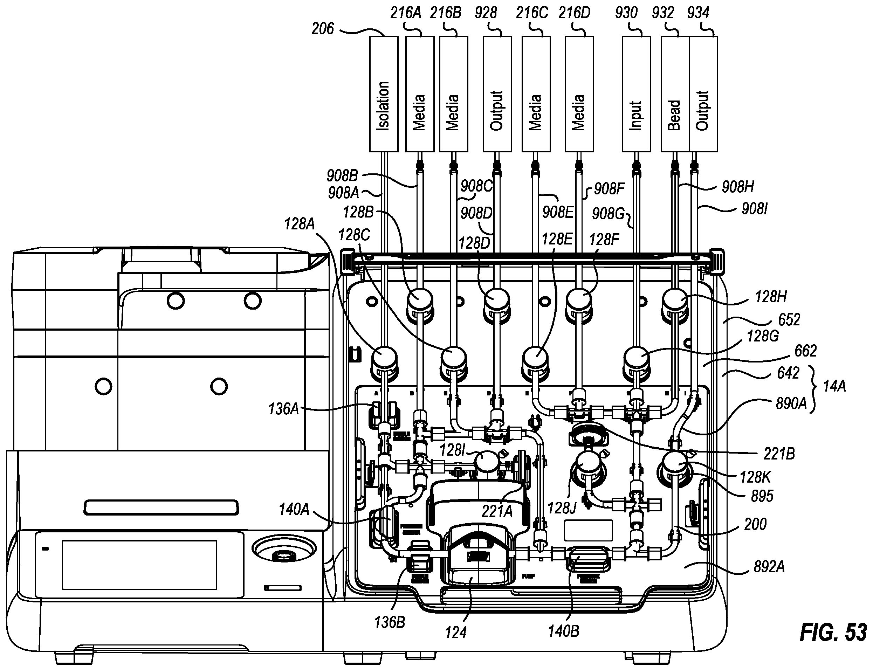

Depicted in Figure 53 is an alternative exemplary embodiment of a bead processing system 14A that can be used as an alternative to bead processing system 14 (Figure 1). Like elements between bead processing systems 14A and 14 are identified by like reference characters. Bead processing system 14A comprises a reusable bead processing apparatus 642 and a single use, disposable consumable kit 890A that is used therewith. Turning to Figures 41 and 42, bead processing apparatus 642 is an alternative exemplary embodiment of previously discussed bead processing apparatus 22 (Figures 2 and 3). Like elements between bead processing apparatus 642 and bead processing apparatus 22 are also identified by like reference characters. In addition, like elements of bead processing apparatus 22 used in bead processing apparatus 642 can include all of the components, alternatives, and functional uses as previously discussed with regard to bead processing apparatus 22. In general, bead processing apparatus 642 includes a base assembly 646, a rocker assembly 648 mounted on base assembly 646 and configured to selectively rock relative thereto, and a pair of bag stands 650A and 650B upstanding from base assembly 646.

Base assembly 646 comprises a housing 652 that bounds a compartment. The same electrical components and other hardware, including electrical circuitry 53 (Figure 4), disposed within housing 38 of bead processing apparatus 22 can be disposed within the compartment of housing 652. Housing 652 generally comprises a main housing 654 and a stage 656 outwardly projecting therefrom. More specifically, main housing 654 extends between a front end 658 and an opposing rear end 660. Disposed at front end 658 is a support panel 662. Support panel 662 can have the same components and be disposed at the same angles as support panel 92 (Figure 4). Disposed at rear end 660 is a back panel 664 on which power inlet 66, vent openings 64 and electrical interface port 58, as previously discussed with regard to bead processing apparatus 22, can be disposed. Opposing side panels 666A and 666B extend between support panel 662 and back panel 664.

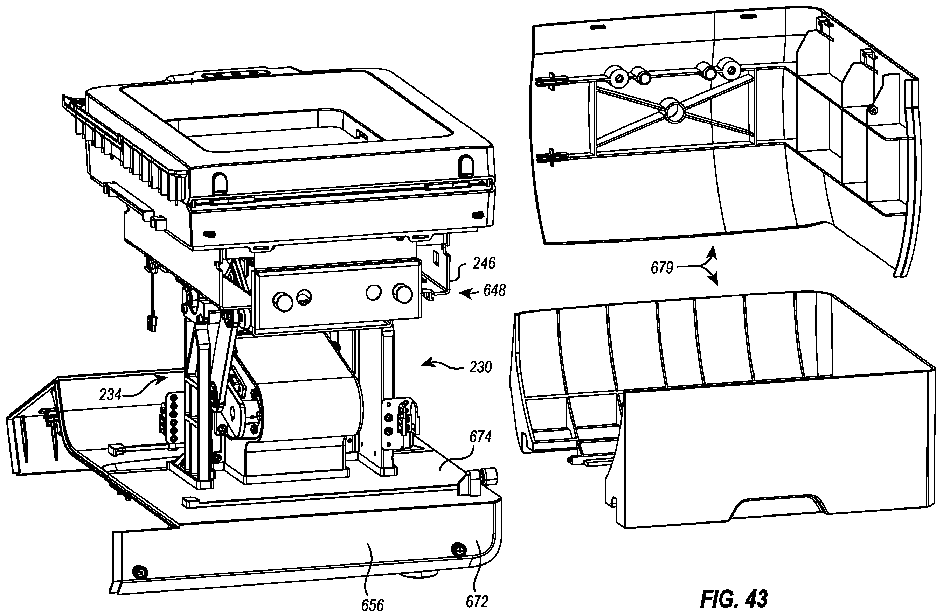

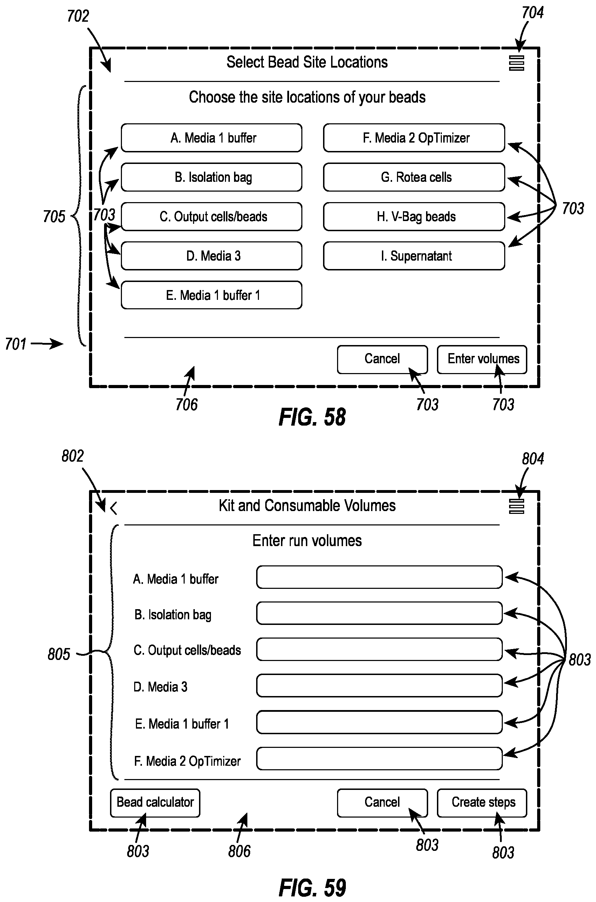

Stage 656 outwardly projects from side panel 666B. Stage 656 includes a front face 668 on which user interface 56 and activation switch 60 are disposed. Screen 56 and activation switch 60 can have the same designs, alternatives, and functions as previously discussed with bead processing apparatus 22. In general, user interface 56 (with display and graphical user interface described with respect to Figures 58-62) can be used in controlling operation of bead processing apparatus 642 and/or displaying operational features and bioprocess parameters thereof. An emergency shut-off switch 670 can also be disposed on front face 668 or be otherwise disposed on housing 652. As better depicted in Figure 43, stage 656 also include a back panel 672 with a shelf panel 674 extending therebetween. Stage 656/shelf panel 674 outwardly projects from side panel 666B and is typically horizontally disposed. As discussed below, rocker assembly 648 upstands from or otherwise upwardly projects above shelf panel 674/stage 656. Portions of rocker assembly 648 may extend into stage 656 below shelf panel 674.

It is appreciated that bead processing apparatus 22 can similarly be described in terms of having a main housing with a stage projecting therefrom. Specifically, with reference to Figures 2-4, bead processing apparatus 22 can include a main housing 39 that includes and extends between support panel 92 and riser panel 72 (which can also be referred to herein as a back panel) and a stage 41 that includes shelf panel 68 and the reminder of housing 38 that projects back from riser/back panel 72. Again, rocker assembly 34 upstands from or otherwise upwardly projects above shelf panel 68/stage 41. Accordingly, depending on the application, the bead processing apparatus can be formed with a stage projecting rear of the main housing or to the side of the main housing. In some embodiments, having the stage projecting from the side of the main housing can produce benefits. For example, having the stage/rocker assembly laterally to the side of the main housing provides easier access to operating the rocker assembly, including loading and unloading the bags therefrom. In addition, having the stage laterally to the side of the main housing enables screen 56 to be mounted on the front thereof which enables the support panel to be enlarged without increasing the overall size of the system. Increasing the size of the support panel can simplify the attachment, removal and operation of the disposable kit thereon, as discussed further below.

As depicted in Figure 43, rocker assembly 648 comprises mount assembly 230 that is disposed on stage 656, a platform assembly 678 that is movably coupled to mount assembly 230 and rocker drive 234 that is used to selectively rock platform assembly 678 relative to mount assembly 230. Mount assembly 230 and rocker drive 234 can have the same components, alternatives, and operational functions as previously discussed with regard to bead processing apparatus 22. An outer housing 679 can be used to encircle mount assembly 230, rocker drive 234, and at least part of platform assembly 678 to help protect against accidental injury.

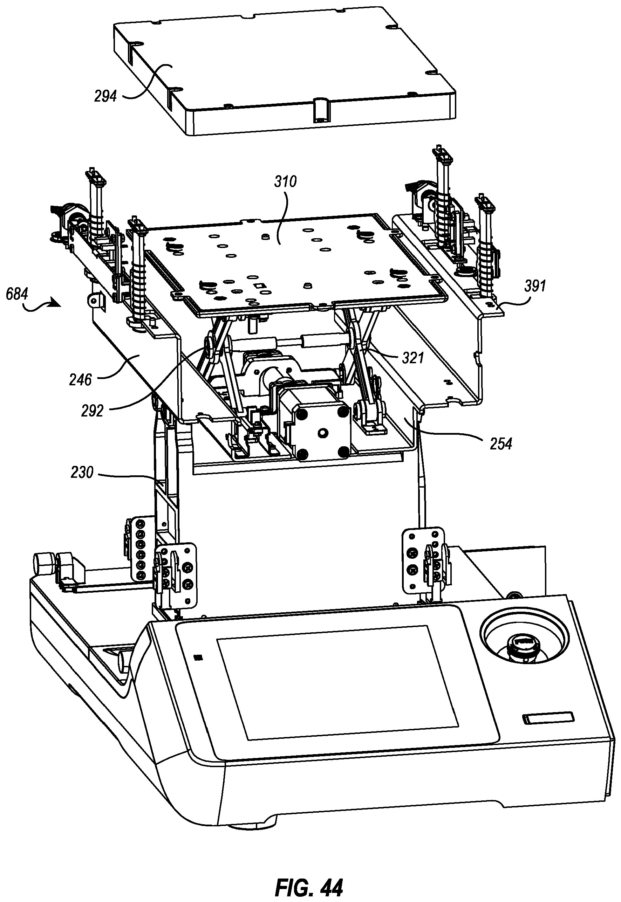

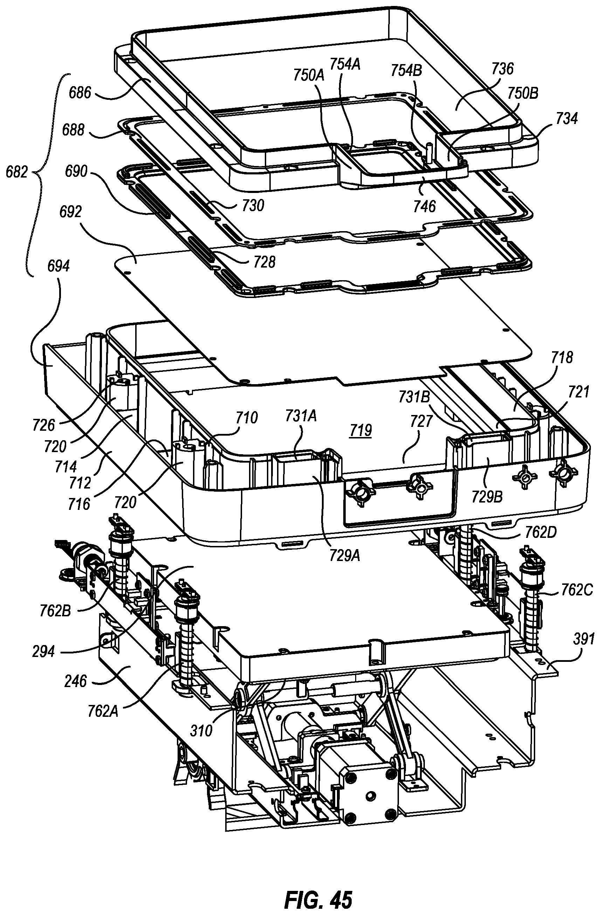

With reference to Figures 43 and 44, platform assembly 678 includes, in part, housing assembly 246, lift assembly 292 and magnet assembly 294, as previously discussed with regard to rocker assembly 34. Again, housing assembly 246, lift assembly 292 and magnet assembly 294 can have the same components, alternatives, and operational functions as previously discussed with rocker assembly 34. As such housing assembly 246 is pivotably/rotatably mounted to mount assembly 230, as previously discussed, and bounds compartment 254. Likewise, lift assembly 292 of rocker assembly 648 includes shelf 310, scissor lift 321 that selectively raises and lowers shelf 310 relative to mount assembly 230, and the other components of lift assembly 292 as previously discussed. As shown, scissor lift 321 is at least partially disposed within compartment 254 of housing assembly 246. Magnet assembly 294 is mounted on shelf 310 (Figure 45) and thus can be selectively raised and lowered relative to mount assembly 230 by lift assembly 292. Specifically, as previously discussed, magnet assembly 294 can be moved between a raised activation position and a lowered deactivation position.

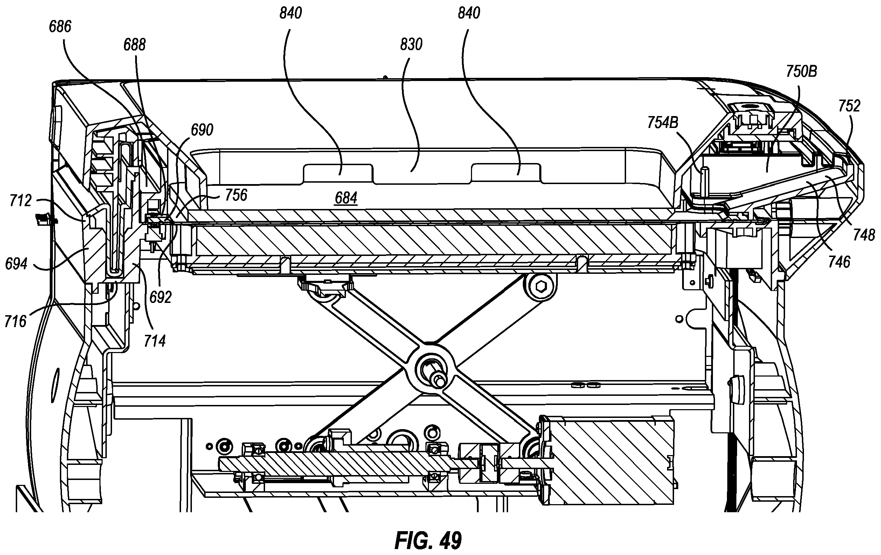

Turning to Figure 45, platform assembly 678 (Figure 43) further includes a platform 682 on which isolation bag 206 (Figure 10) can be removably supported and which is secured to housing assembly 246. In the illustrated exemplary embodiment, platform 682 includes a restraint 686, a contact 688, an insulation seal 690, a support plate 692, and a retention frame 694. Retention frame 694 comprises an inner wall 710 that can form a continuous loop and an outer wall 712 that encircles inner wall 710 so that a slot 714 is formed therebetween. A floor 716 (Figure 49) extends between the lower ends of outer wall 712 and inner wall 710 so as to connect them together. Inner wall 710 has an interior surface 718 that encircles an opening 719 passing through retention frame 694. A flange 721 inwardly projects from interior surface 718 into opening 719.

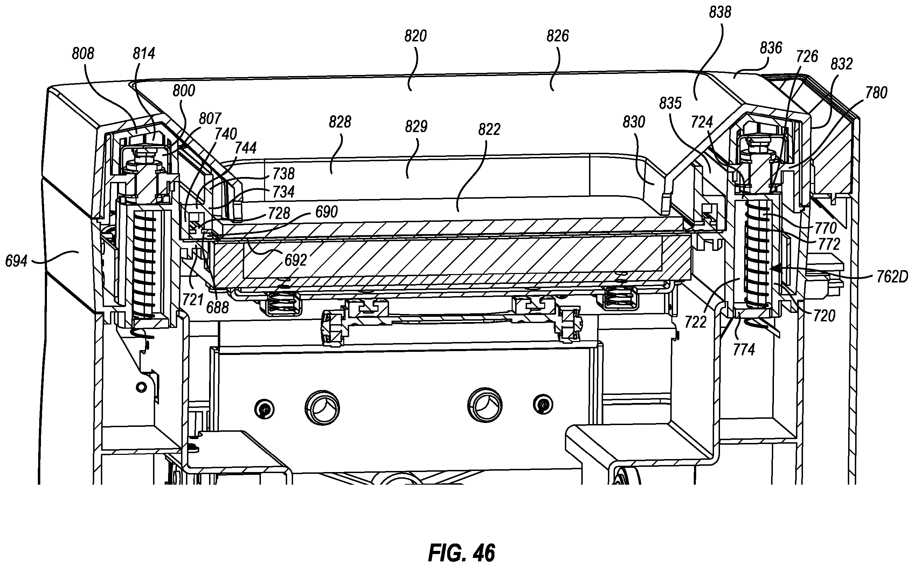

As will be discussed below in further detail, spaced apart tubular sleeves 720 are disposed and secured within slot 714. As depicted in Figure 46, each tubular sleeve 720 bounds a passage 722 that is open at a lower end and is capped by an end wall 724 at an opposing upper end. A constricted opening 726 extends through end wall 724 so as to communicate with passage 722. In the depicted embodiment, four tubular sleeves 720 are formed, two on each opposing side of retention frame 694. In other embodiments, other numbers of sleeves 720 can be formed. Retention frame 694 also includes a passage 727 passing through the upper end of inner wall 710 at a front end of retention frame 694. Guides 729A and 729B are formed within slot 714 adjacent to but on opposing sides of passage 727. Each guide 729A and 729B encircles a downwardly extending channel 731A and 731B, respectively.

During assembly, retention frame 694 is secured to housing assembly 246 so that opening 719 aligns with magnet assembly 294. For example, floor 716 or other portions of retention frame 694 can be secured to flange 391 of housing assembly 246.

Support plate 692 is secured within opening 719 of retention frame 694 and is used to directly support isolation bag 206 (Figure 29) or bead separation bag 570 (Figure 34) depending on the intended use. For example, support plate 692 can be positioned on flange 721 of retention frame 694 so as to be disposed within opening 719. Conventional fastening techniques, such as welding, fasteners or adhesive, can be used to secure support plate 692 to flange 721. In one exemplary embodiment, support plate 692 is typically made of an electrically conductive material, such as an electrically conductive metal. However, support plate 692 must permit the magnetic field produced by magnet assembly 294 to pass therethrough. Support plate 692 can have all or some of the properties, dimensions, and/or alternatives of support plate 380, previously discussed.

With reference to Figures 45 and 46, insulation seal 690 is in the form of a continuous loop and is positioned on top of an outer perimeter edge of support plate 692. Insulation seal 690 is typically made from a non-electrically conductive material that can form a liquid tight seal against support plate 692. For example, insulation seal 690 can be formed from an elastomeric material, rubber, or other materials having similar properties. If needed, an adhesive or sealant can be placed between insulation seal 690 and insulation seal 690 to form a liquid tight seal therebetween. In one embodiment, one or more retention rib 728 can upwardly project from a top surface of insulation seal 690.

Contact 688 is positioned on top of insulation seal 690 so as to be spaced apart from and, more specifically, elevated above support plate 692. Contact 688 also forms a continuous loop and can have a configuration similar to insulation seal 690. For example, contact 688 will typically have a width the same as or small than a width of insulation seal 690 so that when positioned, contact 688 does not project out beyond insulation seal 690. Contact 688 is also made from an electrically conductive material, such as the same types of materials of which support plate 692 can be made. A liquid tight seal can also be formed between insulation seal 690 and contact 688. The seal between insulation seal 690 and contact 688 can be a result of the material properties of insulation seal 690 and/or an adhesive or sealant placed therebetween.

One or more slots 730 can be formed extending through contact 688. Slots 730 can be configured so that when contact 688 is positioned on top of insulation seal 690, ribs 728 pass through or can be pressed through slots 730 so as to secure contact 688 on top of insulation seal 690, thereby preventing or limiting lateral movement of contact 688 relative to insulation seal 690. Figure 46 shows the assembly of support plate 692 supported on flange 721 and insulation seal 690 and contact 688 disposed on the outer perimeter of support plate 692.

As also depicted in Figures 45 and 46, restraint 686 is disposed over insulation seal 690 and contact 688. Restraint 686 comprises a boundary wall 734 that typically has an "h" shaped configuration and that partially encircles an opening 736. More specifically, boundary wall 734 has an inner leg 738, an outwardly spaced outer leg 740, and a seat 744 that extends from the top of outer leg 740 to a central portion of inner leg 738. Outer leg 740 projects down lower than inner leg 738. Restraint 686 also includes a 746 extending between opposing ends of boundary wall 734 that outwardly projects away from opening 736. As depicted in Figure 49, tray 746 comprises an upwardly sloping floor 748 that projects outward and away from opening 736. Floor 748 extends laterally between upstanding sidewalls 750A and 750B connected to boundary wall 734. A lip 752 upstands from a terminal end of floor 748 and extends between sidewalls 750A and 750B. A pair of spaced apart posts 754A and 754B upwardly project from an inner end of floor 748, adjacent to but spaced apart from sidewalls 750A and 750B, respectively. The function of tray 746 will be discussed later.

Restraint 686 functions in part to properly position and secure in position insulation seal 690 and contact 688. As shown in Figure 46, outer leg 740 sits directly on support plate 692 with insulation seal 690 and/or contact 688 butted against or adjacent to the inner surface of outer leg 740. The engagement between restraint 686/outer leg 740 can form a liquid tight seal. Outer leg 740 functions to restrain outward movement of insulation seal 690 and contact 688. Inner leg 738 sits on top of contact 688 inside of rib 728 that has passed through contact 688. Rib 728 is butted against or is adjacent to inner leg 738 so that insulation seal 690 and/or contact 688 are restrained from inward movement. Restraint 686 is also positioned so that tray 746 projects through passage 727 of retention frame 694. As a result, guides 729A and 729B are disposed on opposing sides of tray 746.

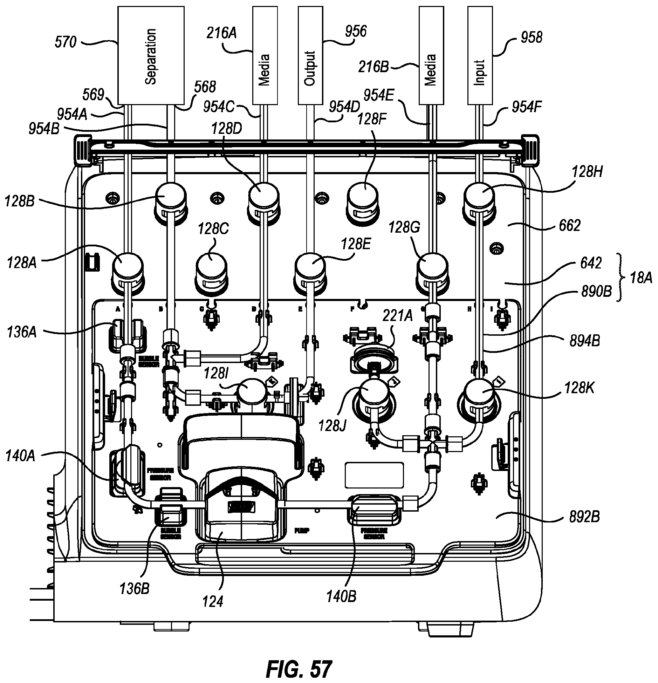

As depicted in Figure 57, platform 682 bounds a cavity 696 that is configured to receive isolation bag 260 and bead separation bag 570. Specifically, as discussed further below, cavity 696 is bounded at least in part by support plate 692 and restraint 686 encircling and upstanding therefrom.

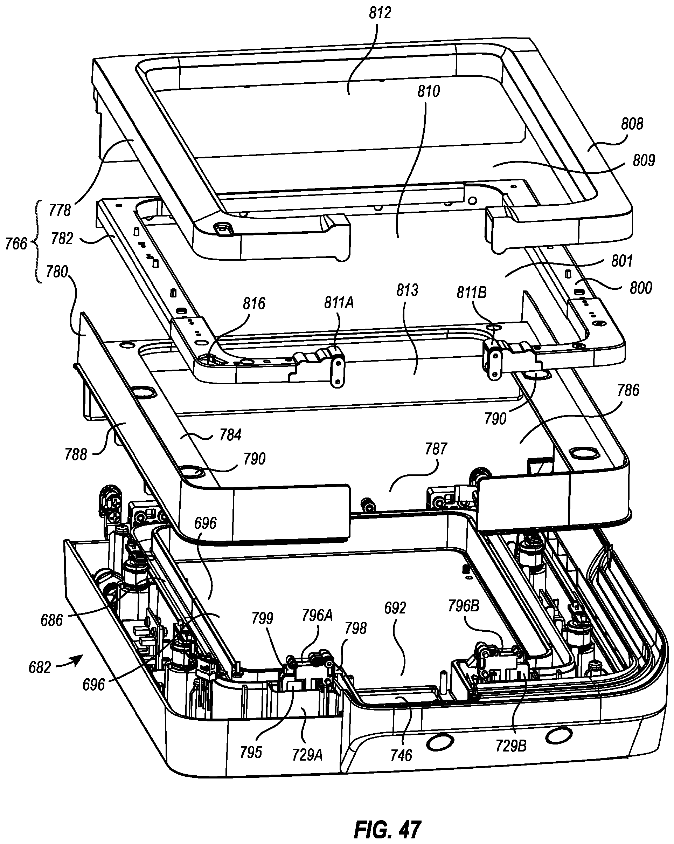

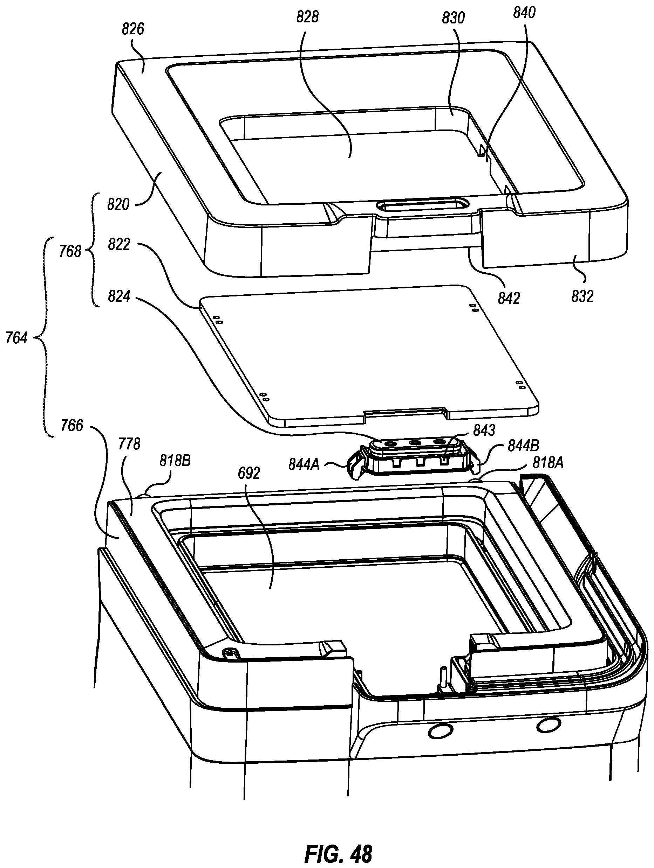

Platform assembly 678 also includes a restraining assembly 760 (shown in Figure 48) that at least partially covers support plate 692 and isolation bag 206/bead separation bag 570 when disposed thereon. Restraining assembly 760 generally comprises four spaced apart spring assemblies 762A - 762D (Figure 45) and a cover assembly 764 (Figure 48) coupled thereto. Cover assembly 764 comprises a cover housing 766 (Figure 47) that is coupled to each of spring assemblies 762 and a lid 768 (Figure 48) that is hingedly mounted to cover housing 766. These various components of restraining assembly 296 will now be discussed in further detail.

With reference to Figure 46, each spring assembly 762 include an elongated rod 770 encircled by a coiled spring 772. A flange 774 outwardly projects from a lower end of rod 770 and secures spring 772 to the lower end of rod 770. During assembly, each spring assemblies 762 is received within a corresponding tubular sleeve 720 of retention frame 694 so that an upper end of each rod 770 passes through a corresponding constricted opening 726. However, spring 772 has a diameter that is larger than constricted opening 726 and thus spring 772 is capture within passage 722 of sleeve 720 and biases against end wall 724. In one embodiment, flange 774 is sized so that it can freely slide within passage 722 as rod 770 is slid within passage 722 but cannot pass through constricted opening 726. Thus, when rod 770 is pulled up through constricted opening 726, spring 772 is compressed against end wall 724, thereby producing a resilient urging force against rod 770.

Turning to Figure 47, cover housing 766 includes an upper cover 778, a lower cover 780, and a middle cover 782 disposed therebetween. Lower cover 780 includes a seat 784 that partially encircles a central opening 786 and is typically horizontally disposed and a side panel 788 vertically projecting from an outside edge of seat 784. Mounting holes 790 extend through seat 784. During assembly, as depicted in Figure 46, lower cover 780 is positioned over retention frame 694 so that the upper end of each rod 700 passes through a corresponding mounting hole 790. In this position, support plate 692 is aligned with central opening 786. Fasteners or another attachment mechanism is then used to secure the upper end of rod 700 to lower cover 780. For example, in the depicted embodiment, C-clips 792A and B are secured to rod 700 at locations above and below seat 784 so as to preclude or at least limit movement of rods 700 relative to lower cover 780. A slot 787 extends through seat 784 and a side panel 788 at a front end lower cover 780. Tray 746 is received within slot 787 when lower cover 780 is positioned and secured over retention frame 694. In the assembled configuration, lower cover 780 (and the other components connected thereto) can be manually raised relative to retention frame 694 by rods 700 sliding up through constricted openings 726. However, as rods 700 are raised, spring 772 are compressed, as discussed above, which produces a resilient urging force wanting to move lower cover 780 back toward retention frame 694.

As also shown in Figure 47, electronic latches 796A and 796B are seated within channels 731A and 731B of guides 729A and 729B, respectively. Each electronic latch 796 has an elongated lower end 795 that can freely side within channel 731 and an enlarged upper end 799 having a dimension larger than channel 731 so that upper end 799 rests on top of guide 729. A latch element 798 is disposed at upper end 799 that can be electronically opened and closed for selectively locking and releasing lid 768, as will be discussed below. In view of the foregoing, electronic latches 796 can freely slid up and down within channels 731 while being guided by guides 729. However, latches 796 are stopped from entirely passing down through channels 731. Electronic latches 796A and 796B are coupled with the electrical circuitry 53 within housing 652 and can be programed to operate as desired.

Middle cover 782 includes a rail 800 that partially encircles an opening 801. Rail 800 has a U-shaped transverse cross section that bounds a channel 807 formed on a bottom thereof (Figure 46). Disposed at the opposing ends of rail 800 are latch covers 811A and 811B. A slot 813 is formed between latch covers 811A and 811B and is configured to receive tray 746. Positioned on rail 800 is a proximity sensor 816 that is electrically coupled with the electrical circuitry 53 within housing 652 and is used to determine whether lid 768 is in an open or closed position. During assembly, middle cover 782 is positioned on top of seat 784 of lower cover 780 so as to cover mounting holes 790. In this position, the upper end of rods 770 are disposed within channel 807. Middle cover 782 can be secured to lower cover 780 using conventional fastening techniques. Latch covers 811A and 811B cover portions of electronic latches 796 while tray 746 is disposed within slot 813 therebetween.

With continued reference to Figures 46 and 47, upper cover 778 includes a cap rail 808 that partially encircles an opening 809. A slot 810 extend through cap rail 808 at a front end of upper cover 778 while a back panel 812 projects down from cap rail 808 at a rear of upper cover 778. Cap rail 808 also has a substantially U-shaped transverse cross section that bounds a channel 814 formed on bottom thereof. In the assembly depicted in Figure 46, cap rail 808 sits on top of seat 784 of lower cover 780 so as to cover middle cover 782. Upper cover 778 is secured to lower cover 780 so that upper cover 778, middle cover 782, and lower cover 780 are joined together and can vertically move up and down in unison through the movement of rods 770, as previously discussed.

Turning to Figure 48, lid 768 is hingedly mounted to upper cover 778 by hinges 818A and 818B. As such, lid 768 can be pivoted between and open position wherein support plate 692 is exposed and a closed position wherein support plate 692 is covered by lid 768 (Figure 41). Lid 768 includes a lid body 820, a lid plate 822, and a catch 824. Lid plate 822 comprises a flat panel typically having a rectangular shape and being separate and discrete from lid body 820. In one embodiment, lid plate 822 is made from a translucent material such as glass or a translucent polymer. In other embodiments, lid plate 822 need not be translucent and can be integrally formed as a unitary element with lid body 820.

Lid body 820 comprises a perimeter wall 826 formed in a continuous loop that encircles an opening 828 passing therethrough. As depicted in Figure 46, in one exemplary embodiment perimeter wall 826 has a substantially U-shaped transverse cross section that includes an inner wall 830 that encircles opening 828, a spaced apart outer wall 832 that encircles inner wall 830, and a transition wall 834 extending therebetween. Perimeter wall 826 bounds a recess channel 835 that receives and/or covers cover housing 766 and restraint 686. Although not required, transition wall 834 can comprise an annular top wall 836 inwardly projecting from outer wall 832 and an annular retaining wall 838 that downwardly slopes from top wall 836 to inner wall 830. Lid plate 822 is centrally mounted to lid body 820 so as to cover opening 828. More specifically, in one embodiment lid plate 822 is secured to a bottom end of inner wall 830, such as by screws or other fasteners or fastening techniques, so as to cover opening 828. The assembled lid 768 forms an upper cavity 829 which is bounded by lid plate 822 and perimeter wall 826 upstanding therefrom. As shown in Figure 49, a plurality of spaced apart channels 840 pass through inner wall 830 along a bottom edge of inner wall 830, *i.e.,* directly against lid plate 822, so as to communicate with upper cavity 829.

Returning to Figure 48, a slot 842 extends through a lower end of outer wall 832 at a front end of lid body 820. Catch 824 is secured to perimeter wall 826 within slot 842 at a front end of lid body 820. Catch 824 comprises a catch body 843 having catch elements 844A and 844B disposed at opposing ends thereof. Catch elements 844A and 844B are configured to engage latch elements 798 of latches 796A and 796B, respectively, to facilitate selective locking of lid 768 when in the closed position.

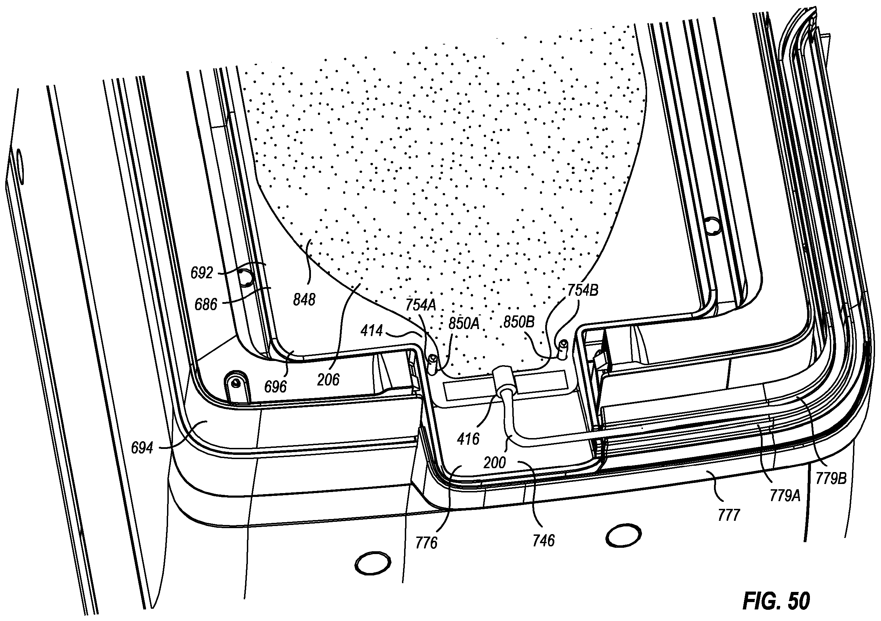

When lid 768 is in the closed position, as shown in Figure 1, an outlet 776 is formed passing between lid 768 and tray 746 that communicates with cavity 696. Secured to the outside of retention frame 694 is an elongated track 777. As better depicted in Figure 50, a pair of grooved channels 779A and 779B extend along the length thereof. One end of track 777 is aligned with and intersects with tray 746 while the opposing end is disposed toward main housing 654.

Rocker assembly 648 operates in substantially the same way as previously described rocker assembly 34. For example, initially lid 768 is moved to the open position and isolation bag 206, as depicted in Figure 50, is positioned within cavity 696 so as to rest directly on support plate 692 and be encircled by restraint 686. Depending on the intended use, isolation bag 206 can be replaced with bead separation bag 570. In this embodiment, however, isolation bag 206 has holes 850A and 850B passing through bag body 411 at perimeter seal 414 or outside of perimeter seal 414 on opposing sides of port 416. Posts 754A and 754B pass through holes 850A and 850B, respectively, so as to properly position and securely retain isolation bag 206 on support plate 692. Tubing 200 extending from port 416 of bag 848 is passed out of cavity 969 through outlet 767 while being supported on tray 746. Tubing 200 is then received within and extends along one of grooved channels 779A and 779B of track 777 toward main housing 654.

Once isolation bag 206 is properly positioned, lid 768 is manually moved to the closed position so that isolation bag 206 is positioned between lid 768 and support plate 692 and, more specifically, between lid plate 822 of lid 768 and support plate 692. Rocker assembly 648 can be configured so that lid 768 automatically locks when moved to the closed positioned or can require manual activation to lock. In the exemplary embodiment, the locking is achieved by latches 796 engaging with catch 824 and, more specifically, latch elements 798 engaging with catch elements 844. In alternative embodiments, a single latch element 798 and catch element 844 can be used or alternative locking structures can be used.

Once lid 768 is in the locked position, a liquid can be delivered into isolation bag 206 through tubing 200. As isolation bag 206 inflates with liquid, cover assembly 764 and track 777 are raised relative to support plate 692. Specifically, with lid 768 in the closed position, isolation bag 206 can be pressed between or be disposed directly adjacent to support plate 692 and lid plate 822. Accordingly, as isolation bag 206 inflates with liquid, isolation bag 206 outwardly pushes against lid 768/lid plate 822 causing all of cover assembly 764/track 777 to rise relative to support plate 692 by rods 770 moving upward. However, as previously discussed, as rods 770 move upward, springs 772 are compressed which produces a resilient downward force by lid 768/ lid plate 822 onto isolation bag 206. This downward force helps to both secure isolation bag 206 to limit movement and flatten isolation bag 206, *i.e.,* limit bulging in the middle, so that it has a more uniform thickness. As previously discussed with regard to rocker assembly 34, this flattening of isolation bag 206 can be helpful in the application of the magnetic field to the liquid within isolation bag 206.

In one exemplary embodiment of rocker assembly 648, restraining assembly 760 can also include stop assemblies 526A-D of rocker assembly 34 (Figures 22-25) that operate with each spring assembly 762A-D, respectively. Specifically, in the same manner as previously discussed with rocker assembly 34, stop assemblies 526A-D can be mounted on retention frame 694 adjacent to tubular sleeves 720 to selectively engage with flanges 774 of spring assemblies 762 (Figure 46) by selectively projecting through tubular sleeves 720. As with rocker assembly 34, stop assemblies 526 can be electronically controlled to either selectively engage or not engage flanges 774 of spring assemblies 762. The engagement of stop assemblies 526 with flanges 774 would limit upward movement of rods 770 and cover assembly 764 and thereby limit expansion of isolation bag 206/bead separation bag 570. Alternatively, when stop assemblies 526 do not engage flanges 774, rods 770 and cover assembly 764 are free to move upward unrestrained except for limits provided by springs 772.

Rocker assembly 648 differs in part from rocker assembly 34 in that clamp assembly 402 (Figure 28) has been eliminated. Specifically, in one embodiment, tray 746 with intersecting track 777 maintain sufficient support and aligned of port 416 of isolation bag 206 and tubbing 200 extending therefrom to eliminate the need of clamp assembly 402, thereby simplifying the design and operation of the rocker assembly.

In the same manner as previously discussed with regard to rocker assembly 34, rocker drive 234 of rocker assembly 648 can be selectively or automatically activated to tilt platform assembly 232/ isolation bag 206 and/or to repeatedly rock platform assembly 232/isolation bag 206 relative to mount assembly 230, as needed for the intended use. In addition, in the same manner as previously discussed with regard to rocker assembly 34, lift assembly 292 of rocker assembly 648 can be selectively or automatically activated to raise and lower magnet assembly 294 relative to support plate 692, as needed for the intended use.

In one exemplary embodiment, cover assembly 764 of rocker assembly 648 can automatically detect leaking of liquid from isolation bag 206 or any other bag disposed on support plate 692 and capture a liquid leaking therefrom. For example, with reference to Figure 46, during use isolation bag 206 (Figure 50) is disposed between support plate 692 and lid plate 822. Insulation seal 690 and contact 688 will be disposed adjacent to isolation bag 206. As previously discussed, contact 688 sits on top of insulation seal 690 so as to be electrically insulated from and spaced apart at an elevation higher than support plate 692. Because insulation seal 690 encircles and forms a liquid tight seal with support plate 692, if liquid starts to leak out of isolation bag 206, the liquid is captured within the cavity encircled by insulation seal 690, *i.e.,* cavity 969, and as liquid continues to leak, will begin to rise along the height insulation seal 690. As previously discussed, support plate 692 and contact 688 are each separately electronically connected to the electrical circuitry 53 within housing 652. The liquid leaking from insulation seal 690 will be electrically conductive. As such, when the leaked liquid rises to a level or is otherwise manipulated, such as by rocking of platform assembly 678, so that the liquid connects with contact 688, an electrical short/electrical circuit will be produced that will provide a signal to electrical circuitry 53, thereby signaling that leaking is occurring. In turn, electrical circuitry 53 can be automatically programed to stop all operations or select operations of bead processing apparatus 22 and/or to provide an audible and/or visual indicator that notifies the operator of the leak.

It is appreciated that the leak detection components are optional and can be eliminated. In that case, insulation seal, contact, and/or restraint 686 can be eliminated. Furthermore, where the leak detection components are removed, support plate 692 need not be made from an electrically conductive material but could be made from other materials, such as plastics or composites. As liquid continues to leak from bag 848, it is captured within cavity 969 by being laterally restrained by restraint 686. As a result of lid plate 822 being translucent, the leaked liquid can be visually detected below lid plate 822. Channels 840 are formed through inner wall 830 of lid 768 so that as the liquid continues to rise with cavity 969, the liquid will eventually flow through channels 840 so as to be disposed on top of lid plate 822, *i.e.,* within upper cavity 829. As a result, the leaked fluid can be more easily visually detected. Lid body 820 projecting above lid plate 822 restrains spilling of the leaked liquid outside of upper cavity 829. The sloping of retaining wall 838 provides improved visibility to upper cavity 829 for detecting the leaked liquid while also directing any liquid back toward lid plate 822 that may splash onto retaining wall 838.

Returning to Figures 41 and 42, each bag stand 650 comprises a pole 860 having a lower end connected to housing 652 and an opposing upper end. Although pole 860 can be of a fixed length, in the depicted embodiment, pole 860 is an adjustable telescoping pole that includes a lower pole 862 and an upper pole 864 slidably disposed within lower pole 862. A releasable fastener 866, such as a clamp or the like, is disposed on lower pole 862 and functions to selectively restrain upper pole 864 relative to lower pole 862. A catch 868 is hingedly mounted to the upper end of pole 860. More specifically, as depicted in Figure 48, an arm 876 outwardly projects from the upper end of pole 860. In one exemplary embodiment, arm 876 can radially or orthogonally outwardly project from pole 860. Arm 876 can have a U-shaped body 878 centrally connected to pole 860 with stems 880A and 880B outwardly projecting, *e.g.*, orthogonally projecting, from opposing ends of body 878.

Catch 868 comprises a plate 870 having an upper end 882 and an opposing lower end 884. A pair of spaced apart C-shaped fingers 886A and 886B project from upper end 886 of plate 870. Finger 886A and 886B are configured to releasably snap fit onto stems 880A and 880B. This coupling enables catch 868 to be releasable secured to arm 876 to simplify the attachment and/or removal of bags from catch 868, as discussed below, and also enables catch 868 to pivot on arm 876 to facilitate attachment, removal, or manipulation of bag on catch 868 while catch 868 is retained on arm 876. Lower end 884 of catch 868 terminates at bottom edge 888 having a plurality of spaced apart notches 872 upwardly recessed therein. Plate 870 also includes a plurality of L-shaped fingers 874 with each finger 874 projecting into a corresponding notch 872. During use, bags used in the operation of bead processing apparatus 642, such as waste bags, collection bags, and/or bags containing media, biological product, beads and/or other product, can be adjacently supported on catch 868 by supporting a hanger of the bags on a corresponding finger 874. The hanger my extend from the bags or be formed by a hole extending through a perimeter of the bags. Each plate 870 can have at least 2, 4, 6, 8, 10 or more notches 872 with a corresponding finger 874 therein. Other numbers can also be used.

Returning to Figure 41, as with previously discussed support panel 92 of bead processing apparatus 22, support panel 662 can comprise a base panel and an overly panel that function the same as previously discussed with support panel 92. Furthermore, as with bead processing apparatus 22, a plurality of mechanical components are connected to or otherwise outwardly project from support panel 662. In this exemplary embodiment, the mechanical components include pinch valves 128A-128K, pump 124, bubble sensors 136A and 136B, and pressure sensor 140A and 140B. It is understood that pinch valves 128, pump 124, bubble sensors 136, and pressure sensor 140 of bead processing apparatus 642 can have the same design, function, and alternatives as pinch valves 128, pump 124, bubble sensors 136, and pressure sensor 140 of previously discussed bead processing apparatus 22. However, the layout and number of the mechanical components in bead processing apparatus 624 have been modified relative to those in bead processing apparatus 22 to simplify the design and operation. For example, the mechanical components of bead processing apparatus 624 only require a single pump 124. Furthermore, rotational assemblies 132 have been eliminated and replaced with additional pinch valves 128. Accordingly, the gas flow and liquid flow through the tubing can now be solely controlled by using pinch valves 128 to selectively pinch and release corresponding tubing.

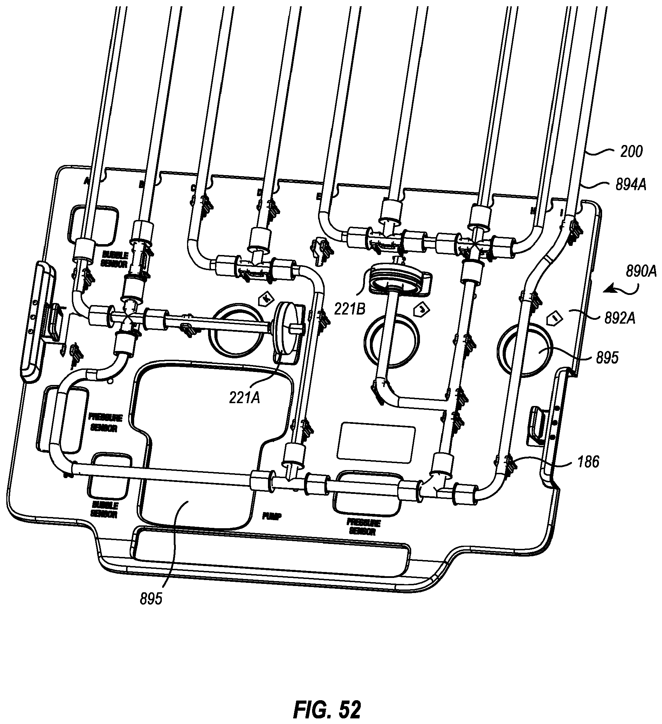

Turning to Figure 52, single use, disposable consumable kit 890A can be used with bead processing apparatus 642 to form bead processing system 14A (Figure 53) for isolating and activating the T-cells suppled thereto. Consumable kit 890A comprises a tray 892A and a line set 894A mounted thereon. Consumable kit 890A is configured to removably nest on top of support panel 662 (Figure 53) so that line set 894A can engage or otherwise interact with the various mechanical components disposed on support panel 662, as discussed above. Except as noted below, tray 892A and line set 894A can have the same elements, the same properties, be made of the same materials, have the same dimensions, and be used in the same ways as previously discussed with tray 172A and line set 174A, respectfully. Tray 892A has a plurality of openings 895 extending between the opposing top and bottom face thereof. However, in contrast to openings 185 of tray 172A, opening 895 are positioned and configured to receive pinch valves 128A- 128K, pump 124, bubble sensors 136A and 136B, and pressure sensor 140A and 140B as positioned on and outwardly projecting from support panel 662.

Tray 892A also includes previously discussed tube restraints 186 mounted on and outwardly projecting from the top face thereof. Tube restraints 186 support and secure the tubing of line set 894A. In contrast to tray 172A, tray 892A does not include bag restraint 192.

As depicted in Figures 52 and 53, line set 894A generally comprises flexible tubing 200 fluid coupled with a plurality of bags and with previously discussed air filters 221A and 221B. Stopcocks 220 and mixing bag 210 of prior line set 174A have been eliminated from line set 894A to simplify production and operation. Flexible tubing 200 is positioned to align with select openings 895 on tray 892A so as to engage with select mechanical components projecting therethrough from support panel 662. Specifically, during use, as depicted in Figure 53, the assembled consumable kit 890A is placed on top of support panel 662 so that the mechanical components on support panel 662 are aligned with corresponding openings 895 formed on tray 892A. Tubing 200 is then manipulated to couple with each of pinch valves 128A - 128K, pump 124, bubble sensors 136A and 136B, and pressure sensor 140A and 140B.

Line set 894A includes tubing sections 908A-908I that extend between tray 892 and corresponding bags. For example, tubing section 908A connects to isolation bag 206, tubing sections 908B, 908C, 908E and 908F connect to media bags 216A-D, respectively, tubing section 908D connects to an output bag 928, tubing section 908G connects to an input bag 930, tubing section 908H connects to a bead bag 932, and tubing section 908I connects to an output bag 934.

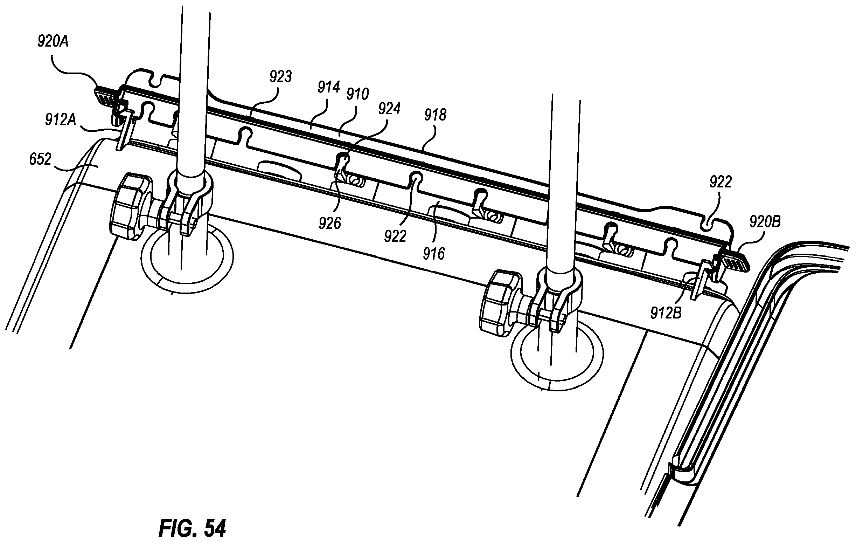

Bead processing apparatus 642 also includes a tubing restraint 910 that is used to releasably secure and organize tubing sections 908A-908I. Tubing restraint 910 is secured to an upper end of housing 652 above support panel 662. More specifically, with reference to Figure 54. A pair of mounts 912A and 912B outwardly project from housing 652 above support panel 662. Tubing restraint 910 releasably connects to mounts 912A and 912B so as to be slightly spaced apart from housing 652. Tubing restraint 910 includes an elongated brace 914 having an inside edge 916 facing toward housing 652 and an opposing outside edge 918. Handles 920A and 920B can be formed at the opposing ends of brace 914. In one embodiment, brace 914 can comprise a flat strip having opposing edges 916 and 918. As needed, a reinforcing flange 923 can outwardly extend from a top face and/or bottom face of brace 914 along the length thereof to reinforce brace 914 so as to help prevent failure or excessive flexing. Notches 922 are recessed into edges 916 and/or 918 of brace 914 so as to extend between the top face and the opposing bottom face of brace 914. Notches 922 are configured to selectively receive and releasably secure tubing sections 908A-908I. In one exemplary embodiment, each notch 922 includes a rounded head 924 that is accessed through a constricted mouth 926.

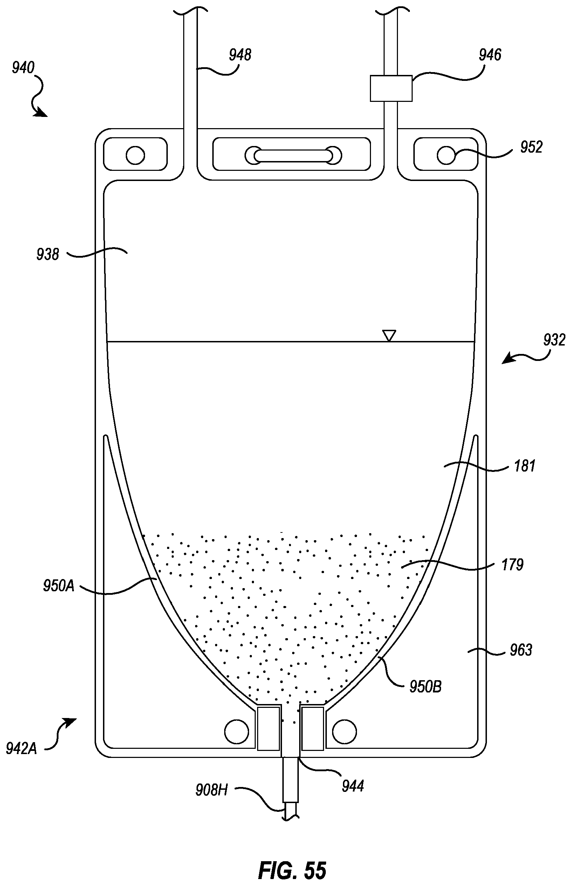

Bead processing apparatus 642 also distinguishes over bead processing apparatus 22 in that bead processing apparatus 642 eliminates bead vial retainer 148 and the associated use of vial 166 and bead vial coupler 208. In contrast to using vial 166 and related hardware, line set 894A incorporates the use of bead bag 932 coupled with tubing section 908H, as referenced above. Turning to Figure 55, in one exemplary embodiment bead bag 932 comprises a flexible bag body 936 comprised of one or more sheets of polymeric film, such as that previously discussed with regard to isolation bag 206. Bag body 936 bounds a compartment 938 that extends between an upper end 940 and an opposing lower end 942. An outlet port 944 is formed at lower end 942 and communicates compartment 938. Outlet port 944 also communicates directly or indirectly with tubing section 908H. One or more inlet ports 946 or inlet tubes 948 is coupled with upper end 940 of bag body 936 and communicate with compartment 938. Disposed within compartment 938 are beads 179 and carrier liquid 181, as previously discussed with vial 166. As with other bags disclosed herein, bag body 936 is typically a pillow type bag comprised of an overlying top sheet and bottom sheet bonded together to form a perimeter seal that bonds compartment 938. In the current embodiment, the perimeter seal includes opposing side edges 428A and 428B that extend from a top edge down to outlet port 944. Opposing sides edges 428A and 428B, or at least the lower portions thereof, inwardly slope toward each other as they extend to outlet port 944. As such, at least the lower end of compartment as a substantially V-shaped configuration the funnels beads 179 to outlet port 944. It is appreciated that opposing sides edges 428A and 428B can be linear or curved as they inwardly slope toward outlet port 944.

Prior to operation of bead processing system 14A, isolation bag 206 (or any other isolation bag disclosed herein) is enclosed within rocker assembly 648 and the remaining bags are typically secured to bag stands 650A and/or 650B, as previously discussed. The operation of assembled bead processing system 14A is then similar to the previously discussed operation of bead processing system 14. For example, primarily with reference to Figure 53, in one embodiment, bead processing system 14A can be operated using the below steps. Each of the following process steps can be performed either automatically through the control of pre-programmed electrical circuitry 53 and/or can be controlled manually through manual inputs to a user interface. Upon activation of bead processing system 14A, all of pinch valves 128 are typically moved to a closed position so as to preclude fluid flow through the tubing section coupled with the pinch valves. In the following method steps where it is discussed that select pinch valves are opened, it is understood that the remaining pinch valves remain closed to control fluid flow through line set 894B. It is appreciated that in some embodiment, some steps can be eliminated, other steps can be added, and the order of steps can be altered.

Step 1: Inject air into isolation bag 206 for partial inflating. This can be accomplished by opening pinch valve 128J and activating pump 124 to pump air from air filter assembly 218B isolation bag 206. Injecting air into isolation bag 206 can substantially improve liquid flow and movement within isolation bag 206 by decreasing contact between the liquid and the surface of isolation bag 206. As such, the injected air, as discussed below, can assist with mixing of the cells and magnetic beads.

Based on the operation of different pinch valves 128, it is appreciated that fluids can travel through a variety of different paths to achieve an intended function. As such, the described process steps set forth herein are only examples and other process steps could be used to achieve the same function.

Step 2: Prime tubing and isolation bag 206 with media. Pinch valves 128A and 128F can be opened while pump 124 is used to pass media from media bag 216D, through pump 124B, and into isolation bag 206. It is appreciated media can be provided from any of media bags 216A, 216B, 216C or 216D. Thus, although media is primarily discussed herein as being drawn from media bag 216D, it is appreciated that it can commonly be drawn from one or more of the other media bags. Commonly, media will be drawn from one media bag until empty or close to empty and then be drawn from another media bag.

Step 3: Suspend beads within bead bag 932. As shown in Figure 55, as bead bag 932 sits stationary, beads 179 will settle toward lower end 942. Beads 179 will more easily flow out of bead bag 932 and flow through line set 894A if beads 179 are suspended within carrier liquid 181. Accordingly, by opening select pinch valves 128, pump 124 can be used to pump air into bead bag 932 from air filter 221A and/or pump media into bead bag 932 from media bag 216A. Pumping the air or media into bead bag 932 functions to suspend beads 179 within carrier liquid 181. In alternative embodiments, beads 179 can be suspended within carrier liquid 181 by mechanical or manual manipulation of bead bag 932.

Step 4: Inject suspended beads 179 from bead bag 932 into isolation bag 206. Pinch valves 128A and 128H can be opened and pump 124 used to pump suspended beads 179 from bead bag 932 to isolation bag 206. In some embodiments, once a quantity of suspended beads 179 has been pumped out of bead bag 932, additional media can be pumped into bead bag 932 to resuspend any beads 179 that may have been retained within bead bag 932. This new suspension can then be pumped into isolation bag 206. The above process of adding media into bead bag 932 can be repeated multiple times to ensure that all of beads 179 are flushed out of bead bag 932 and the related tubing and into isolation bag 206.

Step 5: Pre-wash beads 179 within isolation bag 206. In one embodiment, beads 179 can be prewashed within isolation bag 206 to help remove any unwanted matter from within isolation bag 206. For example, this step can be used to remove any free antibodies in the mix not covalently bound to beads 179. In one exemplary embodiment, pump 124 can be used to pump media from one of media bag 216 into isolation bag 206. Prior to, concurrently with, or after pumping media into isolation bag 206, rocker drive 234 is activated to facilitate repeated rocking of platform assembly 232 having isolation bag 206 mounted thereon. This rocking can help unbind any agglomeration of beads 179 and suspend any unwanted matter. Rocker drive 234 can then be deactivated and lift assembly 292 activated to raise magnet assembly 294 to the raised activation position relative to support plate 692. Beads 179 settle under gravity and are attracted to and held against isolation bag 206/support plate 692 by the magnetic force produced to magnet assembly 294. In one embodiment, rocker drive 234 can rearwardly or negatively tilt platform assembly 232/isolation bag 206 so that port 416 of isolation bag 206 is elevated. A small amount of air or media can then be passed through port 416 to help ensure that no beads 179 are retained therein. The magnetic field can then be applied to isolation bag 206 while in this rearward tilt position. With magnet assembly 294 still in the raised activation position so that beads 179 are secured against isolation bag 206, rocker drive 234 can the tilt platform assembly 232/isolation bag 206 forward/positively so that port 416 is now lowered. This orientation helps to ensure that fluid freely flows out of isolation bag 206 through port 416. Pump 124 is then used to pump the liquid out of isolation bag 206 and into outlet bag 928, or other container, such as by opening pinch valves 128A and 128D. The above pre-washing process of beads 179 can be repeated any desired number of times, such as at last one, two, three or more time.

Step 6: Transfer media into isolation bag 206. Pump 124 is used to pump a defined quantity of media into isolation bag 206 so that beads 179 are diluted to a desired concentration. For example, pinch valves 128A and 128F can be opened and pump 124 used to pump media from media bag 216D into isolation bag 206.

Step 7: Suspend beads 179 within isolation bag 206. Lift assembly 292 is activated to lower magnet assembly 294 to the deactivation position and rocker drive 234 is activated to repeatedly rock platform 290 and isolation bag 206 disposed thereon, thereby homogenously suspending beads 179 within the media.

Step 8: Transfer cell culture to isolation bag 206. Pump 124 is used to pump the cell culture generated from cell separator 12 into isolation bag 206. In the depicted embodiment, the cell culture is disposed within input bag 930. Thus, pinch valves 128A and 128G can be opened and pump 124 used to pump the cell culture from input bag 930 to isolation bag 206. In other embodiments, pump 124 can be used to pump the cell culture directly from cell separator 12 or some other container.

Step 9: Facilitate isolation and activation of desired T-cells. Rocker drive 234 is activated or remains activated from Step 8 to facilitate rocking of platform 290 and isolation bag 206 thereon which mixes beads 179 with the cell culture containing the desired T-cells. Beads 179 having a desired antibody thereon will bind to and activate the desired T-cell as bead 179 comes in contact with the desired T-cell during the mixing process. Such mixing can occur for an extended period of time. By way of example, and not by limitation, the mixing can be between 15 minutes to 60 minutes and more commonly between 20 minutes to 40 minutes. Other time durations can also be used.

Step 10: Capture T-cells bound with beads 179. Lift assembly 292 is activated to raise magnet assembly 294 relative to support plate 692 to the activation position. The magnetic force produced by magnet assembly 294 causes beads 179 and T-cells bound to beads 179 to be held against isolation bag 206/support plate 692 while retained within isolation bag 206. As with the prewashing step, in one exemplary embodiment, rocker drive 234 can first rearwardly tilt platform assembly 232/isolation bag 206 so that port 416 of isolation bag 206 is elevated. A small amount of air or media can then be passed through port 416 to help ensure that no beads 179/cells are retained therein. The magnetic field can then be applied to isolation bag 206 while in this rearward tilt position.

Step 11: Transfer liquid from isolation bag 206 into collection bag 212. Rocker drive 234 can positively tilt platform 290 so that port 416 is downwardly positioned. Pump 124B can then be used to pump fluid from isolation bag 206 into output bag 928 while beads 179 and the cells attached thereto remain securely retained within isolation bag 206 under the magnetic force produced by magnet assembly 294. For example, pinch valves 128A and 128D can be opened and pump 124 used to pump the liquid from isolation bag 206 to output bag 928. This step is to remove the negative cell fraction, *i.e.,* the cells that did not bind to a bead 179, from isolation bag 206.

Step 12: Wash cells bound with beads 179. Pump 124 can be used to pump media from one of media bags 216 into isolation bag 206. Lift assembly 292 is activated to move magnet assembly 294 down to the deactivation position. Consecutively or concurrently, rocker drive 234 is activated to facilitate mixing of beads 179 bound with T-cells in the freshly delivered media. This mixing can again occur for an extended period of time. However, the primary objective of this rocking/mixing is to free any cells or other biological material that may have been unintentionally captured within isolation bag 206 so that it can be removed. Lift assembly 292 is activated to move magnet assembly 294 up to the activation position so as to again capture beads 179 and the T-cells bound thereto. Again, in one embodiment, rocker drive 234 can rearwardly or negatively tilt platform assembly 232/isolation bag 206 so that port 416 of isolation bag 206 is elevated. A small amount of air or media can then be passed through port 416 to help ensure that no beads 179/cells are retained therein. The magnetic field can then be applied to isolation bag 206 while in this rearward tilt position. Rocker drive 234 is then positively tilts platform 290 so that port 416 is downwardly positioned. Pump 124 is then used to pump liquid from isolation bag 206 to collection bag 212. This washing step can be repeated as many times as needed, such as at least one, two, three or more times.

Step 13: Resuspend beads 179 with bound cells. Rocker drive 234 is controlled to disengage tilt and lift assembly 292 is activated to lower magnet assembly 294 to the disengaged position. Pump 124B is used to pump media from a media bag 216 into isolation bag 206. The quantity of media delivered is dependent upon the desired concentration for the T-cells as they are dispensed out of the system. Rocker drive 234 is activated to mix beads 179 with T-cells attached thereto within the freshly delivered media so as to produce homogenous mixture.

Step 14: Transfer suspension within isolation bag 206 to output bag 934 or directly to cell expansion system 16 or other downstream processing equipment or collection container. For example, pinch valves 128A and 128K can be opened and pump 124 used to transfer suspension within isolation bag 206 to output bag 934 or directly to some other downstream equipment or container. In yet another alternative, the suspension could be returned to the input bag 930. The above steps 13 and 14 can be repeated until all of the isolated cells have been removed and/or the cell concentration within the downstream bag/equipment has reached a desired level.

In the above described process, bead processing apparatus 22 uses magnetic beads 179 to isolate and activate desired T-cells. However, in an alternative embodiment, bead processing apparatus 22 can be used with magnetic beads 179 in an opposite process. That is, in contrast to having beads 179 bind to the desired cells, beads 179 can be designed to bind to cells that are not wanted within a mixture of cells. As a result, by using the above process, the unwanted cells can be bound to beads 179 and secured within isolation bag 206 by magnet assembly 294, while the desired cells are washed out of isolation bag 206 and transferred to a collection bag or other downstream apparatus for further processing.

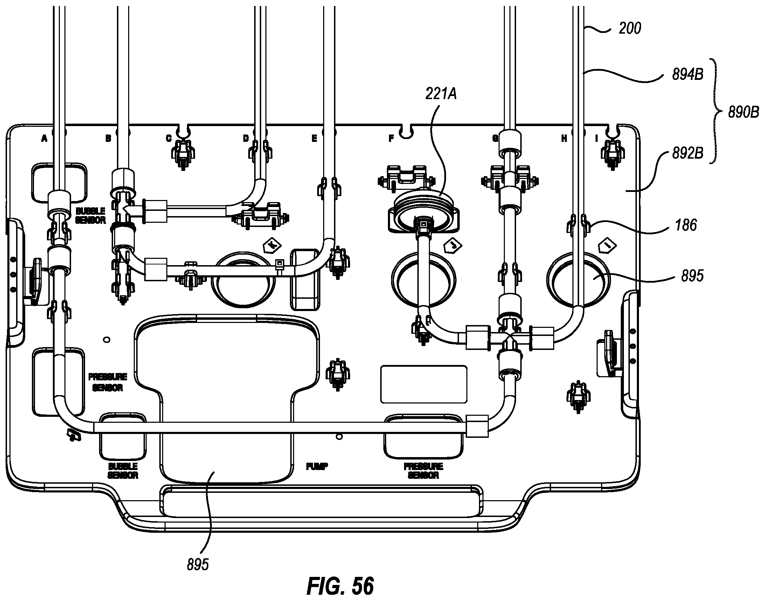

As with bead processing apparatus 22, bead processing apparatus 642 can also be used in the formation of a bead processing system 18A which is an alternative bead processing system 18 (Figure 1). With reference to Figures 56 and 57, bead processing system 18A can be formed by combining bead processing apparatus 642 with a consumable kit 890B that is modified relative to consumable kit 890A. Consumable kit 890B includes a tray 892B and a line set 894B. Tray 892B can have the same properties and be made of the same materials as tray 892A. Although not required, in the depicted embodiment, tray 892B has the same configuration and elements as tray 892A. As such, tray 892B has a top face and an opposing bottom faced with opening 895 extending therethrough to receive corresponding mechanical components and has tube restraints 186 for securing line set 894B thereon.

As depicted in Figures 56 and 57, line set 894B generally comprises flexible tubing 200 fluid coupled with a plurality of bags and with previously discussed air filter 221A. Flexible tubing is positioned to align with select opening 895 on tray 892B so as to engage with select mechanical components projecting from support panel 662. Specifically, during use, as depicted in Figure 56, the assembled consumable kit 890B is placed on top of support panel 662 so that the mechanical components on support panel 662 are aligned with the related openings 895 formed on tray 892A. Tubing 200 is then manipulated to couple with each of pinch valves 128A, 128B, 128D, 128E, 128G 128H, 128I, 128J, and 128K; pump 124; bubble sensors 136A and 136B; and pressure sensor 140A and 140B.

Line set 894B includes tubing sections 954A-954F that extend between tray 892B and corresponding bags. For example, tubing sections 954A and 954B both connect to bead separation bag 570 (or any other bead separation bag disclosed herein), tubing sections 954C and 954E connect to media bags 216A and B, respectively, tubing section 954D connects to an output bag 928, tubing section 954F connects to an input bag 958.

Prior to operation of bead processing system 18A, bead separation bag 570 (or any other bead separation bag, such as bead separation bag 600) is enclosed within rocker assembly 648, the same as previously discussed with isolation bag 206, and the remaining bags are typically secured to bag stands 650A and/or 650B, as previously discussed. The operation of assembled bead processing system 18A is then similar to the previously discussed operation of bead processing system 18. For example, primarily with reference to Figure 57, in one embodiment, bead processing system 18A can be operated using the below steps. Each of the following process steps can be performed either automatically through the control of pre-programmed electrical circuitry 53 and/or can be controlled manually through manual inputs to a user interface. Upon activation of bead processing system 18A, all of pinch valves 128 are typically moved to a closed position so as to preclude fluid flow through the tubing section coupled with the pinch valves. In the following method steps where it is discussed that select pinch valves are opened, it is understood that the remaining pinch valves remain closed to control fluid flow through line set 894B. It is appreciated that in some embodiment, some steps can be eliminated, other steps can be added, and the order of steps can be altered.

Step 1: Lift magnet assembly 241. Lift assembly 292 is used to elevate magnet assembly 294 relative to platform 290/support plate 692 to the raised activation position. In one embodiment, stop assemblies 526A-D can be activated so as to move to the advanced retraining position that limits the ability of rods 770 to rise and thereby limits the ability of cover assembly 421/ lid plate 822 to rise relative to platform 290/support plate 692.

Step 2: Transfer suspension comprised of cells, beads 179 and media through bead separation bag 570. Pump 124 is used to transfer the culture comprised of cells, beads 179 and media that has been processed in cell expansion system 16 through separation bag 570. In the depicted embodiment, the processed cells, beads 179 and media are being dispensed from input bag 958. However, in other embodiments, they can be dispensed directly from cell expansion system 16 or some other container coupled to tubing section 954F. The suspension is pumped into bead separation bag 570 through port 569. The suspension then flows within compartment 415 of bead separation bag 570 (Figure 34) around second end 582 of partition 578 and then out of compartment 415 through port 568. As the suspension is passing through compartment 415, beads 179 are attracted to and secured against platform 290 by the magnetic field produced by magnet assembly 294. In one embodiment, mixing of the suspension can be produced by the activation of rocker drive 234 which can help move beads 179 closer to platform 290/support plate 692 where they are better captured by the magnetic field. In other embodiments, however, the debeading can be performed without activation of rocker drive 234. The optional positioning of stop assemblies 526A-D into the advanced restraining position limits the expansion of bead separation bag 570 so as to further optimize the force of the magnetic field on beads 179 therein. The forgoing enables the efficient collection of beads 179 against platform 290/support plate 692 as the suspension continuously flows through bead separation bag 570. The media and cells leaving separation bag 570 through port 568 travels along tubing 200 to output bag 956 or directly to gene editing system 20 or some other container or system through tubing section 954D.

In one modified version of Step 2, pinch valves 128B, 128I, and/or 128E can remain closed until bead separation bag 570 is at least 30%, 40%, or 50% filled with the mixture of cells, beads 179 and media. Rocker drive 234 then rearwardly tilts platform 290 so that ports 568 and 569 are upwardly tilted. Pinch valves 128B, 128I, and/or 128E are then opened. As additional suspension is pumped into bead separation bag 570 through port 569. any air within bead separation bag 570 flows out through port 568. Once all of the air is removed, rocker drive 234 then tilts platform 290 horizontally. The remainder of the suspension is then pumped through bead separation bag 570 with the debeaded fluid flowing into output bag 956 or some other container or machine.

Step 3: Flow media to output. Pump 124 is then used to pump media from media bag 216B, through bead separation bag 570 and to output bag 956. This process helps to flush out any remaining cells within bead separation bag 570 and/or the tubing.

Beads 179 can remain contained within separation bag 570 and be disposed of or otherwise further processed with line set 894B. Bead processing apparatus 642 has the same benefits and functional properties a previously discussed with regard to bead processing apparatus 22. However, bead processing apparatus 642 is simpler in design, safer to operate, and easier to operate than bead processing apparatus 22.

Figure 58 depicts an exemplary user interface 701 for controlling exemplary magnetic bead processing apparatus, systems and equipment in cell processing workflows as well as other workflows disclosed herein. The user interface 701 can include a screen and display 702 and graphical user interface 704 that displays input/output controls 703 to facilitate control functions and protocol inputs. The user interface 701 can include a touch screen/display 702 or other physical operator input. With the user interface 701, an operator can select and direct the flow of magnetic beads (e.g., along with carrier fluid) into a specific piece of equipment, bioprocess container or flow line. The graphical user interface 704 includes a primary selection area 705 that depicts one or more virtual inputs representing equipment, bioprocess containers, flow lines and/or process parameter inputs in exemplary cell processing workflows as well as other workflows disclosed herein. In this embodiment, the primary selection area 705 includes nine virtual inputs representing four media/buffer bags (media 1 buffer, media 1 buffer 1, media 1 CTS^{™} OPTMIZER^{™} and media 3), an isolation bag, an output bag, a cell separator, a bead bag and a waste bag. Virtual representations of other equipment, bioprocess containers and process parameter inputs can also be included in the primary selection area 705. An operator can select a specific virtual input 703 in the primary selection area 704 to either indicate the location of magnetic beads or direct the flow of magnetic beads to a specific piece of equipment or bioprocess container in the process workflow. This enables an accurate bead calculation during a "RUN" protocol. The graphical user interface 704 can also include a secondary selection area 706, including additional virtual inputs for protocol set-up and operation of workflow equipment. In this embodiment, the secondary selection area 706 includes a "CANCEL" input for cancelling an input made in the primary selection area 705. A "CANCEL" input in the secondary selection area 706 can also trigger a prompt to the operator to confirm cancellation and exit from the protocol creation process. The secondary selection area 706 also includes an "ENTER VOLUME" input, which will take you to the next user interface 801 (described in FIG. 59) for entering culture and other process fluid volumes in selected equipment, bioprocess containers and flow lines in the cell processing workflow as well as other workflows disclosed herein. Other inputs can be included in the secondary selection area 706.

Figure 59 depicts an exemplary user interface 801 for controlling exemplary magnetic bead processing apparatus, systems and equipment in cell processing workflows as well as other worflows disclosed herein. The user interface 801 can include a screen and display 802 and graphical user interface 804 that displays input/output controls 803 to facilitate control functions and protocol inputs. The user interface 801 can include a touch screen/display 802 or other physical operator input. With the user interface 801, an operator can select protocol volumes for pumping cell culture, biological fluids or other process fluids disclosed herein to a specific piece of equipment, bioprocess container or flow line. The graphical user interface 804 includes a primary selection area 805 that depicts one or more virtual inputs 803 representing equipment, bioprocess containers, flow lines and/or process parameter inputs for flowing process fluids at a specific flow rate or volume. In this embodiment, the primary selection area 805 includes six virtual inputs 803 representing process fluid volumes entered by the operator and pumped to four media/buffer bags (media 1 buffer, CTS^{™} OPTMIZER^{™} and media 3), an isolation bag, an output bag, and a cell separator. Virtual representations of other equipment, bioprocess containers and process parameter inputs can also be included in the primary selection area 805. An operator can select input a specific volume for each piece of equipment or bioprocess container in the process protocol to flow that selected volume of process fluid to the equipment or container. The graphical user interface 804 can also include a secondary selection area 806, including additional virtual inputs for protocol set-up and operation of workflow equipment. In this embodiment, the secondary selection area 806 includes a "CANCEL" input for cancelling a volume protocol or an input made in the primary selection area 805. A "CANCEL" input in the secondary selection area 806 can also trigger a prompt to the operator to confirm cancellation and exit from the protocol creation process. The secondary selection area 806 also includes a "CREATE STEPS" input for moving to the next step in the protocol creation process. Other inputs can be included in the secondary selection area 806, including an "BEAD CALCULATOR" input for entering the target cell yield and calculating the bead to cell ratio and bead stock or bead carrier concentration based also on inputs made in the protocol set-up step of Figure 58.

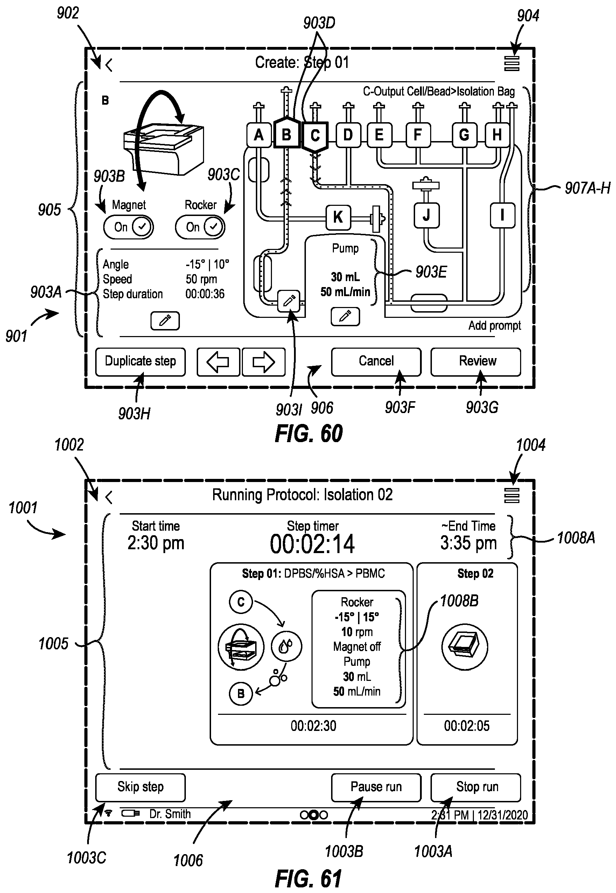

Figure 60 depicts an exemplary user interface 901 for controlling exemplary magnetic bead processing apparatus, systems and equipment in cell processing workflows as well as other workflows disclosed herein. The user interface 901 can include a screen and display 902 and graphical user interface 904 that displays input/output controls 903A-I and fluid flow paths 907A-H to facilitate control functions and protocol inputs. The user interface 901 can include a touch screen/display 902 or other physical input. The graphical user interface 904 includes a primary selection area 905 that depicts one or more virtual inputs 903A-E representing equipment, bioprocess containers, flow lines and/or process input parameters for a specific workflow protocol. In this embodiment, the primary selection area 905 includes a robust set of virtual inputs 903A-E, I for operating a rocker, magnet and pump in the process workflow. For example, virtual inputs 903A are process parameter inputs for entering and controlling the angle range, speed and duration of rocking for the rocker. Virtual input 903C can be actuated to turn the rocker on and can also depict that the rocker is on. Virtual input 903B can be actuated to turn the magnet and can also depict that the magnet is on. Virtual inputs 903D can be actuated to open and close valves and flow process fluids with the pump through the highlighted corresponding flow paths 907B, 907C. Other fluid flow paths 907A-H are also depicted to facilitate the same type of valve actuation and fluid flow control through fluid paths 907A-H. Virtual inputs 903E are process parameter inputs, including flow rates and fluid volumes, for controlling the pump to flow process fluids at the designated flow rate and volume through the highlighted fluid paths 907B, 907C. Virtual input 903I is a virtual pen icon to the left of the virtual representation of the pump. That pen icon/button 903I can be actuated to engage a bubble sensor. An operator can determine whether to activate the bubble sensor when the pump is in use to select whether to Stop the bubble sensor when dry (wet to dry) or Stop the bubble sensor when wet (dry to wet). If the bubble sensor is active, it controls when the pump stops, and thus, overrides the inputs in the pump fields 903E. The primary selection area 905 can also provide virtual images of the rocker, magnet and pump hardware. Virtual representations of other equipment, bioprocess containers and process parameter inputs can also be included in the primary selection area 905. The graphical user interface 904 can also include a secondary selection area 906, including additional virtual inputs 903F-H for protocol set-up and operation of workflow equipment. In this embodiment, the secondary selection area 906 includes a 903G "REVIEW" input for reviewing inputs made in the primary selection area 905 before the protocol is initiated. The secondary selection area 906 also includes a 903F "CANCEL" input for cancelling a protocol or input made in the primary selection area 905. A 903G "CANCEL" input in the secondary selection area 906 can also trigger a prompt to the operator to confirm cancellation and exit from the protocol creation process. Other inputs can be included in the secondary selection area 906, including a 903H "DUPLICATE" input for duplicating the depicted protocol step configured through the user interface 901.

Figure 61 depicts an exemplary user interface 1001 for controlling exemplary magnetic bead processing apparatus, systems and equipment in cell processing workflows as well as other workflows disclosed herein. The user interface 1001 can include a screen and display 1002 and graphical user interface 1004 that displays input/output controls 1003A-C and protocol parameters 1008A-B to facilitate control functions and protocol inputs. The user interface 1001 can include a touch screen/display 1002 or other physical input. The graphical user interface 1004 includes a primary selection area 1005 that depicts one or more virtual inputs representing equipment, bioprocess containers, flow lines and/or process input parameters for a specific workflow protocol. In this embodiment, the primary selection area 1005 includes a set of process parameters 1008A-B. For example, time process parameters 1008A can depict the start time, step timer and end time in the primary selection area 1005 for the running isolation protocol. Equipment process parameters 1008B can depict the rocker angle range, rocker speed, magnet on/off status and pump flow rate and fluid volume set point in the primary selection area 1005. The primary selection area 1005 can also depict the step of the protocol *(e.g.,* step 01 or step 02) that us being run in addition to virtual images of the rocker, magnet, pump and relevant flow paths B-C that are being used. Virtual representations of other equipment, bioprocess containers and process parameter inputs can also be included in the primary selection area 1005. The graphical user interface 1004 can also include a secondary selection area 1006, including additional virtual inputs for protocol set-up and operation of workflow equipment. In this embodiment, the secondary selection area 1006 includes a 1003A "STOP RUN" input for stopping the isolation protocol or protocol step; a 1003B "PAUSE RUN" input for pausing protocol on the current step; and a 1003C "SKIP STEP" input for skipping the isolation protocol. A 1003A "STOP RUN" input can also trigger a prompt to the operator to confirm that the operator wants to stop and exit the run and protocol. Other inputs can be included in the secondary selection area 1006.

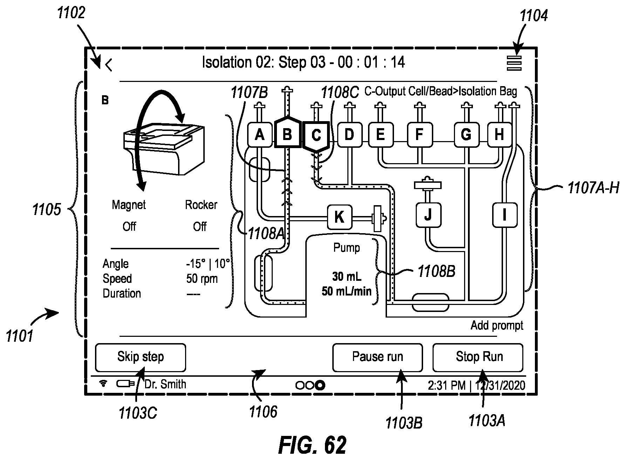

Figure 62 depicts an exemplary user interface 1101 for controlling exemplary magnetic bead processing apparatus, systems and equipment in cell processing workflows as well as other workflows disclosed herein. The user interface 1101 can include a screen and display 1102 and graphical user interface 1104 that displays input/output controls 1103A-C, protocol parameters 1108A-B and fluid flow paths 1107A-H to facilitate control functions and protocol inputs. The user interface 1101 can include a touch screen/display 1102 or other physical input. The graphical user interface 1104 includes a primary selection area 1105 that depicts one or more virtual inputs 1108A-B representing equipment, bioprocess containers, flow lines and/or process input parameters for a specific workflow protocol. In this embodiment, the primary selection area 1105 includes a set of process parameters 1108A-B and a set of virtual flow paths 1107A-H through which process fluids are pumped. For example, process parameters 1108A include a virtual representation of a rocker and magnet assembly; the rocker angle range, speed and duration of rocking, and whether the magnet and rocker are on/off. Equipment process parameters 1108B in the primary selection area 1105 also depict a virtual representation of a pump, the volume of process fluid being pumped and the fluid flow rate for the isolation protocol. The primary selection area 1105 can also depict relevant fluid flow paths 1107 A,C that are being used to pump the process fluid and other fluid flow paths 1107A-H that can be used to pump process fluid to equipment, containers and flow lines in the workflow. Virtual representations of other equipment, bioprocess containers and process parameter inputs can also be included in the primary selection area 1105. The graphical user interface 1104 can also include a secondary selection area 1106, including additional virtual inputs for protocol set-up and operation of workflow equipment. In this embodiment, the secondary selection area 1106 includes a 1103A "STOP RUN" input for stopping the isolation protocol step; a 1103B "PAUSE RUN" input for pausing the current protocol step; and a 1103C "SKIP STEP" input for skipping the isolation protocol step. A 1103A "STOP RUN" input can also trigger a prompt to the operator to confirm that the operator wants to stop and exit the run and protocol. Other inputs can be included in the secondary selection area 1106.

Figures 58-62 depict user interfaces for setting-up and running a biomaterial or cell isolation protocol. Similar user interfaces can be used to set-up and run a bead removal protocol described in the biological workflows disclosed herein. The user interfaces disclosed herein can include an electronic device, comprising a display, a touch-sensitive surface, one or more processors, memory, and one or more programs stored in the memory and configured to be executed by the one or more processors. The one or more programs include instructions for displaying, on the display, one or more graphical user interfaces disclosed herein and executing the functions of the graphical user interfaces.

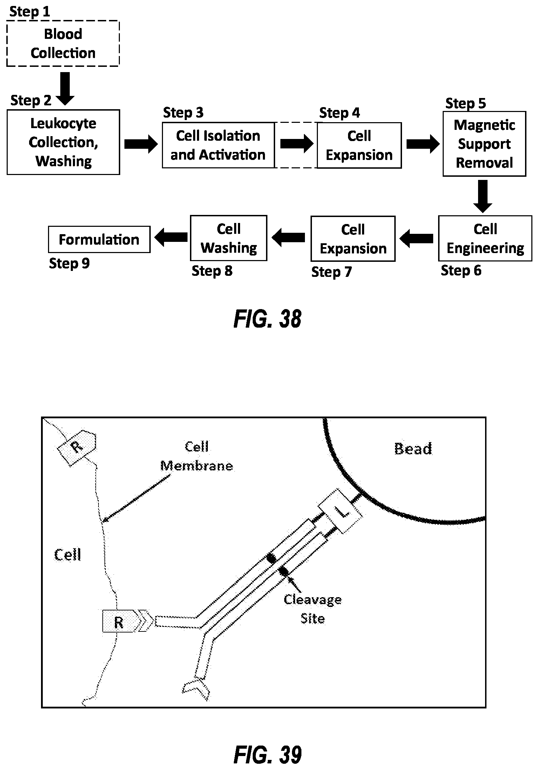

Provided herein are instruments and workflows for cell processing (*see, e.g.,* Figure 1). Figure 38 is a schematic of exemplary workflows provided herein. It is understood that in one embodiment, the biological workflows discussed below with reference to Figures 38 and 39 can be performed using the hardware previously discussed herein and specifically identified in Figure 1. The steps of the schematically represented workflow set out in Figure 38 can be performed where cells are treated under different conditions at different time points and, in some instances, using different types of equipment. Thus, provided herein are modular cell processing workflows in which cells may be processed, for example, as a series of stations and/or using two or more different instruments.

Further, in some instances, the order of steps set out in Figure 38 may be altered and some steps may be repeated consecutively or in another location in the workflow. As examples, in some instances, some workflows may involve performing the following steps in sequences: (A) Step 3, Step 4, Step, 5, Step 6, and Step 7; (B) Step 3, Step, 5, Step 6, and Step 7; or (C) Step 3, Step 4, Step, 5, Step 6, and Step 8. Further, due to the length of time cells are activated, Step 3 and Step 4 may overlap or be one in the same. This is so because, in many instances, cells begin to expand once activated. Further, cells may remain in contact with activating stimuli for an extended period of time. This will often be the case when activation stimuli (*e.g.*, anti-CD3 and anti-CD28 antibodies) remain in contact with cells until Step 5.

Further, some of all of the steps set out in Figure 38 may be performed as a closed system. By closed system is meant that cells remain in closed vessels during a single process or multiple processes. For example, in Step 1 through Step 5, cells may be transferred between bags but the interior of each bag may be a sterile environment and the cells may be transferred from bag to bag using sterile tubing and connectors. In such an instance, Steps 1 through Steps 5 would be considered to be a closed system and would further be considered to be a sterile, closed system. In some instances, Step 2 through Step 9 (or a subset of such steps) will be performed in a closed system.

In many instances, workflows set out herein will be directed to the generation of CAR-T cell populations.

The first step in workflows of Figure 38 (Step 1) is the collection of blood from an individual *(e.g.,* a patient). This individual may not be in need of therapeutic treatment related to the blood collection. Alternatively, this individual may be, for example, afflicted with a condition for which the treatment of involves administration of the formulation of Step 9. The blood obtained from the individual may be processed, for example, by leukapheresis where the blood is removed from the individual's body, the leukocytes are collected, and the uncollected blood components are returned to the individual. An instrument that may be used for PMBC isolation from whole blood is the CELL SAVER^{™} 5+ Autologous Blood Recovery System (Haemonetics Corporation, Boston, MA).

Once the desired number of leukocytes have been harvested, the resulting cell population is generally washed (Step 2) to remove, for example, anticoagulant(s). In an early step, the cell population may be enriched for lymphocytes (Step 2) using, for example, a counterflow centrifugal elutriation system *(e.g.,* a GIBCO^{™} CTS^{™} ROTEA^{™} Counterflow Centrifugation System, Thermo Fisher Scientific), which can separate cells by size and density.

Isolation of desired cells types (Step 3) *(e.g.,* total T cell and T cell subsets, CD34+ stem cells, natural killer cells, as well as other cell types), may be performed using ligands having binding affinity for cell surface receptors. Examples of such cell surface receptors include CD3, CD4, CD5, CD6, CD8, CD25, CD27, CD28, CD137, and CD278 (ICOS). Further, isolation and activation may occur simultaneously. As an example, a mixed population of leukocytes may be exposed to anti-CD3 and anti-CD28 antibodies under conditions in which T cells are separated from other leukocytes and the combination of the anti-CD3 and anti-CD28 antibodies results in T cell activation.

By way of specific example, T cells may be isolated based upon the presence on their surfaces of CD3 markers. Some isolation methods use positive isolation of cells with the desired surface marker. An exemplary method for T cell isolation is as follows. A mixed leukocyte population is incubated (*e.g*., 20-30 minutes at 4°C) with magnetic beads with anti-CD3 antibodies located on the bead surfaces (*e.g.*, DYNABEAD^{™} CD3, Thermo Fisher Scientific, cat. no. 11151D) for sufficient time for the beads to associate with T cells in the population. The cells are then contacted with a magnetic field under conditions that allow for cells bound to the beads to be retained while cells not bound to the beads to be removed (*e.g*., by washing). This results in the separation of T cells from non-T cells of the leukocyte population.

In many instances, once T cells have been isolated, these cells will be contacted with an anti-CD28 antibody capable of stimulating CD28 receptors, resulting in T cell activation. The anti-CD28 antibodies may be bound to magnetic beads. Further, as noted above, in some instances, the capture of T cells by anti-CD3 antibodies and stimulation of T cells by a combination of anti-CD3 and anti-CD28 antibodies may occur simultaneously. A commercially available product that may be used for both T cell isolation and activation is entitled CTS^{™} DYNABEADS^{™} CD3/CD28 (Thermo Fisher Scientific, cat. no. 40203D). These beads may be used for *ex vivo* isolation, activation, and expansion of human T-cells (*e.g*., human T cells) and combine anti-CD3 and anti-CD28 antibodies on the individual beads. Thus, in addition to allowing for the separation of T cell from other cell types, these beads provide both the primary and co-stimulatory signals that are required for activation and expansion of T cells.

Samples of T cells exposed to anti-CD3 antibodies and/or anti-CD3 and anti-CD28 antibodies may be analyzed for activation levels. One type of assay for measuring activation is based upon screened T cells for CD25 (the alpha chain of the IL-2 receptor) expression levels. While the CD25 marker is found on a number of peripheral blood lymphocytes (*e.g.*, regulatory and resting memory T cells), CD25 expression is generally considered to be a prominent T cell activation marker. Thus, methods provided herein include methods for measuring the percentage of activated T cells in a population. This percentage is calculated by comparing the number of nonactivated T cells with the number of activated T cells. Of course, the percentage of activated T cells will change with the duration of exposure to activation signals and as activated T cells expand.

Magnetic beads may be added to sample of biological cells by any number of means. In many instances, beads will be introduced into containers containing biological cells through interaction of the apparatus with a bead vial. However, beads may be introduced into containers by other means. For example, beads may be introduced into bags by injection through a syringe connected to a bag port. When magnetic beads are contacted with biological cells by means not using a vial, then the bead vial coupler 208, as well as other bead vial feature of the apparatus may not be present.

Step 4 in the exemplary workflow of Figure 38 is cell expansion. In many instances, this step will overlap with Step 3 (Cell Isolation and Activation). This is so because often cells will be exposed with activation signals for an extended period of time (*e.g*., from about 1 day to about 20 days, from about 2 days to about 20 days, from about 4 days to about 20 days, from about 4 days to about 15 days, from about 4 days to about 14 days, from about 6 days to about 14 days, etc.). Further, during this period of time, activated cells will often begin to expand.

While expansion conditions may vary conditions, activated T cells may be cultured, for example, at 37°C and 5% CO₂ in cell culture medium (*e.g.*, CTS^{™} OPTMIZER^{™} media without phenol red plus 2-5% CTS^{™} Immune Cell SR (Thermo Fisher Scientific, cat. nos. A3705001 and 15710-049). Further, cytokines and fresh medium may be added every 1-3 days to maintain a cell concentration of 0.5-2x10⁶ cells/ml. T regulatory cells (Tregs) may be expanded in medium containing 100 ng/ml rapamycin (*e.g*., Thermo Fisher Scientific, cat. no. PHZ1235) and 300 IU IL-2/ml (*e.g*., Thermo Fisher Scientific, cat. no. PHC0027). CMV stimulated T cells may be expanded in 100 IU IL2/ml. Th17 cells may be expanded in medium containing polarizing cytokines (IL-6, IL-13, IL-23, and TGF-13, all, for example, from Thermo Fisher Scientific CA USA) in presence of anti-IL-4 and anti-IFN-γ neutralizing antibodies (both, for example, from Thermo Fisher Scientific, CA US) as described in Paulos *et al.* (Paulos et al., Science Transi. Med 55:55ra78 (2010)). Further, 100 IU IL-2/ml may be added day 3 post-activation. IL2 may also be added one day 0, for example, during dilution of cells from the isolation bag to the output.

Expansion of cells (Steps 4 and 7) will generally occur under conditions suitable for cell division. Media that may be using for expansion include CTS^{™} OPTMIZER^{™} T-Cell Expansion SFM (Thermo Fisher Scientific, cat. no. A3705001) and LYMPHOONE^{™} T-Cell Expansion Xeno-Free Medium (Takara Bio, cat. no. WK552S).

In some instances, it may not be necessary or desirable to separate magnetic supports from cells. In such instances, Step 5 set out in Figure 38 may be omitted.

It will generally be desirable at some point in workflow to separate magnetic supports from cells. In some instances when the supports are bound to the cells, it will be necessary or desirable to disrupt the binding of the supports to the cells. For example, the cells and the magnetic supports may be associated with each other through conjugation of antibodies to magnetic supports. Figure 39 shows a schematic of a cell bound to a support (*i.e.,* a bead). The antibody is shown in Figure 39 bound to a cell surface receptor (*e.g.,* a CD3, CD4, CD8, CD11a, CD11b, CD14, CD15, CD16, CD19, CD20, CD22, CD24, CD25, CD28, CD30, CD31, CD34, CD38, CD45, CD56, CD61, CD91, CD114, CD117, CD182, etc.), labeled "R". Further, the cell type(s) bound by the antibodies may be one or more of any number of cell types (*e.g.,* stem cells, leukocytes in general, granulocytes, monocytes, total T cells, helper T helper cells, regulatory T cells, cytotoxic T cells, B cells, natural killer cells, thrombocytes, etc.).

Disruption of association of magnetic supports from cells may be accomplished by a number of means. Some exemplary cell release features are represented in Figure 39. One is the inclusion of cleavage sites in the antibodies that allow for disruption of antibody association with the cells or the support. In some instances, the disruption of antibody association may be based upon cleavage of the antibody into different parts where one part contains the antigen binding domains and another part is associated with the support. An exemplary cleavage mechanism involves antibody cleavage. Antibody cleavage may be mediated, for examples, by naturally occurring protease cleavage sites or protease cleavage sites that have been introduced into the antibodies. Proteases that may be used include a tobacco etch virus (TEV) protease, a TEV protease with an S219V modification (*e.g*., ACTEV^{™} Protease, Thermo Fisher Scientific, cat. no. 12575015), a rhinovirus 3C protease, a TVMV protease, a plum pox virus protease, and a turnip mosaic virus protease, enteropeptidase, thrombin and Factor Xa. Thus, methods set out herein include those where cells and supports are dissociated from each other by cleavage mediated by one or more protease. Also included herein are compositions for performing such methods (*e.g*., antibodies engineered to contain one or more protease cleavage site). Some methods related to the above are set out in U.S. Patent Publication No. 2017/0313772 A1.

Another way that disruption of association of magnetic supports from cells may be accomplished is by competitive release. For example, "L" in Figure 39 may be composed of two components that noncovalently associate with each other. One specific example is biotin (or a biotin derivative) and streptavidin. Biotin and streptavidin noncovalently bind to each other with high affinity (dissociation constant (Kd) 4x10⁻¹⁴ M). Methods set out herein include those where cells are associated with supports through the interaction of biotin and streptavidin (or similar protein, such as avidin). In some specific methods, streptavidin is covalently linked to the support and biotin (or a biotin derivative) is covalently bound to the antibody. In some instances, the biotin will be a biotin derivative that associates with streptavidin with a Kd that is lower than 4x10⁻¹⁴ M (*e.g.,* from about 4x10⁻¹³ M to about 4x10⁻⁷ M, from about 4x10⁻¹² M to about 4x10⁻⁷ M, from about 4x10⁻¹⁰ M to about 4x10⁻⁷ M, from about 4x10⁻¹³ M to about 4x10⁻⁸ M, from about 4x10⁻¹³ M to about 4x10⁻⁹ M, etc.). Exemplary biotin derivatives include N-ethyl biotin and desthiobiotin. A number of biotin derivatives that may be used in methods set out herein are set out in U.S. Patent No. 9,567,346.

One processes that may be employed for the dissociation of cells and supports make use of anti-biotin antibodies. For example, a two antibody linking systems can be used where a first biotinylated antibody is used wherein the first antibody has binding affinity for a cell surface protein (*e.g.,* a receptor). A second anti-biotin antibody may be conjugated to a support. Thus, the cells are associated with supports, in part, through the binding of the binding of the bead bound second antibody (anti-biotin antibody) to the first antibody (biotinylated, anti-cell surface protein antibody). Disruption of association between the supports and cells may be mediated by disruption of the binding of the second antibody to the biotin of the first antibody. This may be accomplished by the contacting the cell/bead complex with a releasing agent (*e.g*., biotin or biotin derivative). Compositions and methods related to the above are contained in U.S. Patent No. 10,196,631.

It has been found that when beads bind to the surfaces of cells through antibodies, the bead are often released by the cell as the cells expand, as a result of downregulation of the cell surface marker bound to the antibody on the beads. Thus, in many instances, cells may be separated from beads without the performance of an active dissociation step. In many such instances, separation of cells from beads will occur after cells (*e.g.,* T cells) have been expanded for from about 4 to about 21 days (*e.g.,* from about 4 to about 21, from about 5 to about 21, from about 6 to about 21, from about 5 to about 14, from about 5 to about 12, from about 5 to about 10, from about 6 to about 14, from about 6 to about 12, from about 6 to about 10, etc. days). Also in many such instances, separation of cells from beads will occur after cells (*e.g.,* T cells) after greater than 70% (*e.g.,* from about 70% to about 99%, from about 70% to about 98%, from about 70% to about 95%, from about 70% to about 90%, from about 70% to about 85%, from about 75% to about 99%, from about 80% to about 99%, from about 85% to about 99%, from about 85% to about 95%, from about 85% to about 90%, from about 90% to about 99%, from about 90% to about 97%, etc.) of the cells are dissociated from beads. Of course, the user can separate the beads from the cells at any time point. However, in many instances, separation of the beads from the cells at an early time point will result of doing in a lower cell yield than if the beads are separated at a later time point. This is so because more beads will often be captured by the magnet at the earlier time point. Thus, in many instances, separation of cells from beads will occur will occur at a time point where cell yields are greater than 80% (*e.g.,* from about 80% to about 99.5%, from about 85% to about 99.5%, from about 88% to about 99.5%, from about 90% to about 99.5%, from about 95% to about 99.5%, from about 98% to about 99.5%, from about 80% to about 98%, from about 85% to about 98%, from about 90% to about 98%, etc.). Cell yield is measured in such instances by the percentage of the total number of cells present being separated from the beads.

Cells not bound to or associated with magnetic supports may be separated from these supports using a magnetic field to capture the supports under conditions where the cells are not associated with a magnetic material. The examples below set out experiments and data using magnetic beads. Further, using instruments such as those set out herein, it has been shown to be possible to remove greater than 99% of the magnetic beads present in a bead/cell mixture. Thus, provided herein are methods for separation of magnetic beads from cells present in a bead/cell mixture, wherein greater than 95% (*e.g.,* from about 95% to about 99.9999%, from about 97% to about 99.9999%, from about 98% to about 99.9999%, from about 99% to about 99.9999%, from about 98% to about 99.95%, from about 98% to about 99.90%, etc.) of the beads originally present are separated from the cells.

As set out in the examples below, debeading of a bead/cell mixture may be performed using bead processing system 18, as previously discussed herein, in conjunction with a bag of the type, for example, shown in Figure 34. Such a bag allows the fluid being passed therethrough to not "short circuit" contact the device magnet. Thus, the fluid must pass over a substantial length of the magnet before exiting the bag.

The flow rate through the bag is also a parameter that affects the debeading efficiency. It has been found the slower flow rates result in more efficient debeading and, hence, higher cell purity with respect to the number of beads present post-debeading.

Flow rates may vary from about 10 ml/min to 400 ml/min (*e.g*., from about 10 ml/min to 400 ml/min, from about 20 ml/min to 400 ml/min, from about 30 ml/min to 400 ml/min, from about 40 ml/min to 400 ml/min, from about 40 ml/min to 300 ml/min, from about 40 ml/min to 200 ml/min, from about 40 ml/min to 100 ml/min, from about 50 ml/min to 300 ml/min, from about 50 ml/min to 200 ml/min, from about 45 ml/min to 150 ml/min, etc.). Often the flow rate will be selected to allow for removal of at least 99% (from about 99% to about 99.9999%, from about 99% to about 99.99%, from about 99.5% to about 99.9999%, from about 99.8% to about 99.9999%, from about 99% to about 100%, etc.) of the beads. Thus, provided herein are methods for the separation of at least 99% of bead present in a bead/cell mixture. In many instances, such separation methods will result in the bead to cell ratio decreasing by a factor of at least 7 (*e.g.,* a factor of from about 2 to about 7, from about 3 to about 7, from about 4 to about 7, from about 2 to about 7, from about 5 to about 7, from about 2 to about 6, from about 3 to about 6, from about 4 to about 6, from about 5 to about 6, from about 2 to about 5, from about 3 to about 5, from about 3 to about 5, etc.). By way of example, going from a bead to cell ratio of 3:1 to a bead to cell of 0.3:1 would be a one factor decrease. Further, going from a bead to cell ratio of 3:1 to a bead to cell of 0.03:1 would be a two factor decrease.

Example 3 shows data generated using bead processing systems and methods set out herein. These data show that over 99.99% of the beads were removed in the debeading process. It was further estimated that only 1 bead per 450,000 of the original beads present co-localized with the debeaded cells. Results from three replicates using conditions similar to those of Example 3 but with a 200 ml/min flow rate were 0, 1 and 2 total beads and, thus, 0 (0 beads), 13 (1 bead), a 27 (2 beads) co-localized beads per 3x10⁶ lysed cells. Thus, in some instances, cell may be separated from as much as 100% of the originally present.

Step 6 set out in Figure 38 is cell engineering. In some instances, this step will not be performed. Further, when this step is performed, it may differ greatly for the cell type being engineered and the purpose of the cell engineering.

T cells, for example, may be engineered to expression a chimeric antigen receptors (CARs). CARs are receptors that are designed to bind to cell surface proteins on target cells (*e.g.,* human leukocyte antigen antigens. Further, T cells may be engineered to express CARs on their surface, allowing them to recognize specific antigens (*e.g*., tumor antigens). These CAR T cells can then be expanded by methods of the present invention and infused into the patient. Typically, this will occur after the T cells are washed (Step 8 in Figure 38) and formulated for patient administration (Step 9 in Figure 38).

In some instances, cells (*e.g.,* a T cell) may be engineered to express a CAR wherein the CAR T cell exhibits an antitumor property. CARs can be designed to comprise an extracellular domain having an antigen binding domain fused to an intracellular signaling domain of the T cell antigen receptor complex zeta chain (*e.g.,* CD3 zeta). Such a CAR, when expressed in a T cell is able to redirect antigen recognition based on the antigen binding specificity.

The antigen binding moiety of a CAR may comprise a target-specific binding element otherwise referred to as an antigen binding moiety. The choice of moiety used will often depend on the type and number of ligands that define the surface of a target cell. For example, the antigen binding domain may be chosen to recognize a ligand that acts as a cell surface marker on target cells associated with a particular disease state. Thus, the antigen moiety domain in the CAR of may be associated with viral, bacterial and parasitic infections, autoimmune disease and cancer cells.

The expression of natural or synthetic nucleic acids encoding CARs is typically achieved by operably linking a nucleic acid encoding the CAR polypeptide or portions thereof to a promoter and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

Some methods used to engineer cells employ replication deficient lentiviral vectors to deliver nucleic acids to the cells. For example, nucleic acid molecules encoding CARs are often introduced into cells using such vectors. In particular, lentiviral vectors with the VSV-G pseudotype allow for efficient transduction under automated manufacturing methods. Further, cell engineering may be performed using a number of viral systems, such as Moloney Murine Leukemia Virus (MMLV), gibbon ape leukemia virus (GALV), feline endogenous retrovirus (FERV), baboon endogenous retrovirus (BaEV), and various gamma or alpha retroviral vectors.

Cell engineering may also be performed by introduction of nucleic acid molecules into cells by transfection of electroporation. One instrument that may be used in such methods is the NEON^{™} Transfection System (Thermo Fisher Scientific, cat no. MPK10025), which has been found to allow for up to 90% transfection with difficult to transfect cells and can be used to transfect 6x10⁶ cells per reaction.

Nucleic acid molecules into cells may remain episomal or may integrate into cellular nucleic acid molecules (*e.g*., chromosomal DNA, mitochondrial DNA, etc.). In many instances, integration of nucleic acid molecules will be mediated by nonhomologous end joining or homologous recombination. Further, in many instances, it will be desirable for nucleic acid molecules to integrate into cellular nucleic acid at a specific locus, such as a "safe harbor". In such instances, a "nucleic acid cutting entity" capable of generating site single-stranded or double-stranded breaks in nucleic acids may be used. A number of nucleic acid cutting entities are known in the art. For example, in some embodiments the nucleic acid cutting entity includes one or more zinc finger proteins, transcription activator-like effectors (TALEs), CRISPR complex (*e.g*., Cas9 or CPF1), homing endonucleases or meganucleases, argonaute-nucleic acid complexes, or macronucleases.

Support (*e.g*., magnetic supports, such as beads) based purification of nucleic acid molecules may be based on liquid and stationary phases, allowing for selectively separation of nucleic acid molecules from each other as well as from other types of molecules.

Provided herein are compositions and methods for the purification of nucleic acid molecules. In many instances, such methods will involve the association of nucleic acid molecules with supports (*e.g*., magnetic supports, such as beads). These supports may then be held in place by a magnetic field allowing for separation of materials associated with the supports from materials not associated with the supports.

Nucleic acid molecules (as well as other type of molecules and sometimes cells comprising such molecules) may be associated with supports by, for example, covalent bonds, non-covalent bonds (*e.g.,* ionic interactions), precipitation, or a combination of such processes. Exemplary methods for nucleic acid purification involve the use of supports composed of or containing (1) silica, (2) glass, (3) diatomaceous earth, (4) anion exchange materials, (5) cellulose, and (6) affinity association materials (*e.g*., oligo dT, biotin-streptavidin, etc.).

Nucleic acid molecule association mechanisms with various materials and groups vary. Silica for example, is believed to associate with nucleic acid molecules by attraction between negatively charged groups of nucleic acid molecules with negatively charged groups of silica. While the mechanism of action is not fully known, it is believed that salt bridges form between the negatively charged groups and/or the silica surface and the DNA become dehydrated.

Similar to silica, anion exchange materials associate with negatively charged nucleic acid molecules through negatively charged groups (*e.g*., carboxylic acid groups). Further, associated with a support (*e.g*., magnetic supports, such as beads), nucleic acid molecules (as well as other types of molecules, such as proteins) may be precipitated to enhance association with the support. Precipitation may be mediated by contacting the support with, for example, a high salt, alcohol solution (*e.g*., 70% ethanol), followed by washing with an alcohol solution to remove the salt. One advantage of nucleic acid molecule precipitation is that it allows for the removal of solutes (*e.g*., salts) from the support bound nucleic acid molecule prior to nucleic acid release by solubilization (*e.g*., solubilization by an aqueous solution without ethanol). In some instances, this will result in a low salt, soluble nucleic acid molecule solution for which there is it not necessary to remove salts (*e.g.,* by dialysis). Examples of such processes are set out below in Examples 4 and 5.

Once nucleic acid molecules are associated with a support (*e.g*., magnetic supports, such as beads) and separated from other materials, it will generally be desirable to separate the associated nucleic acid molecules from the supports. This will normally involve release of the nucleic acid molecules from the supports, followed by physical separation of the support from the nucleic acid molecules. Release may be mediated in any number of way, including alteration of pH, ionic strength, and/or osmolality. Release may also be mediated by the use metal ion chelation (*e.g*., mediated by EDTA, EGTA, etc.) and competitive ligand binding.

When purification of total nucleic acid in sample is desired, then nucleic acid molecules will often be associated with supports by a mechanism that is not nucleic acid type or sequence specific. When purification of a specific type of nucleic acid in sample is desired, then nucleic acid molecules will often be associated with supports by a mechanism that results in association with one or more specific feature of the desired nucleic acid molecules. By way of example, mRNA may be separated from other materials, including other nucleic acid molecules (*e.g*., genomic DNA molecules, plasmid DNA molecules, ribosomal RNA molecules, tRNA molecules, etc.) by the presence of 3' polyA regions. Thus, mRNA may be purified by contact with a support (*e.g*., magnetic supports, such as beads) capable of binding to polyA nucleic acid regions, followed by washing of support and the bound mRNA, then followed by release of the mRNA from the support, and then separation of the support from the mRNA. A number of supports that may be used in such processes are commercially available (*e.g.*, Thermo Fisher Scientific, DYNABEADS^{™} mRNA Purification Kit, cat. no. 61006 and New England Biolabs, Magnetic mRNA Isolation Kit, cat. no. S1550S).

Methods for the production and purification of mRNA are set out in Examples 4 and 5. Initially in these examples, biotinylated DNA molecules is produced by a polymerase chain reaction (PCR) with a biotinylated primer. The resulting biotinylated DNA molecule encodes a mRNA molecule that is operably linked to a T7 promoter and is bound to magnetic bead with surface bound streptavidin. Once bound to the magnetic bead, the DNA molecule/bead complexes are separated from PCR reaction mixture components. Bead bound DNA is then transcribe by an *in vitro* transcription (IVT) reaction mixture. The resulting mRNA in not associated with the magnetic beads and, thus, may be separated from the beads by the beads being held in place by a magnetic field. The mRNA molecules are then separated from IVT reaction mixture components by association with magnetic beads comprising carboxylic acid groups. In this instance, mRNA molecules are held in place while IVT reaction mixture components are removed by washing. Thus, the first separation is a negative selection and the second separation is a positive selection.

Further, in many instances, IVT transcription templates may be reused a number of times. For example, it has been found that T7 promoter driven IVT transcription templates bound to supports may be reused multiple (*e.g*., from about 2 to about 15, from about 3 to about 15, from about 4 to about 15, from about 5 to about 15, from about 2 to about 10, from about 2 to about 8, etc.) times by re-adding fresh IVT reaction mixture components.

Bead processing assemblies and systems set out herein may be used for all or part of workflows involving processes such as those set out above and in Examples 4 and 5, as well as other processes set out herein. Again, using the processes set out in Examples 4 and 5 for illustration, a single solution container (*e.g*., bag) could be used for initial purification of mRNA. This is so because the magnetic supports (streptavidin beads) with the transcription template for purification is first held in place and then is held in place again when the transcription template is separated from the transcription product (*i.e.,* the mRNA molecules). When additional purification of the mRNA is desired, then a second container (*e.g.*, bag) will often be used. This is so because the mRNA molecule released from the magnetic supports and removed from the container may be further purified by associated with other magnetic supports (carboxylic acid beads) and, in many instance, these other magnetic supports may be held in place after washing and release of the mRNA molecules.

In many instances, reagent addition and removal may be performed again using bead processing assemblies and systems set out herein. Further, a heating pad or element may also be added to the bead processing assemblies and systems set out herein for altering reaction temperatures for various purposes, including enhancing the release of from solid supports and/or solubilization of molecules (*e.g*., precipitated nucleic acid molecules) after purification. Heating may also be useful for other purposes. For example, when bead processing assemblies and systems set out herein are used for *in vitro transcription*, it will generally be advantageous to heat the reaction mixture to 37°C or other suitable temperature. Further, higher temperature (*e.g*., 65°C) may be used to release nucleic acid molecule from solid supports (*e.g*., mRNA from magnetic beads with carboxylic acid groups).

Along these lines, bead processing assemblies and systems set out herein may contain heating and/or cooling elements for increasing or decreasing the temperature of containers (*e.g*., bags). These heating and/or cooling elements may be used to, for example, accelerate reactions, deaccelerate reactions, solubilize materials, or precipitate materials.

Further, in-line or reservoir heating and/or cooling maybe used in conjunction with or as part of bead processing assemblies and systems set out herein. Heating and cooling elements allow for heating or cooling of materials either prior to introduction into a container or after materials have come out of a container. Using the schematic shown in Figure 11 for purposes of illustration, bag 216A may either contain of be in contact with a heating element, bag 216B may either contain of be in contact with a cooling element, and bag 216C may be maintained at room temperature. Further, mixing bag 210 may either contain of be in contact with a heating or cooling element. Additionally, collection bag 212 may also either contain of be in contact with a heating or cooling element.

In some instances, the biological material that is sought to be purified may be a protein. Any number of methods may be used for such purification, including for example, covalent bonds, non-covalent bonds (*e.g*., ionic interactions), precipitation, or a combination of such processes. In some instances, binding partner affinity may be used, with the binding partner (ligand) varying with the protein that is sought to be purified.

When a protein is purified using bead processing assemblies and systems set out herein, one binding partner will often be associated with a solid support (*e.g.,* a magnetic bead) and the other binding partner will be associated with the protein to be purified.

Proteins that may purified by methods set out here may vary greatly. Proteins purified by methods set out here may also be bound to affinity reagents that bind to the protein either to a naturally occurring region of the protein or to an exogenously added tag. In addition to examples such as Protein A binding to antibodies, such methods include those where an antibody with specificity for the protein being purified is linked to a solid support. For example, when the protein is an antibody (*e.g*., an IgG, an IgA, an IgD, an IgM, an IgE, etc.) or a mixture of antibodies (*e.g*., IgG antibodies present in serum, etc.), the ligand may be Protein A, Protein G, Protein L, or one or more functional variant of one or more of these proteins.

Thus, proteins purification methods may also be based on association with an exogenously added affinity tag. By this it is meant that the affinity tag is not normally present in the naturally occurring protein. Exemplary tags and binding partners that may be present in compositions and used in methods set out herein include maltose-binding protein (MBP)/amylose, and the glutathione-S-transferase (GST)/glutathione tags, polyhistidine (His)/metal ions (*e.g.,* copper and cobalt), streptavidin/biotin (*e.g.,* N-ethyl-biotin), and antigen-antibody reactions (epitope) tags (*e.g.,* c-Myc tag/anti-c-Myc antibody, FLAG/ anti-FLAG antibody, and hemagglutinin (HA) tag/anti-HA antibody.

Methods in protein purification workflows, as well as other workflows, may vary widely but, in some instances, solid supports such as magnetic particles (*e.g*., magnetic beads) may be held in place by a magnetic field and then contacted with a protein binding partner. In other instances, supports such as magnetic particles (*e.g*., magnetic beads) may be contacted with a protein binding partner and then held in place by a magnetic field. In both such instances, the supports may be held in place and washed.

It may be desirable to release a protein from a support, for example, after a process by which the protein has been separated from other materials (*e.g*., cell debris). The process by which the protein is released from the support will be determined by the protein and/or the nature of the association between the protein and the support.

Protein release from a support may be mediated by the use of protease cleavage either within the protein or between the protein and an exogenously added tag. Exemplary proteases that may be used include, a rhinovirus 3C protease, a TVMV protease, a plum pox virus protease, turnip mosaic virus protease, tobacco etch virus (TEV) protease, thrombin, Factor Xa, and enteropeptidase.

One type of protein purification method that allows for both purification and release of the protein from supports uses biotin and biotin derivative (*e.g*., biotin, desthiobiotin, N-ethyl-biotin, etc.) in conjunction and biotin binding proteins.

Proteins may be biotinylated by a number of methods including chemical and enzymatical methods. Chemical protein biotinylation often results in nonspecific biotinylation of amine, carboxylic acid, and sulfhydryl groups. Enzymatic protein biotinylation may be designed to results in biotinylation of a specific groups within a protein. One example of an enzyme that may be used for protein biotinylation is the *E. coli* biotin holoenzyme synthetase, biotin ligase (BirA). This enzyme catalyzes transfer of biotin to an amino group of a specific lysine of the acetyl-CoA carboxylase biotin carboxyl carrier protein (BCCP) subunit.

In some instances, an antibody or other protein with ligand binding activity is associated with a support through a low affinity biotin derivative (*e.g*., desthiobiotin, N-ethyl-biotin, etc.)/biotin binding protein (*e.g*., avidin, streptavidin, neutravidin, etc.) association. Release of the low affinity biotin derivative from the biotin binding protein is mediated by competition with high affinity biotin (*e.g*., d-biotin).

Cell capture and release methods that may be used for protein purification, as well as for cells, viruses, virus like particles and other biological molecules, are those that use the CAPTURESELECT^{™} N-Ethyl Biotin (NEB) Anti-CD4 Conjugate (Thermo Fisher Scientific, cat. no. 7113762100) and CAPTURESELECT^{™} N-Ethyl Biotin (NEB) Anti-CD8 Conjugate (Thermo Fisher Scientific, cat. no. 7113772100). In this instance, a biotinylated anti-CD4 antibody or anti-CD8 antibody is bound to a magnetic bead through a biotin/streptavidin association. In summary, the methods set out in this product areas follow. First, anti-CD4 antibodies or anti-CD8 antibodies biotinylated with NEB are contacted with streptavidin coated magnetic beads. The bead are then contacted with CD4+ or CD8+ T cells under conditions that allows for binding of the T cells through an NEB/streptavidin association. The beads are then washed after a short incubation. In many instances, the beads will be held in place by a magnetic field during the washings. After washing, the beads are contacted with a release reagent containing d-biotin. The supernatant with the CD4+ or CD8+ T cells is then removed. In many instances, the beads will be held in place by a magnetic field during the washings. The result being a purified population of CD4+ T or CD8+ T cells with few or no beads present.

Method such as the above many be performed in a closed system and automated and performed in a closed system. With respect to automation, all reactions steps may be performed in one or more containers (*e.g*., bags) with tubes connected for the addition and removal of reagents. By way of example, bead processing assemblies and systems such as those set out herein may be used to hold the beads in place after addition and incubation of the beads with a release reagent.

Also provided herein are cell/virus surface display methods. Using yeast (*e.g., Saccharomyces cerevisiae*) surface display as an example, nucleic acid encoding a protein of interest, or portion thereof, may expressed as a fusion with an *S*. *cerevisiae* cell surface protein under conditions in which the protein of interest, or portion thereof, is present on the exterior of the yeast cell. One yeast gene that can be used for this purpose encodes the A-agglutinin-binding subunit (Aga2p) cell surface protein. The result being, after expression, that the protein of interest, or portion thereof, is located on the surface of the yeast cell as a component of a cell surface fusion protein. These cells are then contacted with a support to which a binding partner is bound, after which unbound yeast cells are removed by washing. Nucleic acid encoding potential proteins of interested may then be isolated cloned and/or sequenced. In many, the protein of interest will be an antibody that isolated from a library of antibodies with specificity to a support bound antigen.

Similar phage display methods may also be performed using bead processing assemblies and systems provided herein.

Bead processing assemblies and systems provided herein may also be used to purify viruses and virus like particles. Viruses and virus like particles purified using methods set out here may be enveloped on non-enveloped virus and virus like particles.

Viruses and virus like particles may be isolated by association with supports (*e.g*., magnetic beads). The manner by which viruses and virus like particles associate with supports may vary with the structures of the individual viruses and virus like particles. By way of example, adeno-associated virus (AAV) is non-enveloped and antibodies have been developed with binding affinity to AAV capsid proteins. Further, AAV capsid variations (serotypes) are known and result in different AAV serotype having different cell and tissue specificities.

One commercially available AAV capsid binding antibody is a VHH antibody referred to as AAVX antibody (*see* CAPTURESELECT^{™} Biotin Anti-AAVX Conjugate, Thermo Fisher Scientific, cat. no. 7103522500). The AAVX antibody is a single antibody that has binding affinity for multiple capsid serotypes. The AAVX antibody, as well as other antibodies with specificity for AAV capsid proteins, may be used to purify AAV virus like particles. Of course, other AAV antibodies may also be used, many of which have activity more directed to specific AAV capsids (*e.g*., CAPTURESELECT^{™} Biotin Anti-AAV8 Conjugate, cat. no. 7103382500; CAPTURESELECT^{™} Biotin Anti-AAV9 Conjugate, cat. no. 7103332500; etc.). In many such methods, compositions containing AAV virus like particles may be with an anti-AAV capsid antibody that is bound to a support (*e.g*., magnetic supports, such as beads) for a sufficient period of time to allow for binding of AAV particles to the support. In many instances, the support will be washed to separate the solid support from unbound materials, after which AAV particles will be released from the solid support. One reagent that may be used for AAV particle release is 50 mM citric acid, pH 3.0. Resulting solutions containing AAV particles may then be neutralized using, for example, using 100 mM Tris, pH 9.0.

Supports for purification of some AAV serotypes (*e.g*., AAV2 and AAV6) may also comprise heparin (*see* Auricchio et al., "Isolation of highly infectious and pure adeno-associated virus type 2 vectors with a single-step gravity-flow column", Hum. Gene Ther., 12:71-76 (2001)). Thus, supports (*e.g*., magnetic supports, such as beads) comprising heparin may be used in methods set out herein.

Enveloped viruses and virus like particles may also be purified using compositions, methods and devices provided herein. Enveloped viruses and virus like particles, as well as cells and exosomes, may be purified, for example, through the use of affinity agents. As part of such methods, enveloped viruses and virus like particles many be purified based upon association of a ligand with a protein present in the envelop of the virus and virus like particles that one seeks to purify. An example of such a method is set out in Mekkaoui et al., "Lentiviral Vector Purification Using Genetically Encoded Biotin Mimic in Packaging Cell", Mol. Ther. Methods Clin. Dev., 11:155-165 (2018). In this method, packaging cells were genetically engineered to express a biotin-mimicking peptide fused to CD8a amino acid sequences, referred to as cTag8. Lentiviral particles generated by budding acquire an envelope comprising the cTag8. Enveloped lentiviral particles may then by association with supports comprising a biotin binding partner (*e.g*., streptavidin). One advantage of such methods is that biotin-mimicking peptide may be used that have low affinity from the biotin binding partner used (*e.g.,* streptavidin, avidin, nitrated avidin, nitrated streptavidin) than biotin or biotin derivatives (*e.g*., desthiobiotin, N-ethyl-biotin, anti-biotin antibodies, etc.). Nitrated avidin and streptavidin are modified protein (*e.g*., with a nitro group added to a tyrosine) that allow for ligand release under milder conditions that their respective unmodified proteins (see Morag et al., "Immobilized nitro-avidin and nitro-streptavidin as reusable affinity matrices for application in avidin-biotin technology", Anal. Biochem., 243:257-263 (1996)). Thus, biotin and/or biotin derivatives may be used to release the lentiviral particles by competitive binding. Of course, such tags may also be used to purify molecules to which they are associated with (*e.g*., proteins).

Provided herein are instruments, compositions, methods and workflows for the purification of viruses, virus like particles and vesicles (*e.g*., exosomes). In many instances, such methods will involve contacting compositions (*e.g*., cell cultures, cell culture supernatants, conditioned cell culture media, cell lysates, etc.) with supports (*e.g*., magnetic supports, such as beads) to which viruses, virus like particles and vesicles (*e.g*., exosomes) will associate under conditions that allow for the viruses and/or virus like particles to associate with the supports, followed by washing of the supports, and then inducing dissociation of the viruses and/or virus like particles from the supports.

The viruses, virus like particles and/or vesicles (*e.g*., exosomes) may associate with the supports may be mediated in any number of ways, including ionic interactions and ligand/binding partner interactions such as antibody/antigen interaction.

As set out above, release of viruses, virus like particles and/or vesicles (*e.g*., exosomes) associated with supports may be mediated in a number of ways. These include inducing dissociation by altering the pH, the ionic strength, the ionic charge, the temperature (*e.g*., increasing the temperature), and solution polarity.

In many instances, the supports will be held in place by a magnetic field during the washing and after dissociation of the viruses, virus like particles and/or vesicles (*e.g*., exosomes) from the supports.

Extracellular vesicles (*e.g*., exosomes, microvesicles, apoptotic bodies, etc.) and liposomes may be purified using methods similar to those set out herein for enveloped virus and virus like particles.

Extracellular vesicles are replication deficient lipid bilayer particles that are released from almost all types of cell. Extracellular vesicles range in size from about 20 to as large as 10 microns or larger, although the majority of extracellular vesicles are smaller than 200 nm.

Exosomes are type of extracellular vesicle, typically 30 to 150 nm. Exosomes are believed to be released upon fusion of multivesicular bodies with the cell membrane by a large number of mammalian cells. A wide variety of molecules have been found to be associated with extracellular vesicles and extracellular vesicles, such as exosomes, are believed to transport nucleic acids, proteins, and lipids for purposes such as intercellular communication and activation of target cell signaling pathways.

In cancers, extracellular vesicles, such as exosomes, are believed to participate in processes such as the regulation of immune responses and the promotion of angiogenesis. Extracellular vesicles, such as exosomes, derived from patient samples (*e.g*., blood) can be used for non-invasive early detection and diagnosis of cancers. Further extracellular vesicles, such as exosomes, may be used as natural drug delivery vehicles for, for example, cancer therapy.

Extracellular vesicles, such as exosomes, may be purified by association (*e.g*., non-affinity or affinity association) with supports (*e.g*., magnetic supports, such as beads). One example of a type of non-affinity exosome purification method is through the use of ion exchange groups. Anion exchange groups, similar to those used for nucleic acid molecule purification may be used for extracellular vesicles (*e.g*., exosomes) purification.

It has had been found that the DYNABEADS^{™} Intact Virus Enrichment (optimized for SARS-CoV-2) (Thermo Fisher Scientific, cat no. 10700D) commercially available product can be used to purify exosomes. The DYNABEADS^{™} in this product are 1 µm anion exchange magnetic beads that bind negatively charged vesicles and molecules. As shown by this example, many of the purification methods set out herein may be used to purify different types of biological materials (*e.g*., nucleic acid molecules, proteins, virus like particles, cells, etc.).

A number of affinity based methods have been developed for the purification of exosomes. One type of method uses antibodies targeting tetraspanin proteins (*e.g*., CD9, CD63, and CD81), which are often enriched on exosome surfaces (see Liangsupree et al., "Modern isolation and separation techniques for extracellular vesicles ", J. of Chromatography A 1636:461773 (2021)). Two advantage of immunoaffinity purification of exosomes are (1) purification selectivity and (2) the ability to purify exosomes derived from different cell types. For example, epithelial cell adhesion molecule (EpCAM CD326) has been found to be highly expressed in certain cancer cells, including lung, stomach, colon, prostate, and ovarian cancer cells.

In some instances, affinity mediated purification of extracellular vesicles (*e.g*., exosomes) may be mediated by affinity reagents (*e.g*., antibodies) having binding affinity for one or more of the following proteins: CD1 (*e.g.,* CD1a, CD1b, CD1c, CD1d, and CD1e), CD2, CD3 (*e.g.,* CD3d, CD3e, and CD3g), CD4, CD5, CD6, CD7, CD8 (*e.g.,* CD8a and CD8b), CD14, CD16, CD19, CD21 (Complement Receptor 2), CD23, CD24, CD27, CD28, CD29 (integrin beta 1), CD30, CD42 (*e.g.,* CD42a, CD42b, CD42c, and CD42d), CD44, CD45, CD51, CD63, CD79 (*e.g.,* CD79a and CD79b), CD80, CD81, CD86, CD94 (KLRD1), CD95, CD97, CD114 (G-CSF receptor), CD115 (CSF1 receptor), CD116, CD117, CD118, CD119, CD120 (*e.g.,* CD120a and CD120b), CD121 (*e.g.,* CD121a and CD121b), CD122, CD123, CD124, CD125, CD126, CD127, CD128, CD130, CD131, CD132, CD134, CD135, CD137, CD138, CD140 (*e.g.,* CD140a and CD140b), CD150, CD152, CD153, CD154, CD157, CD158 (*e.g.,* CD158a. CD158b1, CD158b2, CD158b, CD158c, CD158d, CD158e1, CD158e2, CD158f1, CD158f2, CD158g, CD158h, CD158i, CD158j, and CD158k), CD160, CD161, CD 167 (*e.g.,* CD167a and CD 167b), CD172 (*e.g.,* CD172a, CD172b, and CD172g), CD179 (*e.g.,* CD179a, CD179b, CD179c, and CD179d), CD181, CD182, CD183, CD191, CD194, CD200, CD202b, CD212, CD215, CD217, CD218 (*e.g.,* CD218a and 218b), CD220, CD221, CD222, CD223, CD226, CD227, CD235a (Gly A), CD244, CD247 (CD3-Zeta), CD252, CD253, CD254, CD256 (APRIL), CD257 (BAFF), CD258, CD261, CD262, CD263, CD264, CD265, CD266, CD267, CD268, CD269, CD263, CD264, CD265, CD266, CD267, CD272, CD273, CD274, CD275, CD276, CD278 (ICOS), CD279, CD304, CD305, CD314, CD326, CD331, CD332, CD333, CD335, CD336, CD337, CD357, CD358, CD360, and CD366.

An example of an affinity purification reagent that may be used to purify exosomes is the Exosome-Human CD81 Isolation Reagent (from cell culture) product available from Thermo Fisher Scientific (cat. no. 10616D).

Provided herein are compositions and methods for the generation and purification of extracellular vesicles (*e.g*., exosomes) from cells grown in culture. In some such methods, cells are grown in culture for sufficient period of time for extracellular vesicles (*e.g*., exosomes) formation, then the extracellular vesicles (*e.g*., exosomes) are separated from the cells (*e.g.,* by centrifugation). Once separated from cells, the extracellular vesicles (*e.g*., exosomes) may then be separated from surrounding materials (*e.g.,* proteins, culture media, etc.) by association with supports (*e.g.,* magnetic beads) and processing using bead processing assemblies and systems set out herein. Of course, bead processing assemblies and systems may be designed to automate workflows such as those set out above or at least a number of steps of such workflows.

In some instances, the cells used for the generation and purification of extracellular vesicles (*e.g*., exosomes) will contain or express one or more molecule that is included in or a component of the extracellular vesicles (*e.g*., exosomes). Such cells may (1) naturally produce the molecule, (2) be exposed to the molecule under conditions in which the molecule is taken up by the cells, or (3) be engineered to produce the molecule (*e.g.,* a protein, such as a chimeric antigen receptor (CAR).

In some instances, provided herein are methods for the generation and purification of exosomes for cells grown in culture, where the exosomes comprise a therapeutic agent. One example of such a method is where the cells are exposed to a therapeutic agent that is taken up by the cells, followed by purification of exosomes generated by these cells.

Extracellular vesicles (*e.g*., exosomes) may also be loaded with molecules (*e.g*., anti-cancer drugs such as paclitaxel, siRNA, proteins, etc.) by electroporation (*see, e.g.,* Zhou et al., "Bone marrow mesenchymal stem cells-derived exosomes for penetrating and targeted chemotherapy of pancreatic cancer", Acta Pharm. Sin. B., 10:1563-1575 (2020)). Extracellular vesicles (*e.g*., exosomes) may also be loaded with molecules by transfection (*e.g*., lipid mediated transfection). Further, exosomes may be loaded before or after support (*e.g*., magnetic supports, such as beads) mediated purification.

Provided herein are methods for the purification of extracellular vesicles (*e.g*., exosomes) obtained from cell engineering to contain or comprise a specific biological molecule. In some instances, the biological may be a genome editing complex or one or more components (*e.g*., a Cas9 protein, a guide RNA molecule/Cas9 protein complex, etc.) comprising and/or nucleic acid encoding such a complex (*e.g*., DNA or RNA encoding a Cas9 protein and/or a guide RNA molecule). In some instances, the biological may be an RNA molecule (*e.g.,* an siRNA molecule, a microRNA molecule, a mRNA molecule, etc.).

One therapeutic application of extracellular vesicles (*e.g*., exosomes) is in cancer treatment. It has been shown that exosomes released by chimeric antigen receptor T cells (CAR-T cells) release exosomes that carry CARs on their surfaces. These exosomes have been found to contain cytotoxic molecules and cytotoxic activity against tumors (Fu et al., "CAR exosomes derived from effector CAR-T cells have potent antitumour effects and low toxicity", Nat. Commun., 10:4355 (2019) and Yang et al., "The exosomes derived from CAR-T cell efficiently target mesothelin and reduce triple-negative breast cancer growth", Cell. Immunol., 360:104262 (2021)). Further, such exosomes are believed to induce fewer side effects in individuals (*e.g*., cancer patients) to which they are administered compared to CAR-T cells.

Provided herein, in part, are methods for the purification of extracellular vesicles (*e.g*., exosomes) generated by T cells. Such methods may comprise maintaining T cells in culture media for time period sufficient for the generation of extracellular vesicles (*e.g*., exosomes), followed by purification on these extracellular vesicles (*e.g.*, exosomes). In many instances, the extracellular vesicles (*e.g*., exosomes) generated by the T cells will be engineered to express CAR prior to generation of the extracellular vesicles (*e.g*., exosomes). In some instances, such extracellular vesicles (*e.g*., exosomes) will be administered to an individual (*e.g*., a cancer patient).

In some instances, instruments, compositions, methods and/or workflows may involve the depletion of a sample of extracellular vesicles (*e.g.,* exosomes). One example such depletion is where a sample contains exosomes derived from red blood cells. In such instances, a support with binding affinity for CD235a (Gly A) may be used to remove red blood cells and/or extracellular vesicles (*e.g*., exosomes) formed by red blood cells from the sample. A commercially available product that may be used in such workflows is CD235a (Glycophorin A) MicroBeads, Human (Miltenyi, cat. no. 130-050-501).

Method such as the above many be performed in a closed system and automated and performed in a closed system. With respect to automation, all reactions steps may be performed in one or more containers (*e.g*., bags) with tubes connected for the addition and removal of reagents. By way of example, bead processing assemblies and systems such as those set out herein may be used to hold the beads in place after conditions within the bag have been changed to induce release of viruses and/or virus like particles from the support.

### Examples

The subject matter set out herein can be further understood by reference to the following examples, which are provided by way of illustration and are not meant to be limiting. Further, portions of the following experiments were performing using the instruments and materials exemplified in the Figures. For example, the below T cell isolation and activation performed under the conditions of rocking was achieved using bead processing apparatus 22 with consumable kit 170A (Figures 9 and 11), as previously discussed. Likewise, the below discussed debeading was achieved using bead processing apparatus 22 with consumable kit 170B (Figures 9 and 35), as previously discussed.

### Example 1: T Cell Isolation and Viability Assessment

250x10⁶ CD3+ cells were incubated with CTS^{™} DYNABEADS^{™} CD3/CD28 (Thermo Fisher Scientific, cat. no. 40203D) for 30 minutes at room temperature in PBS/1% human serum albumin. Cell density during cell-bead incubation was 10x10⁶ CD3⁺ cells/mL, and the bead to cell ratio was 3:1. During this time, cells and beads were mixed with rocking (10 degrees, 5 RPMs). The same mixing procedures were used to wash cells after CD3⁺ isolation to remove unbound cells.

To analyze CD3⁺ depletion from PBMC for isolation efficiency, samples were collected from the PBMCs prior to isolation and from the negative fraction, following the above approaches. Samples were stained with a fluorescently labeled anti-CD3 antibody and the number of CD3⁺ cells in the input and negative fraction was analyzed by flow cytometry. The isolation efficiencies (depletion of CD3⁺ cells from the negative fraction) were calculated by subtracting the fraction of CD3⁺ cells in the negative fraction from 1 and multiplied by 100%.

The average isolation efficiency from two replicates of an experimental run was 93% (Standard Deviation (SD) 0.47)

After CD3⁺ cell isolation, the T cells were transferred to a bioreactor and expanded for 6 days in CTS^{™} OPTMIZER^{™} T Cell Expansion SFM (Thermo Fisher Scientific, cat. no. A1048501), supplemented with 100 IU/mL IL-2 (Thermo Fisher Scientific, cat. no. PHC0021). To assess viability, a sample (2 ml) was aseptically collected from the bioreactor, resuspended, debeaded, and analyzed for percent viability using SYTOX^{™} Blue Dead Cell Stain (Thermo Fisher Scientific, cat. no. S34857) and flow cytometry.

| **Table 1: Cell Viability** | | |
|---|---|---|
| **Conditions** | **Avg (SD)** | **Avg (SD)** |
| | **Day 1** | **Day 6** |
| **Rocking** | 90.50% (0.56) | 88.40% (1.31) |

### Example 2: T Cell Purity Assessment

T cell purity on day 1 and day 6 of expansion was assessed by flow cytometry.

T cells were stained using an anti-CD3⁺ antibody, monocytes were stained using an anti-CD14 antibody, B-cells were stained using an anti-CD19 antibody, and natural killer (NK) NK cells were stained using an anti-CD56 antibody. All antibodies were directly conjugated to a fluorochrome, allowing direct detection by flow cytometry.

Data obtained from two replicates of an experimental run are set out below in Table 2. These data were generated using cells that had been incubated under rocking and shaking conditions.

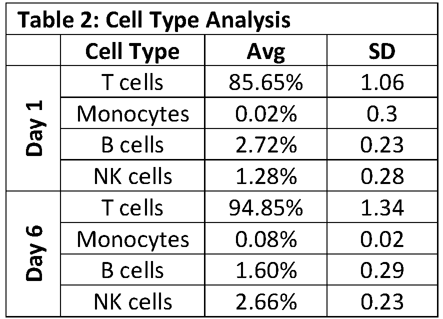

### Example 3: Debeading Effectiveness

After 6 days of expansion, cells were debeaded by a continuous flow approach with a 46 continuous flow rate using a bag similar to that shown in FIG. 34 and the flow system set out in FIG. 35. Cells from the bioreactor are passed over the magnet to collect magnetic beads, while allowing cells to pass through. Debeaded cells are collected in the output bag.

Following bead removal, sample containing 3x10⁶ CD3⁺ cells are removed from the output bags, lysed, then concentrated to a small volume (20 µL). Beads in the entire lysates are counted to estimate the number of beads per 3x10⁶ CD3⁺ cells. Beads are counting using a light microscope and KOVA^{™} Glasstic slide counting chamber (Fisher Scientific, cat no. 22-270141), according to the manufacturer's protocol. Resulting data from two replicates of an experimental run are set out in Table 3.

| **Table 3** | | | | | | |
|---|---|---|---|---|---|---|
| **Flow Rate (ml/min)** | **Post Lysis/ Concentration Vol** | **Total Beads** | **Grids Counted** | **Beads per µL** | **Beads per 3x10⁶ Lysed Cells** | **Avg 2 Replicates (SD)** |
| 46 | 18 µL | 2 | 162 | 1 | 20 | 20 (0) |

The number of beads present prior to the debeading process is estimated to be 9x10⁶, based upon the beads being mixed with cell as a 3:1 ratio. Thus, at a flow rate of 46 ml/min, it is estimated that over 99.99% of the beads were removed in the debeading process. It is estimated that, on average, of an estimated 9,000,000 beads originally present, only 1 bead per 450,000 co-localized with the debeaded cells.

### Example 4: Solid phase in vitro transcription (IVT)

### Part 1: IVT Template/Magnetic Bead Complex Production

### Materials and Methods:

DYNABEADS^{™} MYONE^{™} Streptavidin C1 (Thermo Fisher Scientific, cat. no. 65002), 2xStreptavidin binding and washing buffer (10 mM Tris-HCl (pH 7.5) 1 mM EDTA 2 M NaCl), nuclease free water, 10mM Tris (pH 7-8), Tris-EDTA (pH 7-8) (TE), DYNAMAG^{™}-2 Magnet (Thermo Fisher Scientific, cat. no. 12321D), Thermal Mixer (Thermo Fisher Scientific, cat. no. 12321D).

### Immobilization of biotinylated PCR-product (template) to DYNABEADS^{™} Streptavidin

### Preparation of biotinylated PCR-product:

A biotinylated PCR product containing the T7-promoter upstream of the UTR and ORF, optionally with a defined polyA-tail in the end, was prepared. The forward primer was biotinylated and had a distance to the T7 promoter of at least 50-100 base pairs. The PCR product was then diluted to 20 ng/µL in 1x Streptavidin binding and washing buffer (the 2x DYNABEADS^{™} Streptavidin binding and washing buffer was diluted 1:1 in nuclease free water prior to use). Reaction conditions were as set out below.

| **Dynabeads^{™} MyOne^{™} Streptavidin C1** | **Biotinylated PCR product 1µg (in 1xStreptavidin binding and washing buffer)** | **Final NA amount/bead** | **Final bead concentration** |
|---|---|---|---|
| 100 µl = 1 mg | 50 µl of 20 ng/µl = 1 µg | 1 µg/mg | 10 mg/mL |

**Preparation of Streptavidin beads is performed as follows:**
1. The DYNABEADS^{™} MyOne^{™} Streptavidin C1 beads were suspended in a tube by vortexing
2. The tube with the Dynabeads^{™} MyOne^{™} Streptavidin C1 beads was placed on a roller for at least 20 minutes
3. 100µL (1 mg) of resuspended DYNABEADS^{™} MyOne^{™} Streptavidin C1 beads was transferred to an RNase free tube
4. The tube with the resuspended DYNABEADS^{™} MyOne^{™} Streptavidin C1 beads was placed on a DYNAMAG^{™}-2 Magnet for 1 minute, then remove the supernatant
5. The beads were washed once in 100µL 1x Streptavidin binding and washing buffer, by resuspension using a pipette or brief vortexing
6. The tube was placed on a DYNAMAG^{™}-2 Magnet for 1 minute
7. The supernatant was removed from the tube
8. The beads were suspended in 50µL Streptavidin buffer

### DNA Immobilization:

1. 50 µL (1 µg diluted in 1x Streptavidin binding and washing buffer) of biotinylated PCR-product was added to the washed and resuspended DYNABEADS^{™} MYONE^{™} Streptavidin C1 beads
2. The tube was incubated for 30 minutes at room temperature (RT) on a thermal mixer 1500 RPM
3. The tube was placed on DYNAMAG^{™}-2 Magnet for 1 minute, the supernatant was then removed (Optional: Quantify how much unbound DNA remains in this supernatant using the QUBIT^{™} 1x dsDNA HS Assay Kit (Thermo Fisher Scientific, cat. no. Q33230))
4. The DNA-bead complex was washed four times by resuspension in 100 µL TE-buffer (pH8), using a pipette or brief vortexing
5. The tube was placed on DYNAMAG^{™}-2 Magnet for 1 minute, then discard the supernatant
6. The washing was repeated three times, for a total of 4 washes. For last wash step, the supernatant was not discarded
7. The DYNABEADS^{™} MYONE^{™} Streptavidin C1 beads with immobilized template were retained for IVT as set out below.

### Part 2: Immobilized Template IVT

### Materials and Methods:

MEGASCRIPT^{™} T7 Transcription Kit (Thermo Fisher Scientific, cat. no. AMB1334), 10 mM Tris HCl pH 8, Streptavidin buffer (5 mM Tris-HCl (pH 7.5) 0.5 mM EDTA 1 M NaCl), DYNABEADS^{™} MYONE^{™} Carboxylic Acid beads (Thermo Fisher Scientific, cat. no. 65012), nuclease free water

Immobilized IVT Template: 1 mg DYNABEADS^{™} MYONE^{™} Streptavidin C1beads with 1 µg biotinylated DNA template immobilized (from above step 6/7)

IVT Reaction Mixture: MEGASCRIPT^{™} kit reagents scaled up to 100 µL reaction set out below:

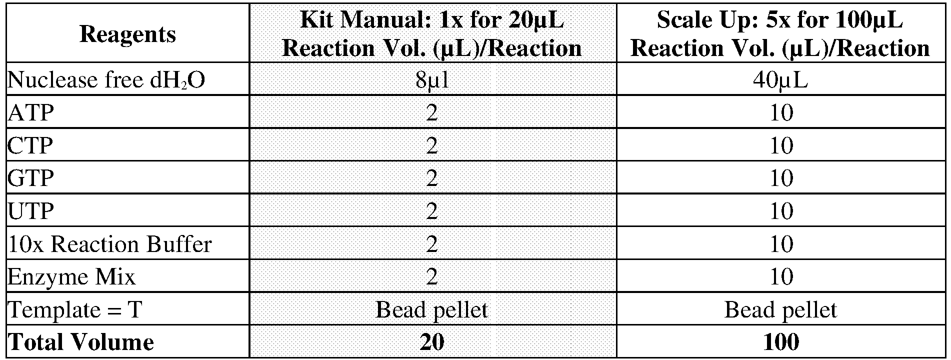

1. The tube with the Immobilized IVT Template was placed on the DYNAMAG^{™}-2 Magnet and discard the supernatant (step 7 above)
2. The Immobilized IVT Template was washed by resuspension in 200µL 10 mM Tris-HCl, pH 8
3. The tube with the Immobilized IVT Template was placed on the DYNAMAG^{™}-2 Magnet and discard the supernatant
4. The Immobilized IVT Template was resuspended in 100µL of MEGASCRIPT^{™} reaction mix
5. The tube was incubated for 1-3 hours (100µL) at 37°C on a thermal mixer at 1500 RPM
6. The tube with the Immobilized IVT Template was placed on the DYNAMAG^{™}-2 Magnet and the supernatant containing the *in vitro* transcript was transferred to a new, RNase free tube
7. The tube with the supernatant was placed on ice or freeze at -70°C for future use
8. Optionally, the mRNA concentration is then measured using the QUBIT^{™} RNA HS Assay Kit (Thermo Fisher Scientific, cat. no. Q32852). A 100 µL, 2 hour reaction typically yields about 4µg/µL mRNA, giving up to 400µg per reaction.

### mRNA Cleanup

mRNA capture by precipitation onto DYNABEADS^{™} MYONE^{™} Carboxylic Acid beads is performed as follows:
1. DYNABEADS^{™} MYONE^{™} Carboxylic Acid beads were mixed by vortexing the beads for 10 seconds, then placed on a roller for 20 minutes at room temperature (RT)
2. 30 µL (300 µg (concentration: 10 mg/mL)) of bead suspension was added into each clean nuclease free tubes
3. The tubes was placed on the DYNAMAG^{™}-2 Magnet until the solution looks clear (approximately 30-60 seconds) and discard the supernatant
4. The DYNABEADS^{™} MYONE^{™} Carboxylic Acid beads were washed once in 100µL nuclease free water and place on DYNAMAG^{™}-2 Magnet, the supernatant was discarded
5. 100 µL mRNA solution was added to the tubes, containing up to 150 µg RNA, and mixed until the beads are well resuspended
6. 200µL of 1.5X RNA Binding Buffer (RBB) (Thermo Fisher Scientific, cat. no. 37035D) was added and mix by pipetting, until a homogenous suspension formed
7. 10 minutes of incubation on a thermal mixer 1000 rpm room temperature was then done
8. The tube was placed on the DYNAMAG^{™}-2 Magnet until the solution looks clear (approximately 2-3 minutes, since the solution is quite viscous) and discard the supernatant
9. 500 µL of WB solution (70% EtOH in nuclease free water) was added and the beads were resuspended by vortexing
10. The tube was placed on the DYNAMAG^{™}-2 Magnet until the solution looks clear (approximately 30-60 seconds) and the supernatant was discarded
11. Steps 9-10 were repeated twice, for a total of 3 washes, then all residual WB solution was removed
12. The beads were dried at RT for 10 minutes while on the DYNAMAG^{™}-2 Magnet
13. The tube was removed from contact with the DYNAMAG^{™}-2 Magnet and 100 µL of Elution Buffer (TE buffer pH 7-8, 10mM Tris HCl pH 7-8) or nuclease free water was added, followed by resuspension of the beads by pipetting and incubation at 65°C for 5 minutes on a Thermal mixer at 1000 rpm
14. After incubation, optionally, centrifuge the tubes for a short period of time collect liquid from the lid
15. The tube was placed on the DYNAMAG^{™}-2 Magnet until the solution appeared clear (approximately 30-60 seconds)
16. The supernatant was collected and transferred to clean nuclease free tubes and place on ice or freeze for future use

### Example 5: Large Scale mRNA Production and Purification

**Part 1. Template immobilization on DYNABEADS^{™} MYONE^{™} Streptavidin C1 (Thermo Fisher Scientific, cat. no. 65002), beads:**
For initial set up, load the buffers and reagents to in a glass bioreactor. Bags may also be used Thoroughly resuspend DYNABEADS^{™} MYONE^{™} Streptavidin C1 prior to the transfer to reactor. Vortex the beads until they are properly resuspended and place on a roller for ≥ 20 minutes. Also, dilute 10 mg of biotinylated template DNA/PCR-product in 1X Streptavidin Binding and washing Buffer (5 mM Tris-HCl (pH 7.5) 0.5 mM EDTA 1 M NaCl) up to a total volume of 0.5 liters (L). If bags are used, then the circuit of tubing and bags with a reaction bag in the main compartment and a large waste disposal bag may be welded together.

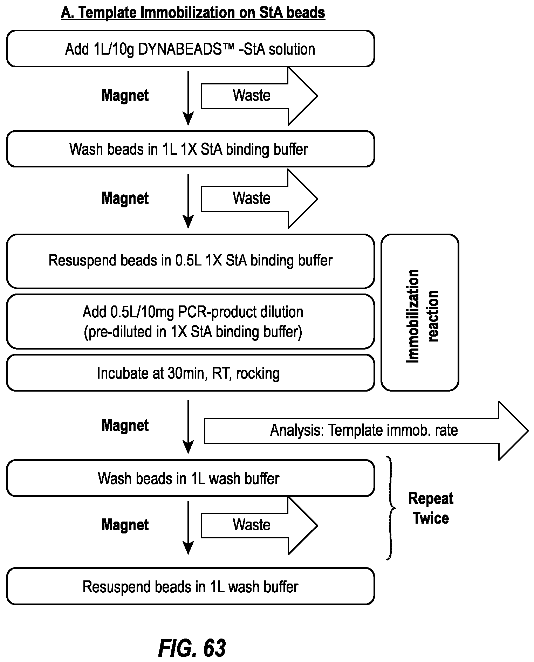

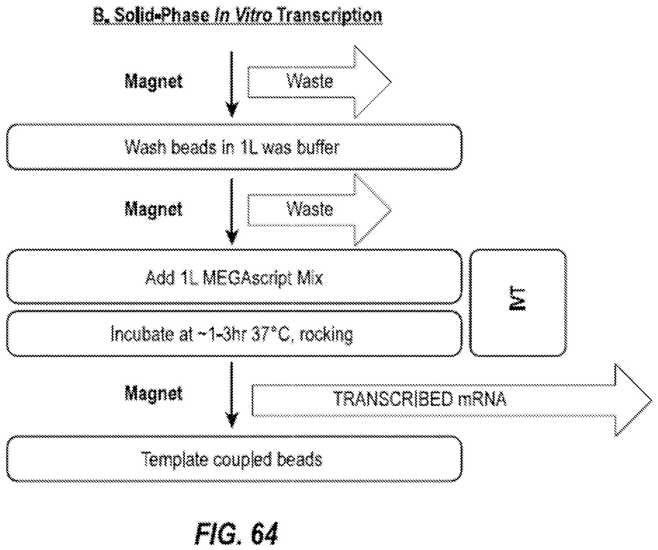

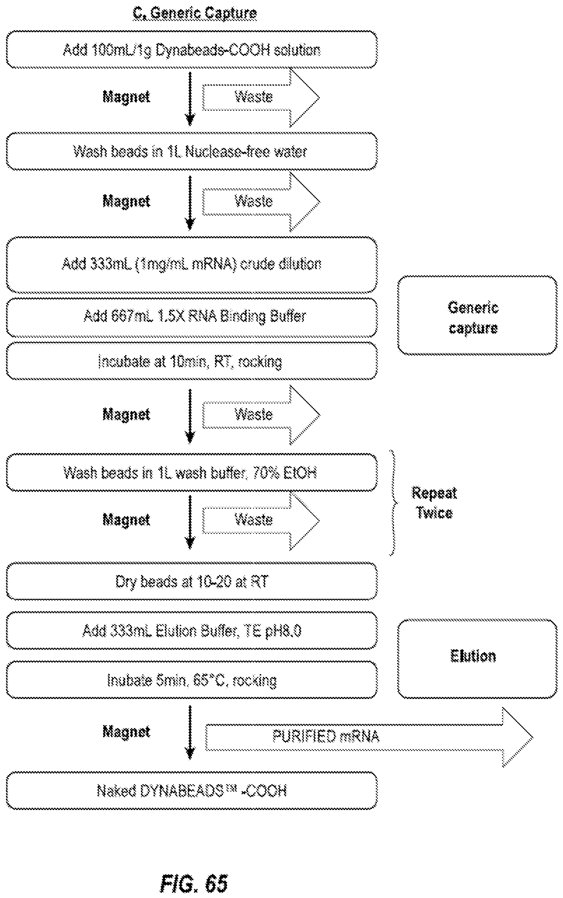

Outlines of the workflows set out in this example are shown in Figures 63-65.
1. Transfer 10g DYNABEADS^{™} MYONE^{™} C1 Streptavidin beads (1 liter, 10 mg/mL) to the reaction bag.
2. Apply magnet until the supernatant is completely translucent and transfer the supernatant to the waste bag.
3. Wash: Remove the magnet and transfer 1 liter of 1X Streptavidin Binding Buffer and to the reaction bag and mix 1 minute with gentle rocking and add a small volume of air to aid with the mixing.
4. Apply the magnet until the supernatant is completely translucent and transfer the supernatant to the waste bag.
5. Remove the magnet and transfer 0.5 liter of 1X Streptavidin Binding Buffer to the reaction bag and mix 1 minute with gentle rocking and add a small volume of air to aid with the mixing.
6. Transfer 0.5 liters of 10 mg biotinylated template DNA dilution (pre-diluted in 1X Streptavidin Binding Buffer).
7. Incubate for 30 minutes at room temperature with thorough rocking.
8. Apply the magnet until the supernatant is completely translucent and transfer the supernatant to a collection bag and store at 4°C. The supernatant can be used to quantify the degree of biotinylated template DNA immobilized on DYNABEADS ^{™} M-280 Streptavidin beads.
9. Wash: Remove the magnet and transfer 1 liter of 1X Streptavidin binding and washing buffer and to the reaction bag and mix 1 minute with gentle rocking and add a small volume of air to aid with the mixing.
10. Apply magnet until the supernatant is completely translucent and transfer the supernatant to the waste bag.
11. Repeat step 9-10 three more times (a total of 4 washes).
12. Remove the magnet and resuspend the bead-template complex in 1 liter of Wash Buffer and add a small volume of air to aid with the mixing.

### Part 2. Solid-phase In Vitro Transcription:

Load the buffers and reagents to bags and prepare the MEGAscript MIX and keep on ice prior to the transfer to bag. MEGASCRIPT^{™} kit reagents are scaled up to using the ratios of components set out in Example 4.
1. Apply magnet until the supernatant is completely translucent and transfer the supernatant to the waste bag.
2. Wash: Remove the magnet and transfer 1 liter of Wash Buffer and to the reaction bag and mix 1 minute with gentle rocking and add a small volume of air to aid with the mixing.
3. Apply magnet until the supernatant is completely translucent and transfer the supernatant to the waste bag.
4. Remove the magnet and transfer 1 liter of MEGASCRIPT^{™} reaction mix to the reaction bag.
5. Incubate for at 37°C temperature with thorough rocking and add a small volume of air to aid with the mixing until the target concentration of transcribed mRNA is attained.
6. Apply magnet until the supernatant is completely translucent and transfer the transcribed mRNA to a collection bag.
7. The template coupled beads can be reused multiple times: Repeat steps 3-6 up to 5 additional times.

### Part 3. Generic Capture, mRNA purification with DYNABEADS^{™} MYONE^{™} Carboxylic Acid beads:

Load the buffers and reagents to bags, thoroughly resuspend DYNABEADS^{™} MYONE^{™} Carboxylic Acid beads (Thermo Fisher Scientific, cat. no. 65012) prior to the transfer to bags. Vortex the beads until they are properly resuspended and place on a roller for ≥ 20 minutes. Dilute 333 mg of IVT crude mRNA in TE buffer of 10 mM Tris Buffer up to a total volume of 333 mL. Weld together the circuit of tubing and bags with a large waste disposal bag
1. Transfer 1g DYNABEADS^{™} MYONE^{™} Carboxylic Acid beads (100 mL, 10 mg/mL) to the reaction bag.
2. Apply magnet until the supernatant is completely translucent and transfer the supernatant to the waste bag.
3. Wash: Remove the magnet and transfer 1 liter of Nuclease-free Water and to the reaction bag and mix 1 minute with gentle rocking and add a small volume of air to aid with the mixing.
4. Apply magnet until the supernatant is completely translucent and transfer the supernatant to the waste bag.
5. Remove the magnet and transfer 333 mL, 333mg IVT crude mRNA mix (1 mg mRNA/mL dilution) to the reaction bag and mix 1 minute with thorough rocking and add a small volume of air to aid with the mixing.
6. Transfer 667 mL of 1.5X RNA Binding Buffer to the reaction bag. Apply a high rpm setting on the pump to ensure that the Binding Buffer is transferred as quickly as possible to the reaction bag to ensure optimal precipitation of mRNA onto DYNABEADS^{™} MYONE^{™} Carboxylic Acid beads.
7. Incubate for 10 minutes at room temperature with thorough rocking and add a small volume of air to aid with the mixing.
8. Apply magnet until the supernatant is completely translucent and transfer the supernatant to the waste bag.
9. Wash: Remove the magnet and transfer 1 liter of Wash Buffer and to the reaction bag and mix 1 minute with gentle rocking and add a small volume of air to aid with the mixing.
10. Apply magnet until the supernatant is completely translucent and transfer the supernatant to the waste bag.
11. Repeat step 8-9 two more times (a total of 3 washes).
12. Dry beads for 10-20 minutes at room temperature until the beads are completely dry. Pump filtered air through the reaction bag to aid with the drying process.
13. Transfer 333 mL of Elution Buffer to the reaction bag.
14. Incubate for 5 minutes at 65°C room temperature with thorough rocking and add a small volume of air to aid with the mixing.
15. Apply magnet until the supernatant is completely translucent and transfer the purified mRNA to a collection bag.

It will also be appreciated that systems, processes, and/or products according to certain embodiments of the present disclosure may include, incorporate, or otherwise comprise properties features (*e.g*., components, members, elements, parts, and/or portions) described in other embodiments disclosed and/or described herein. Accordingly, the various features of certain embodiments can be compatible with, combined with, included in, and/or incorporated into other embodiments of the present disclosure. Thus, disclosure of certain features relative to a specific embodiment of the present disclosure should not be construed as limiting application or inclusion of said features to the specific embodiment. Rather, it will be appreciated that other embodiments can also include said features without necessarily departing from the scope of the present disclosure.

Moreover, unless a feature is described as requiring another feature in combination therewith, any feature herein may be combined with any other feature of a same or different embodiment disclosed herein. Furthermore, various well-known aspects of illustrative systems, processes, products, and the like are not described herein in particular detail in order to avoid obscuring aspects of the example embodiments. Such aspects are, however, also contemplated herein.

The described embodiments are to be considered in all respects only as illustrative and not restrictive. The scope of the invention is, therefore, indicated by the appended claims rather than by the foregoing description. While certain embodiments and details have been included herein and in the attached disclosure for purposes of illustrating embodiments of the present disclosure, it will be apparent to those skilled in the art that various changes in the methods, products, devices, and apparatus disclosed herein may be made without departing from the scope of the disclosure.

## Claims

1. A method for separating biological cells of a first cell type from biological cells of a second cell type using a bead processing system, the method comprising:
a. contacting a sample comprising the biological cells of the first cell type and the second cell type with magnetic beads (179) so that the magnetic beads attach to the biological cells of the first cell type through an antibody linkage, the sample and magnetic beads being disposed within a processing bag (206) resting on a platform (290);
b. raising a magnet (299) relative to the platform so that the magnet produces a magnetic field that securely fixes in place the magnetic beads relative to the platform, the magnetic beads having the biological cells of the first cell type attached thereto;
c. passing a fluid through the sample and the magnetic beads while the magnetic field is applied thereto, the fluid passing with a force sufficiently strong to wash away the biological cells of the second cell type from the biological cells of the first cell type; and
d. lowering the magnet relative to the platform so that the magnetic beads having the biological cells of the first cell type attached thereto are no longer securely fixed in place relative to the platform by the magnetic field of the magnet.

2. The method as recited in claim 1, wherein the antibody has a binding affinity for a cell surface receptor, the cell surface receptor being CD56.

3. The method as recited in claim 1, wherein the first cell type is natural killer (NK) cells.

4. The method as recited in claim 1, wherein the antibody has binding affinity for a protein selected from the group consisting of CD3, CD4, CD5, CD6, CD8, CD25, CD27, CD28, CD137, and CD278.

5. The method as recited in claim 4, wherein the antibody has binding affinity for a protein comprising CD3 or CD28.

6. The method as recited in claim 5, wherein the first cell type is T cells.

7. The method as recited in claim 1, further comprising: detaching at least a majority of the biological cells of first cell type from the magnetic beads; applying a magnetic field to the magnetic beads so as to securely fix the magnetic beads in place; and passing a fluid through the magnetic beads and the separated biological cells of first cell type while the magnetic field is being applied, the fluid passing with a force sufficiently strong to wash away at least a majority of the biological cells of the first cell type from the magnetic beads while at least a majority of the magnetic beads remain fixed in place by the magnetic field.

8. The method as recited in claim 7, wherein greater than 95% of the magnetic beads are retained in place by the magnetic field while the at least a majority of the biological cells of the first cell type are washed away from the magnetic beads.

9. The method as recited in claim 8, wherein greater than 95% of the magnetic beads are separated from the biological cells of the first cell type.

10. The method as recited in claim 1, wherein greater than 95% of the magnetic beads detached from the biological cells of first cell type are separated from the biological cells of first cell type.

11. The method as recited in claim 1, wherein the magnetic beads are from about 0.5 µm to about 3 µm in diameter.

12. The method as recited in claim 1, further comprising tilting the platform on which the processing bag is resting in a first direction relative to horizontal, the magnetic field being applied to the magnetic beads within the processing bag while the platform is tilted in the first direction.

13. The method as recited in claim 12, wherein the magnet is raised after the platform is tilted in the first direction.

14. The method as recited in claim 12, further comprising injecting a gas or a liquid through a port (416) of the processing bag while the platform is tilted in the first direction.

15. The method as recited in claim 12, further comprising:
tilting the platform in a second direction opposite to the first direction so that the platform on which the processing bag is resting is angled relative to horizontal; and
withdrawing liquid from the processing bag while the platform is tilted in the second direction.

## Patentansprüche

1. Verfahren zur Trennung biologischer Zellen eines ersten Zelltyps von biologischen Zellen eines zweiten Zelltyps unter Verwendung eines Bead-Verarbeitungssystems, wobei das Verfahren Folgendes umfasst:
a. Inkontaktbringen einer Probe, die die biologischen Zellen des ersten Zelltyps und des zweiten Zelltyps umfasst, mit magnetischen Beads (179), sodass die magnetischen Beads über eine Antikörperbindung an die biologischen Zellen des ersten Zelltyps binden und die Probe und die magnetischen Beads in einem Verarbeitungsbeutel (206) entsorgt werden, der auf einer Plattform (290) ruht;
b. Anheben eines Magnets (299) relativ zur Plattform, so dass der Magnet ein Magnetfeld erzeugt, das die magnetischen Beads relativ zur Plattform sicher an Ort und Stelle fixiert, wobei die biologischen Zellen des ersten Zelltyps an die magnetischen Beads gebunden sind;
c. Durchleiten einer Flüssigkeit durch die Probe und die magnetischen Beads, während das Magnetfeld darauf angewendet wird, wobei die Flüssigkeit mit einer ausreichend starken Kraft durchströmt, um die biologischen Zellen des zweiten Zelltyps von den biologischen Zellen des ersten Zelltyps wegzuspülen; und
d. Absenken des Magneten relativ zur Plattform, sodass die magnetischen Beads, an denen die biologischen Zellen des ersten Zelltyps haften, nicht mehr durch das Magnetfeld des Magneten relativ zur Plattform fest fixiert sind.

2. Verfahren nach Anspruch 1, wobei der Antikörper eine Bindungsaffinität für einen Zelloberflächenrezeptor aufweist, wobei der Zelloberflächenrezeptor CD56 ist.

3. Verfahren nach Anspruch 1, wobei der erste Zelltyp natürliche Killerzellen (NK) sind.

4. Verfahren nach Anspruch 1, wobei der Antikörper eine Bindungsaffinität für ein Protein aufweist, das aus der Gruppe ausgewählt wird, die aus CD3, CD4, CD5, CD6, CD8, CD25, CD27, CD28, CD137 und CD278 besteht.

5. Verfahren nach Anspruch 4, wobei der Antikörper eine Bindungsaffinität für ein Protein aufweist, das CD3 oder CD28 umfasst.

6. Verfahren nach Anspruch 5, wobei der erste Zelltyp T-Zellen sind.

7. Verfahren nach Anspruch 1, ferner umfassend: Ablösen mindestens eines Großteils der biologischen Zellen des ersten Zelltyps von den magnetischen Beads; Anlegen eines Magnetfelds an die magnetischen Beads, um die magnetischen Beads sicher an ihrem Platz zu fixieren; und Leiten einer Flüssigkeit durch die magnetischen Beads und die abgetrennten biologischen Zellen des ersten Zelltyps, während das Magnetfeld angelegt wird, wobei die Flüssigkeit mit einer Kraft fließt, die stark genug ist, um mindestens einen Großteil der biologischen Zellen des ersten Zelltyps von den magnetischen Beads wegzuspülen, während mindestens ein Großteil der magnetischen Beads durch das Magnetfeld an seinem Platz fixiert bleibt.

8. Verfahren nach Anspruch 7, wobei mehr als 95 % der magnetischen Beads durch das Magnetfeld an Ort und Stelle zurückgehalten werden, während mindestens ein Großteil der biologischen Zellen des ersten Zelltyps von den magnetischen Beads weggespült wird.

9. Verfahren nach Anspruch 8, wobei mehr als 95 % der magnetischen Beads von den biologischen Zellen des ersten Zelltyps abgetrennt werden.

10. Verfahren nach Anspruch 1, wobei mehr als 95 % der von den biologischen Zellen des ersten Zelltyps abgelösten magnetischen Beads von den biologischen Zellen des ersten Zelltyps getrennt werden.

11. Verfahren nach Anspruch 1, wobei die magnetischen Beads einen Durchmesser von etwa 0,5 pm bis etwa 3 pm haben.

12. Verfahren nach Anspruch 1, ferner umfassend das Neigen der Plattform, auf der der Verarbeitungsbeutel ruht, in eine erste Richtung relativ zur Waagrechten, wobei das Magnetfeld auf die magnetischen Beads im Verarbeitungsbeutel angewendet wird, während die Plattform in die erste Richtung geneigt ist.

13. Verfahren nach Anspruch 12, wobei der Magnet angehoben wird, nachdem die Plattform in die erste Richtung geneigt ist.

14. Verfahren nach Anspruch 12, weiterhin umfassend das Injizieren eines Gases oder einer Flüssigkeit durch einen Anschluss (416) des Verarbeitungsbeutels, während die Plattform in die erste Richtung geneigt ist.

15. Verfahren nach Anspruch 12, das ferner das Folgende umfassend:
Neigen der Plattform in eine zweite Richtung gegenüber der ersten Richtung, sodass die Plattform, auf der der Verarbeitungsbeutel ruht, relativ zur Waagrechten abgewinkelt ist; und
Entnahme von Flüssigkeit aus dem Verarbeitungsbeutel, während die Plattform in die zweite Richtung geneigt ist.

## Revendications

1. Une méthode pour séparer les cellules biologiques d'un premier type cellulaire des cellules biologiques d'un deuxième type cellulaire à l'aide d'un système de traitement de billes, la méthode comprenant :
a. contacter un échantillon comprenant les cellules biologiques du premier type cellulaire et du deuxième type cellulaire avec des billes magnétiques (179) de sorte que les billes magnétiques se fixent aux cellules biologiques du premier type cellulaire par le biais d'une liaison d'anticorps, l'échantillon et les billes magnétiques étant éliminés dans un sac de traitement (206) posé sur une plate-forme (290) ;
b. soulever un aimant (299) par rapport à la plateforme de sorte que l'aimant produise un champ magnétique qui fixe en toute sécurité les billes magnétiques par rapport à la plateforme, les billes magnétiques étant reliées aux cellules biologiques du premier type cellulaire ;
c. faire passer un liquide à travers l'échantillon et les billes magnétiques pendant que le champ magnétique y est appliqué, le liquide passant avec une force suffisamment forte pour éliminer les cellules biologiques du deuxième type cellulaire des cellules biologiques du premier type cellulaire; et
d. abaisser l'aimant par rapport à la plateforme de sorte que les billes magnétiques sur lesquelles sont fixées les cellules biologiques du premier type cellulaire ne sont plus fixées en place en toute sécurité par rapport à la plateforme par le champ magnétique de l'aimant.

2. La méthode telle que récitée à la revendication 1, dans laquelle l'anticorps a une affinité de liaison pour un récepteur de surface cellulaire, le récepteur de surface cellulaire étant le CD56.

3. La méthode telle que récitée à la revendication 1, dans laquelle le premier type cellulaire est celui des cellules tueuses naturelles (NK).

4. La méthode telle que récitée à la revendication 1, dans laquelle l'anticorps a une affinité de liaison pour une protéine sélectionnée dans le groupe composé de CD3, CD4, CD5, CD6, CD8, CD25, CD27, CD28, CD 137 et CD278.

5. La méthode telle que récitée à la revendication 4, dans laquelle l'anticorps a une affinité de liaison pour une protéine comprenant CD3 ou CD28.

6. La méthode telle que récitée à la revendication 5, dans laquelle le premier type cellulaire est celui des lymphocytes T.

7. La méthode selon la revendication 1, comprenant en outre : détacher au moins une majorité des cellules biologiques du premier type cellulaire des billes magnétiques ; appliquer un champ magnétique sur les billes magnétiques afin de les fixer en place en toute sécurité ; et en faisant passer un liquide à travers les billes magnétiques et les cellules biologiques séparées du premier type cellulaire pendant l'application du champ magnétique, le liquide passant avec une force suffisamment forte pour éliminer au moins une majorité des cellules biologiques du premier type cellulaire des billes magnétiques tandis qu'au moins la majorité d'entre elles restent fixées en place par le champ magnétique.

8. La méthode telle que récitée à la revendication 7, dans laquelle plus de 95 % des billes magnétiques sont retenues en place par le champ magnétique tandis qu'au moins une majorité des cellules biologiques du premier type cellulaire sont éliminées des billes magnétiques.

9. La méthode telle que récitée à la revendication 8, dans laquelle plus de 95 % des billes magnétiques sont séparées des cellules biologiques du premier type cellulaire.

10. La méthode telle que récitée à la revendication 1, dans laquelle plus de 95 % des billes magnétiques sont séparées des cellules biologiques du premier type cellulaire.

11. La méthode telle que récitée à la revendication 1, dans laquelle les billes magnétiques ont un diamètre d'environ 0,5 pm à environ 3 pm.

12. La méthode telle que citée à la revendication 1, comprenant en outre l'inclinaison de la plateforme sur laquelle le sac de traitement repose dans une première direction par rapport à l'horizontale, le champ magnétique étant appliqué aux billes magnétiques à l'intérieur du sachet de traitement pendant que la plateforme est inclinée dans la première direction.

13. La méthode telle que récitée à la revendication 12, dans laquelle l'aimant est soulevé après que la plateforme a été inclinée dans la première direction.

14. La méthode telle que récitée à la revendication 12, comprenant en outre l'injection d'un gaz ou d'un liquide à travers une chambre implantable (416) du sachet de traitement alors que la plateforme est inclinée dans la première direction.

15. La méthode telle que récitée à la revendication 12, comprenant en outre :
incliner la plateforme dans une deuxième direction à l'inverse de la première direction de sorte que la plateforme sur laquelle repose le sachet de traitement soit orientée par rapport à l'horizontale ; et
retirer le liquide du sachet de traitement pendant que la plateforme est inclinée dans la deuxième direction.
